# EUROPEAN PATENT APPLICATION

(11) **EP 4 506 459 A1**
(43) Date of publication of application: **12.02.2025**
(21) Application number: 23382830.0
(22) Date of filing: 08.08.2023
(51) Int. Cl.: C12N 15/82, C12N 9/22

(54) **A NICOTIANA TABACUM PLANT WITH MUTATIONS IN FT AND SPL GENES AND ITS USE AS BIOFACTORY**

(71) Applicant: Consejo Superior De Investigaciones Científicas - CSIC, 28006 Madrid (ES); Universitat Politécnica de Valencia, 46022 Valencia (ES)
(72) Inventor: Orzaez Calatayud, Diego, Valencia (ES); Vazquez Vilar, Marta, Valencia (ES); Fernández del Carmen, Asunción, Valencia (ES); Ferrandis Mestre, Cristina, Valencia (ES); De Paola, Carmine, Valencia (ES); Darós Arnau, José Antonio, Valencia (ES); García Pérez, Elena, Valencia (ES)
(74) Representative: Pons

(57) **Abstract**

The invention belongs to the technical field of the biofactory plants. The invention relates to a plant belonging to *Nicotiana tabacum* characterized in that it comprises monoallelic or biallelic mutations in the coding region of genes of the squamosa-promoter binding like (SPL) family and/or the flowering locus T (FT) family, particularly mutations in the sub-groups SPL genes: SPL3/4/5, SPL9/15, SPL2/10/11 and SPL13; and in the FT5 gene. The invention further relates to a method for producing said *Nicotiana tabacum* plant and its use as biofactory for producing recombinant proteins.

## Description

The invention belongs to the technical field of the biofactory plants. The invention relates to a plant belonging to *Nicotiana tabacum* characterized in that it comprises monoallelic or biallelic mutations in the coding region of genes of the squamosa-promoter binding like (SPL) family and/or the flowering locus T (FT) family, particularly mutations in the sub-groups SPL genes: SPL3/4/5, SPL9/15, SPL2/10/11 and SPL13; and in the FT5 gene. The invention further relates to a method for producing said *Nicotiana tabacum* plant and its use as biofactory for producing recombinant proteins.

### BACKGROUND ART

In the last decade, some plant species such as *Nicotiana benthamiana* and *Nicotiana tabacum* species, have been used by an emerging industrial sector known as molecular farming to produce, for example, vaccines against SARS-Cov2 and seasonal flu (Hager et al., New England Journal of Medicine 386, no. 22: 2084-96 (2022)), recombinant antibodies (Diamos et al., Frontiers in Bioengineering and Biotechnology 7 (2020)), human growth factors, as well as a wide range of other chemical specialties (Hanittinan et al., Biotechnology Reports 28: e00524 (2020)).

Unlike food crops, which have been the subject of intensive breeding efforts for centuries to increase their agronomic performance and nutritional value, the improvement of biofactory plants has received little attention. In fact, their breeding goals are often different from those of traditional crops. On the other hand, the technologies that can be applied in plant improvement have recently been expanded. Together with traditional marker-assisted breeding and genetic engineering (including gene silencing techniques), genome editing has recently been added to the plant breeders' toolbox.

The main advantages of genome editing are its high precision and the fact that it can generate a plant free of transgenic sequences. In the field of molecular farming, CRISPR/Cas mediated gene editing has been successfully used to achieve unconventional goals in the context of plant breeding, such as the removal of plant-specific protein glycosylation patterns to facilitate obtaining biosimilar pharmaceutical products (Jansing et al., Plant Biotechnology Journal 17, no. 2: 350-61 (2019)). However, it has not been used so far to obtain plants that produce higher yields of recombinant protein.

The levels of accumulation of recombinant proteins in the plant depend on different factors, which have to do with both the nature of the protein to be produced, and the physiological state of the organs and tissues of the plant used as a biofactory. The most widely used system for industrial production of recombinant proteins in plants is the transient transformation of leaves by agroinfiltration. This method replaces the stable transformation of the germ line with a direct and transient transformation of the somatic tissues (leaves) and it is mainly used in the plants *Nicotiana benthamiana* and *Nicotiana tabacum.* Agroinfiltration speeds up product development, but operationally is a limiting factor in the process and its optimization becomes key for its economic viability.

SPLs are a family of plant-specific transcription factors that are characterized by the presence of the DNA-binding domain SBP in their sequence. The specific role of each SPL gene in the plant has been extensively studied in *Arabidopsis thaliana.* Arabidopsis possesses 16 SPL genes that can be divided in eight clades: AtSPL1/12/14/16, AtSPL2/10/11, AtSPL3/4/5, AtSPL6, AtSPL7, AtSPL8, AtSPL9/15, and AtSPL13. In Arabidopsis, AtSPL2/3/4/5/9/10/11/13/15 are targets of microRNA156 (miR156) and have been shown to play an important role in regulating the transition from juvenile to adult and vegetative to reproductive in various plant species. During early phases of development, elevated levels of miR156 repress the expression of targeted SPL genes. As the plant develops, there is a decrease in the levels of miR156 with a concomitant increase in the levels of SPLs, which allows the transition to the adult vegetative phase and to the reproductive phase with the acquisition of the competence to flower. SPL2/9/10/11/13/15 contribute to both the juvenile-to-adult phase transition (vegetative phase change) and the vegetative-to-reproductive phase transition (reproductive phase change or flowering), with SPL9/13/15 being more important than SPL2/10/11. These SPLs also reduce the rate of foliar initiation, lengthen the plastochronous and reduce the formation of lateral branches. Thus, phenotypes of loss-of-function mutants have been described in these genes that show later flowering and an increased number of leaves. SPL3/4/5 do not play a major role in vegetative phase change but are involved in the regulation of flowering and/or flower meristem differentiation and inflorescence development after flowering induction.

Although efficiency improvements have been introduced through optimizations of the agroinfiltration vector (*Agrobacterium tumefaciens*, viral systems), genetic improvements of the plant that increase its ability to achieve higher yields of recombinant protein have not been explored so far.

Along with higher yields, other improvement objectives in the field of biofactories are necessaries such as increasing efficiency in the transformation and regeneration of new transgenic lines, increasing productive biomass per surface unit, and increasing biosafety of the process.

### DESCRIPTION OF THE INVENTION

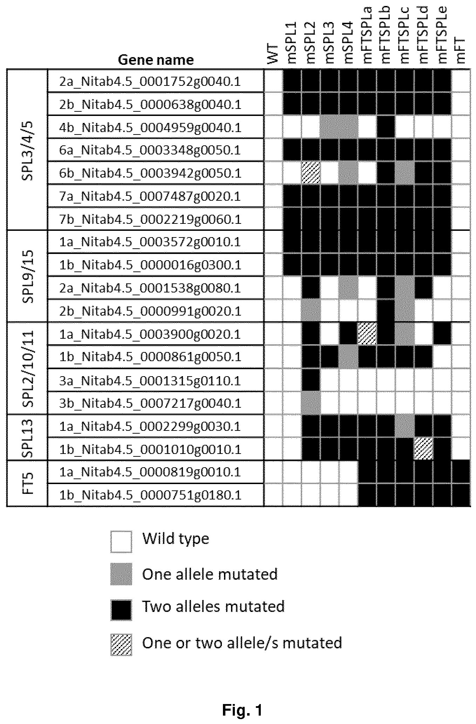

The present invention relates to a plant belonging to *Nicotiana tabacum* characterized in that it comprises monoallelic mutation (heterozygous) or biallelic mutations (heterozygous or homozygous) in the coding region of genes of the squamosa-promoter binding like (SPL) family and/or the flowering locus T (FT) family, particularly mutations in the sub-groups SPL genes: SPL3/4/5, SPL9/15, SPL2/10/11 and SPL13; and in the FT5 gene (Figure 1). Given the allotetraploid nature of *Nicotiana tabacum,* SPL and FT genes described in the present invention are grouped in pairs of homeologous genes in most of the cases. To facilitate their identification, all SPLs were named according to the *Arabidopsis thaliana* TAIR10 SPL gene belonging to the same subgroup. Then, the average protein length for each pair in the group was calculated, number 1 was assigned to the pair with the highest value, number 2 to the following, and so on. Each pair member was marked as "a" if the most likely subgenome donor was *N. sylvestris* and "b" if it was *N. tomentosiformis.* In some cases, there are SPL genes that do not have an homeologous partner, probably due to loss during evolution. Therefore, they were named with a number followed by U, standing for "unique".

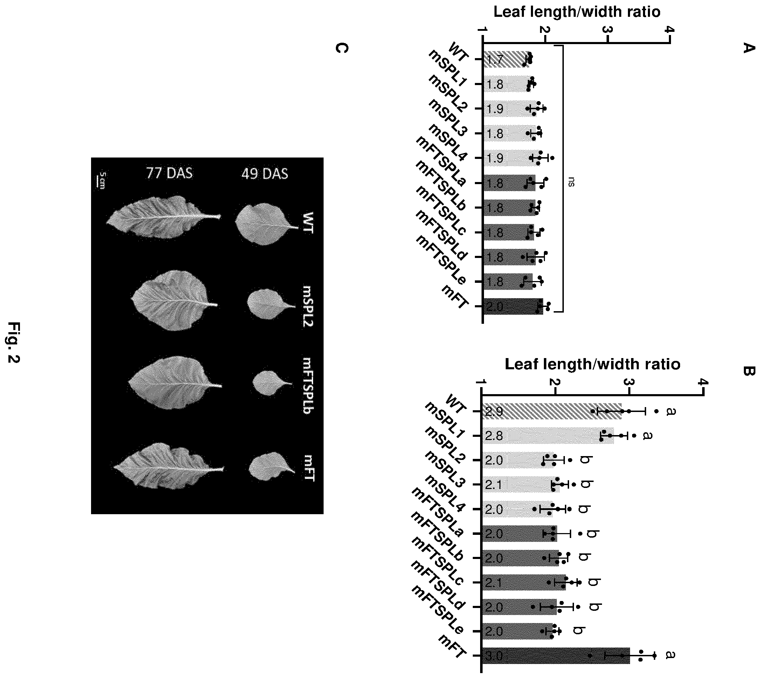

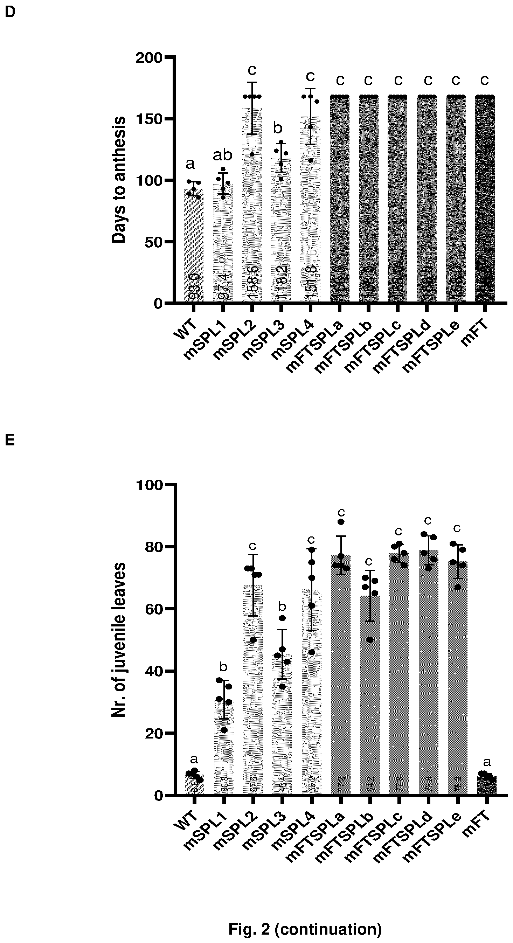

The combinations of mutations in these genes give rise to new varieties that simultaneously show delayed and/or prevented flowering and a higher rate of production of leaves with juvenile characteristics (Figure 2). The resulting plants present a series of favorable characteristics for applications, among others, as biofactories: (i) greater efficiency in the production of recombinant products (proteins, nucleic acids, or metabolites) by transient transformation, (ii) greater speed in the generation of stable transgenic lines, and (iii) improvements in biosecurity, associated with the elimination of flowering. Further, the plants of the invention include mechanisms to reverse flowering, allowing these varieties to reproduce by seed.

In the present invention, different sets of genes from the SPL and FT families were mutated using the CRISPR/Cas9 technique in *N*. *tabacum* var. K326, following a CRISPR/Cas9 multiplexing approach, and resulting in multiSPL mutant collections (mSPL1 to mSPL4, formed by plants mutated only in SPL genes), multiFT (mFT, formed by plants mutated only in FT genes) and multiFTSPL (mFTSPLa to mFTSPLe, formed by mutated plants in genes of both gene families) (Fig 1). Unexpectedly, the new combination of mutations was found not only to delay the juvenile-to-adult transition, but to abolish it completely and indefinitely, regardless of plant age.

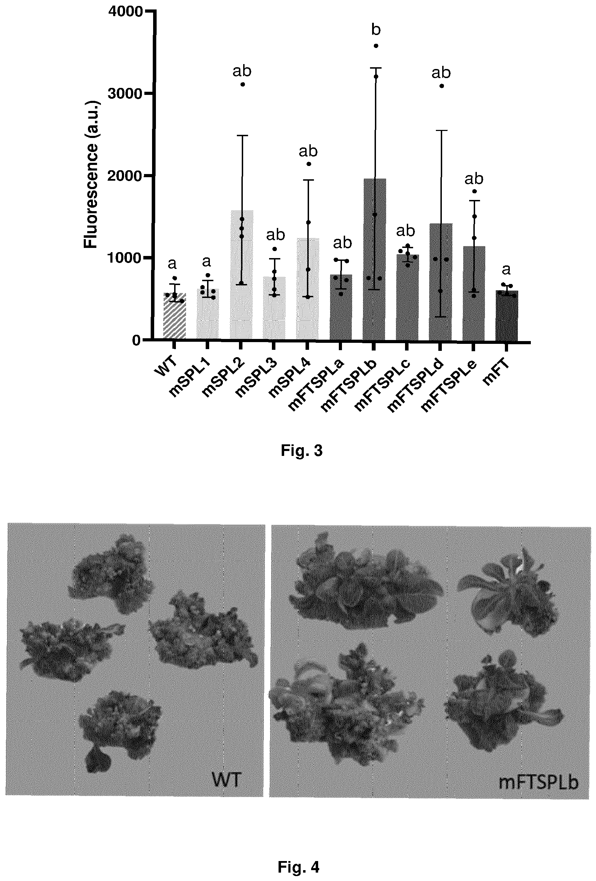

Remarkably, the mFTSPLb line presented the highest recombinant protein production efficiencies observed among all the lines analyzed, 3.4 times higher than the wild type on average (Figure 3). The genotype of this line had mutations in all the genes of the SPL13, SPL4 and SPL9/15 families, 4 out of the 6 genes of the SPL3 family, one of the two genes of the SPL5 family, two of the 8 genes of the SPL2/10/11 family, and finally the mutated FT5a and FT5b genes, all homozygous.

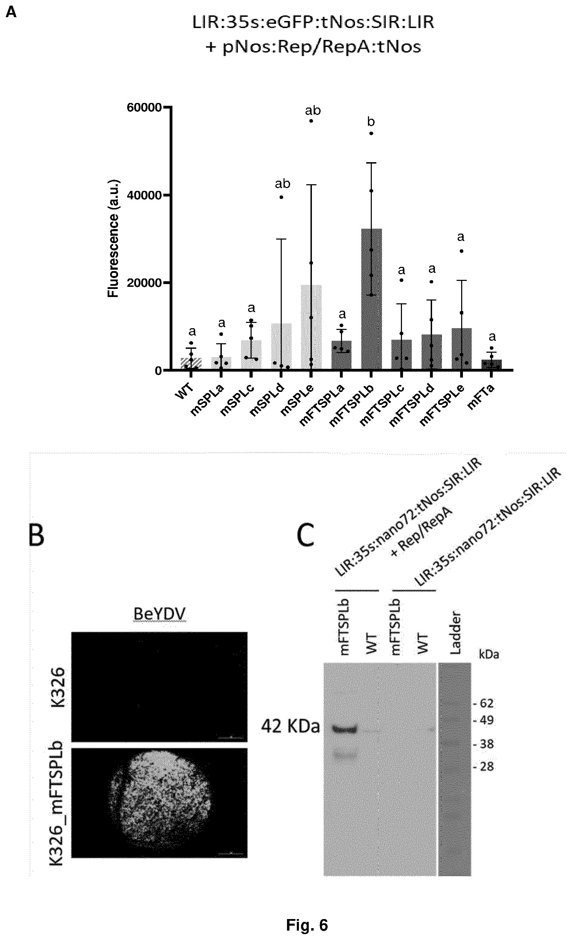

In addition, in genetic transformation experiments, it was observed that the mFTSPLb line also had higher explant growth rates (Figure 4) and the yields of recombinant GFP obtained by agroinfiltration of a recombinant geminivirus vector based on the Bean Yellow Dwarf virus (BeYDV) were up to 11.6 times higher in the mFTSPLb line compared to the WT (Figure 6).

Thus, a first aspect of the invention relates to a plant belonging to *Nicotiana tabacum* or part thereof, reproductive or propagating plant material or a plant cell, hereinafter the "plant of the invention" characterized in that it comprises:
**(a)** at least one monoallelic heterozygous mutation, biallelic heterozygous mutation or biallelic homozygous mutation in the coding region of the following SPL genes (all SPL genes in (a) comprising at least one mutation):
   - gene Nitab4.5_0001752g0040.1 (NtSPL3/4/5_2a) comprising, or consisting of, nucleotide sequence SEQ ID NO: 1,
   - gene Nitab4.5_0000638g0040.1 (NtSPL3/4/5_2b) comprising, or consisting of, nucleotide sequence SEQ ID NO: 2,
   - gene Nitab4.5_0003348g0050.1 (NtSPL3/4/5_6a) comprising, or consisting of, nucleotide sequence SEQ ID NO: 4,
   - gene Nitab4.5_0007487g0020.1 (NtSPL3/4/5_7a) comprising, or consisting of, nucleotide sequence SEQ ID NO: 6,
   - gene Nitab4.5_0002219g0060.1 (NtSPL3/4/5_7b) comprising, or consisting of, nucleotide sequence SEQ ID NO: 7,
   - gene Nitab4.5_0003572g0010.1 (NtSPL9/15_1a) comprising, or consisting of, nucleotide sequence SEQ ID NO: 8,
   - gene Nitab4.5_0000016g0300.1 (NtSPL9/15_1b) comprising, or consisting of, nucleotide sequence SEQ ID NO: 9,
   - gene Nitab4.5_0002299g0030.1 (NtSPL13_1a) comprising, or consisting of, nucleotide sequence SEQ ID NO: 12, and
   - gene Nitab4.5_0001010g0010.1 (NtSPL13_1b) comprising, or consisting of, nucleotide sequence SEQ ID NO: 13,
   **and**
**(b)** at least one monoallelic heterozygous mutation, biallelic heterozygous mutation or biallelic homozygous mutation in the coding region of at least one of the genes selected from the list consisting of (at least one SPL gene in (b) comprising at least one mutation):
   - gene Nitab4.5_0004959g0040.1 (NtSPL3/4/5_4b) comprising, or consisting of, nucleotide sequence SEQ ID NO: 3,
   - gene Nitab4.5_0003942g0050.1 (NtSPL3/4/5_6b) comprising, or consisting of, nucleotide sequence SEQ ID NO: 5,
   - gene Nitab4.5_0001538g0080.1 (NtSPL9/15_2a) comprising, or consisting of, nucleotide sequence SEQ ID NO: 10,
   - gene Nitab4.5_0000991g0020.1 (NtSPL9/15_2b) comprising, or consisting of, nucleotide sequence SEQ ID NO: 11,
   - gene Nitab4.5_0003900g0020.1 (NtSPL2/10/11_1a) comprising, or consisting of, nucleotide sequence SEQ ID NO: 14,
   - gene Nitab4.5_0000861g0050.1 (NtSPL2/10/11_1b) comprising, or consisting of, nucleotide sequence SEQ ID NO: 15,
   - gene Nitab4.5_0001315g0110.1 (NtSPL2/10/11_3a) comprising, or consisting of, nucleotide sequence SEQ ID NO: 16,
   - gene Nitab4.5_0007217g0040.1 (NtSPL2/10/11_3b) comprising, or consisting of, nucleotide sequence SEQ ID NO: 17, and any combination thereof,
wherein the plant or part thereof, reproductive or propagating plant material, or a plant cell, is not obtained by means of an essentially biological process.

The term "plant" as used herein includes whole plants, any "reproductive or propagating material" for a plant, progeny of the plants and parts of plants, including seeds, siliques, fruits, leaves, flowers, shoots, stems, tubers, roots, isolated plant cells, callus, tissues and organs. References to a plant may also include plant cells, plant protoplasts, plant tissue cultures, plant calluses, plant clusters and plant cells which are intact in plants or parts of plants, such as embryos, pollen, ovules, seeds, leaves, flowers, branches, fruits, grains, spikes, ears, hulls, stems, roots, root tips and the like. The offspring, variants and mutants of any of the plants described herein are within the scope of the present invention. The seeds of any of said plants are also included. As it is used herein, the term "parts of a plant" includes any part or parts of a plant including the seeds, siliques, fruits, leaves, flowers, shoots, stems and / or roots.

As the expert in the field is aware and knowledgeable about, the present invention may also be performed in plant cells. The term "plant cell" as used herein includes plant cells derived and/or isolated from plant cell tissue or from plant cell culture.

The invention herein described relates to a plant that belongs to *Nicotiana tabacum* species. *Nicotiana tabacum,* or cultivated tobacco, is an annually grown herbaceous allotetraploid plant of the *Nicotiana* genus. *Nicotiana tabacum* originated through the hybridization of *Nicotiana sylvestris* and *Nicotiana tomentosiformis.* The plant is tropical in origin, is commonly grown throughout the world, and is often found in cultivation. It grows to heights between 1 and 2 meters.

The genome of allotetraploid species derive from the fusion of two diploid genomes coming from two different species. Homeologous chromosomes are pairs of phylogenetically equivalent chromosomes coming from each of the ancestor species. Homeoleogous chromosomes do not segregate during meiosis. Homeologous genes are related genes located in equivalent positions in homeologous chromosomes.

In alloteraploid organisms, the same as in diploid species, there are two alleles for each trait of genes in each pair of chromosomes, one coming from the father and one from the mother. An allele is one of two or more alternative forms of a gene, and they are found at the same place, or locus, on the chromosome. Heterozygous refers to having different alleles for a particular trait. If the two versions are different, you have a heterozygous genotype for that gene. The relationship between the two alleles affects which traits are expressed.

The plan of the invention comprises mutations, preferably simultaneously, in the coding region of the Squamosa-Promoter binding Like (hereinafter, SPL) genes of *N*. *tabacum.* SPLs are a family of plant-specific transcription factors that are characterized by the presence of the DNA-binding domain SBP in their sequence. SPLs are crucial in regulating plant growth and development, synthesis of secondary metabolites, and stress resistance. SPLs constitute a large gene family comprising 42 members in *N. tabacum* var. TN90. These genes can be categorized into eight differentiated clusters based on their conserved motifs and phylogenetic analysis, namely SPL1/12, SPL8, SPL13, SPL6, SPL7, SPL2/10/11, SPL9/15, and SPL3/4/5. Twenty-eight out of the 42 NtSPL genes are targets of miR156 (He L, et al., Genes (Basel), (2023), 14(1):183). The level of miR156 and, thus the level of mir156-targeted SPLs, determines the timing of juvenile-to-adult phase transition and flowering time in tobacco. Mir156-targeted SPL genes regulate leaf initiation rate, leaf size, shoot maturation and floral transition. However, the knowledge of the exact role of each NtSPL is still limited (Zhang T, et al., Sci China Life Sci (2015); 58(3):253-60).

Particularly, the plant of the invention characterized in that it comprises monoallelic heterozygous mutations, biallelic heterozygous mutations and/or biallelic homozygous mutations in the coding region of genes of the following squamosa-promoter binding like (SPL) genes, preferably being all targets of miR156:
- The gene with number id *Nitab4.5_0001752g0040.1* of the *Nicotiana tabacum* genome database "*N. Tabacum* v4.5 Scaffolds Edwards 2017" (https://solgenomics.net/), belongs to family SPL3/4/5, wherein the coding region comprises the nucleotide sequence SEQ ID NO: 1:
   SEQ ID NO: 1 
ATGGAAACAGCTGGTCATCATAATATCTCTACTAGTGAGGATGATTATAAGAAGAAAGGCAGT
TGTAATAATAATATTACAACAAATAATAACCATATTAAGAAAGGGTCATTAAATAATGGAGGGTCA
AGTACTAATCAAAGGTGTTGTCAAGCTGAGAAGTGCAGTGCTGATTTAAGTGATGCTAAGCAGTAC
CATAGGAGGCATAAAGTCTGTGAATACCATGCAAAGGCTCAAGTCGTCGTCGTCGCCGGACTCCGG
CAACGCTTCTGTCAACAATGCAGCCGATTTCATGAGCTGACGGAATTTGATGAATCAAAAAGAAGT
TGCCGAAGGCGTTTGGCTGGACACAACGAAAGGCGAAGAAAGATCAGCTCTTCATCATCTGCAGCA
GAGTCTCAAATTGCAGAAAGTTCAAGTAGAAAAGGGACAGTGCAACTCAATAATAAGGACATTAAC
ATGATGTGTGGTCAAGTAAATGAAAAGGGAAGAATTCAGATTTCAATCCAAGAAAATTCCACTTTC
AAAAATTTCCACCCCCGTTGA
- The gene with number id Nitab4.5_0000638g0040.1 of the *Nicotiana tabacum* genome database "*N. Tabacum* v4.5 Scaffolds Edwards 2017" (https://solgenomics.net/), belongs to family SPL3/4/5, wherein the coding region comprises the nucleotide sequence SEQ ID NO: 2:
   SEQ ID NO: 2 
ATGGAAACAGCTGGTCATCATAATATTTCTACTAGTGAAGATGATTATAAGAAGAGAGGCAGT
TGCAATAATAATATTACAACAAATAATAACCATATTAAGAAAGGGTCATTAAATAATGGAGGGTCA
AGTACTACTCAAAGGTGTTGTCAAGCTGAGAAGTGCAGTGCTGATTTAAGTGATGCTAAGCAGTAC
CATAGGAGGCATAAAGTCTGTGAATACCATGCAAAGGCTCAAGTCGTCGTCGCCGGATTCCGG
CAACGCTTCTGTCAACAATGCAGCCGATTTCATGAGTTGACGGAATTTGATGAATCGAAAAGGAGT
TGCCGAAGGCGTTTGGCTGGACACAACGAAAGGCGAAGAAAGAGCTCATCATCAACTGCAGAGTCA
CATAATATTGCAGAAAGTTCAAGCAGAAAAGGGACAGTGCAACTCAATAATAAGGACATTAACATG
ATATGTGGTCAAGTAAATGACAAGGGAAGAATTCAGATTTCAATTCAAGAAAATTCCACTTTCAAA
AACTTCCACCTCCGTTGA
- The gene Nitab4.5_0004959g0040.1, belongs to family SPL3/4/5, wherein the coding region comprises the nucleotide sequence SEQ ID NO: 3:
   SEQ ID NO: 3 
ATGGAAGCAGCTGATAATAATGACTTGTTCATTTTTGCCACTGATAATGATGACAAGAAGAGA
ACCCCCAGTTATAATAATAGGAGTAGTAGTAGTAGTAATAATAACAATGAAAAGATCAAAGGGTCA
AGTACTTCAATGAGGTGTTGCCAAGCTGATAAGTGTACTGTTGATTTAAGTGATGCCAAACAATAC
CATAAGAGGCATAAAGTCTGTGAATACCATGCAAAGGCTCAAGCCGTCGTCGCCGGACTCCGG
CAACGCTTTTGTCAACAATGCAGCCGATTTCACGAGGTAGGAGAGTTTGATGAATCGAAAAGGAGT
TGCAGAAGGCGTTTGGCTGGGCACAACGAAAGGCGAAGGAAGACTACTACTTCTTCATCATCA
GATCAGTCTAATGCTGAAGTTTCATCGAGTAGAAAAGCCATAATAGGGACAGACACTCATCAGATA
ATAAACTTCCAAGAAAATGCAGCAGCAGGTTATAAACACTTTCAGATTGGATGA
- The gene Nitab4.5_0003348g0050.1, belongs to family SPL3/4/5, wherein the coding region comprises the nucleotide sequence SEQ ID NO: 4:
   SEQ ID NO: 4 
ATGGAAACTAGCAAATGGGAAGAGAAGAGAAGCATGATGGAAGAGGAGGATGACGAGGAAGACGAA
GATGTTGTAGAGGACAACAAAAGGAAGAGGGTCTTGACTCGCTCGGGAAGGAAGGTACCTGGCGGA
GAGGGTTCAAAGCTGCCTTCCTGTCAAATTGAGGACTGCACTGCAGATATGGCAAATGCCAAGACA
TACCATCGCCGCCACAAGGTCTGTGAATTTCATGCAAAGGCTCCGGCAGTACTTATTGGCGGACTC
CGACAGCGTTTCTGTCAGCAATGCAGCAGGTTTCATCAGTTGGGGGAGTTCGATGAAGCAAAAAGA
AGTTGCAGGAGACGTTTGGCAGGCCACAACGAACGCCGTCGTAAAAGTTCATGTGATTCTCCTGGA
GAAGGGTCAAGTTAA
- The gene Nitab4.5_0003942g0050.1, belongs to family SPL3/4/5, wherein the coding region comprises the nucleotide sequence SEQ ID NO: 5:
   SEQ ID NO: 5 
ATGGAAACTAGCAAATGGGAAGAGAAGAGAAGCATGACGGCAGCAGAAATGGAAGAGGAGGATGAC
GAGGAAGACGAAGATGTTGTGGAGGACAACAAAAGGAAGAGGGTCTTGACTCACTCGGGAAGGAAG
GTACCTGGCGGAGAGGGTTCAAAGCTGCCTTCCTGTCAAATTGAGGACTGCACTGCAGATATGGCA
AATGCCAAGACATACCATCGCCGCCACAAGGTCTGTGAATTTCATGCAAAGGCTCCAGCAGTACTT
ATTGGTGGACTCCGACAGCGTTTCTGTCAGCAATGCAGCAGGTTTCATCAGTTGGGAGAGTTCGAT
GAAGCAAAAAGGAGTTGCAGGAGACGTTTGGCAGGCCACAATGAACGCCGTCGTAAAAGTTCATGT
GATTCTCCTGGAGAAGGGTCAAGTTAA
- The gene Nitab4.5_0007487g0020.1, belongs to family SPL3/4/5, wherein the coding region comprises the nucleotide sequence SEQ ID NO: 6:
   SEQ ID NO: 6 
ATGGAAGATAACAAATGGGAAGGGAAGAGAAGCATGAATGAAGCAGAAGAGGAACATGAAAATGCT
GAAGAGGATAACAAAAGGAAGAGGGTATTGACTCTCTCTGGAAGGAATCTATCTGGTGGTGGGCCA
GCACTGCCTTCTTGCCAGGTTGAGCAGTGCACTGCAGATATGGCAGGTGCCAAGCCATATCATCGC
CGCCACAAGGTGTGCGAGTTTCATTCAAAGGCTCCGGTAGTACTTATTAGTGGAATCCAGCAGCGC
TTCTGTCAGCAATGTAGCAGATTTCATCAGTTGCCAGAGTTTGATGAAGCAAAAAGGAGTTGCCGG
AGGCGTTTGGCAGGTCATAATGAGCGCCGCCGTAAAACTTCATATGATTCTCATGGAGAAAGTTCA
AGCTGA
- The gene Nitab4.5_0002219g0060.1, belongs to family SPL3/4/5, wherein the coding region comprises the nucleotide sequence SEQ ID NO: 7:
   SEQ ID NO: 7 
ATGGAAGATAACAAATGGGAAGGGAAGAGAAGCATGAATGAAGCAGAAGAGGAAGAGGAGGAACAT
GAAAATGTTGAAGAGGATAACAAAAGGAAGAGGGTATTGACTCTCTCCGGAAGGAAGCAATCTAGT
GGTGGGCCAGCACTCCCTTCTTGCCAGATCGAGCAGTGCACTGCAGATATGGCAGATGCCAAGCCA
TACCATCGCCGCCACAAGGTGTGCGAGTTTCATTCAAAGGCTCCGGTAGTACTTATTAGTGGAATC
CAGCAACGCTTCTGCCAGCAATGTAGCAGATTTCATCAGTTAGCAGAGTTTGATGAAGCTAAAAGG
AGTTGCCGGAGGCGTTTGGCAGGTCATAATGAGCGCCGCCGTAAAACTTCATATGATTCTCATGGA
GAAAGTTCAAGCTGA
- The gene Nitab4.5_0003572g0010.1, belongs to family SPL9/15, wherein the coding region comprises the nucleotide sequence SEQ ID NO: 8:
   SEQ ID NO: 8 
ATGGCTATACGCCTGAGGCTAACGGGAATAGAGTGGCTAGCGCTTGATCAGTTTGCTCGCTGCAAT
GAAGAGTACTCTGTGGTTTACCTTGATTGGCTTGGTTGTTCTATCTCAGTATCAGATAAAGGGCTA
ACAATATCTTTGATCAGTTGCTTCTCTTTGCCTGGAATTAACTGTTGGTACTTTGCTTCCAGGTTC
CATCGGTTGCCAGAATTTGACCAAGGGAAACGCAGTTGCCGCAGGCGCCTAGCAGGCCATAATGAG
CGTCGGAGGAAACCTCCACCTGGATCTCTTTTGTCTAATCGCTATGGAAGTCTTTCTTCATCAATA
TTTGAAAACAATGGCAGATCTGGAAGTTTTCTGGTCGACTTCACTGCATATCCAAATCTCACTGGA
GGTGCATGGCCAAATACTAGATCATCTGATCGAGGATGGGATAATCAATCCACTGCATCAGGGAAG
CTTCTCCAAAGTCATTGGCTGAACGGTTCTGAAAATCCTACATCCGACCTTGTTCTGCAAGGTTCA
GTTGCAAGGGGTGCCAATTATTCTGGTCCTGGTATTATTCCTTCCGGAAATTGCTTCTCTGGAGTC
TCAGATTCCAGTGGTGCTCTCTCTCTTCTGTCAAATGAGTCATGGGGCTCGAGGAACCAATCCTCT
AGCCTCGGGGTTAACGGCTTGGTTAACACTGATGGCGGACATACCGTTCAGCCATCGGGTTCCCAT
GCTGCTCCTGTCAATCACTACTCAGGCTCTCTATGGGGATTTAAAGGAAATGAAGCTAGTAGCAGT
TCACATGCAATACCTCCTGATCTCGGGCTTGGTCACATTTCTCAACATGCTGTCAATCAGTACTCT
GCTGAGCTTGGGATGGCTCAGCACAGTGGAAGACAGTACATGGAACTGGAGCACTCAAAGGGTTAC
GATTCTTCTGTTCAGAATGTGCACTGGACACTTTGA
- The gene Nitab4.5_0000016g0300.1, belongs to family SPL9/15, wherein the coding region comprises the nucleotide sequence SEQ ID NO: 9:
   SEQ ID NO: 9 
ATGAATGGGCTCAGTAAGTCGGAAATGGAACTTCTGGCTTCTGCTTCTTCTTCTACTTCTACTACT
TCTACTTCCCCTGACTCTCCTCCTAACACTTTAAAATTTGGTCAAAAAATCTACTTTGAACATGTT
GGACCTCACCACCCCAAATCAGCAACTGGGTCGTCTTCATCTCCGGTCACCGGAACTCCGGCGCCG
ACGACGTCCAAGAAAGGAAGAGGGGGTGGTGTGGTTCAAGGTCGACAGCCATCTAGGTGTCAAGTT
GAAGGGTGTGAAGCAGATCTGAGTGATGTTAAGGCTTATTATTCAAGGCACAAAGTCTGTGCTACA
CATTCTAAGTCTCCTGTGGTCATTGTTGCTGGTCTTGAACAAAGATTTTGTCAACAGTGTAGCAGG
TTCCATCGGTTGCCAGAATTTGACCAAGGGAAACGCAGTTGCCGCAGGCGCCTAGCAGGCCATAAT
GAGCGTCGCAGGAAACCTCCACCTGGATCTCTTTTGTCTAATCGCTATGGAAGTCTTTCTTCATCA
ATATTTGAAAACAATGGCAGATCTGGAAGTTTTCTGGTCGACTTCACTGCATATCCGAATCTCACT
GGAGGTGCATGGCCAAATACTAGATCATCTGAGCGGGGATGGGATAATCAATCCACTGCATCAGGG
AAGCTTCTCCAAAGTCATTGGCTGAACAGTTCTGAAAATCCTACATCCGACCTTGTTCTGCAAGGT
TCAGTTGCTAGGGGTGCCAATTATTCTGGTCCTGGTATTATTCCTTCCGGAAACTGCTTCTCTGGA
GTCTCAGATTCCAGTGGTGCTCTCTCTCTTCTGTCAAATGAGTCGTGGGGCTCGAGGAACCAATCC
TCTAGCCTCGGGGTTAACGACTTGGTTAACACTGATGGCGGACATACCGTTCAGCCATCGGGTTCC
CATGCTGCTCCTGTCAATCACTACTCAGGCCCTCAATGGGGATTTAAAGGAAATGAAGCTAGTAGC
AGTTCACATGCAATACCTCCTGATCTCGGGCTGGGTCACATTTCTCAACTTGCTGTCAATCAGTAC
TCTGCTGAGCTTGGGATGGCTCAGCACAGTGGAAGACAGTACATGGAACTGGAGCACTCAAAGGGT
TACGATTCTTCTGTTCAGAATGTGCACTGGACACTTTGA
- The gene Nitab4.5_0001538g0080.1, belongs to family SPL9/15, wherein the coding region comprises the nucleotide sequence SEQ ID NO: 10:
   SEQ ID NO: 10 
ATGAATGGGCAGTGGAATTCGGTAATGGAACTGGGTTCAGTTTCTTCTCCTGGTAGTAATTCAAGT
TCCTCCGACTCTCTCAATGGTTTGAACTTTGGCAAGAAAATCTACTTTGAAAATGTGGGTGGTGGG
TCATCGCCGGTGACCGGAGCCGGGAACTTGCCGCCGGCGTCCAAGAGGGGGAGGGGTGGGTTGATG
CAGGGTGGTCAGCCACCTAGGTGTCAAGTTGAAGGTTGTCAGGTAGATCTGAGTGATGCTAAGGCT
TATTATTCGAGGCATAAAGTTTGTGGTATGCACTCTAAGTCTCCTACTGTTATTGTTGCTGGCCTT
GAACAGAGGTTTTGTCAACAATGTAGCAGGTTCCATCAATTGCCAGAATTTGACCAAGGAAAAAGA
AGTTGCCGCAGGCGCCTGGCATGCCATAATGAGCGCCGCAGGAAGCCTCCATCTGGATCTCTTTTC
TCTACACATTACGGGAGCCTTTCTTCATCAATTTTTGAAAATAATAGCAGCAAATCTGGAAGCTTT
CTGTTAGACTTCAGCTCACACCCACATGTCAACGAGAGTTCGTGGCCAAATACTAGAGCATCTGAA
CAAGGATGGGATCATCAATCTACTGCATCAGGGAAGTGTCTTCAGCGTCCTTGGCTGAACAATTCT
GAAAATGCTACATCTGAGTTAGTTCTGCAAGGTTCAGCCACCAGGACCAATTATGTTCCTTCAGGG
 
GAATGTTTTTCTGGAGTCTCAGACTCCAGTACTGGTGCTCTCTCTCTTCTGTCAAATCGGTCCTGG
GGATCAAGGAATCCACCCTCAAGTCTTGGAGCTAACATTGATGGGATACACACCATTCAACCTTCT
GATTCCCATGGTGCACCTACCAATCACTTCTCAAGCCCTTCGTTACGGTTTAAAGGAAATGAAGCT
AGCAGCAGTTCACACGAGATGCCTTCTGATTTGGGTTTGGGTCAAATTTCACAGGCTGCTGACGAT
CAGTACTGTGGTGAGCTGGGGATAGGTCAGCAGAGTGGACGACAAAACATGGAACTGGACCACTCT
AACGGTTATCATCCTTCTGTTCAGAATGTGCACTGGTCTCTTTGA
- The gene Nitab4.5_0000991g0020.1, belongs to family SPL9/15, wherein the coding region comprises the nucleotide sequence SEQ ID NO: 11:
   SEQ ID NO: 11 
ATGAATGGGCAGTGGAATTCGGTAATGGAACTGGGTTCAGTTTCTTCTTCTGGTAGTAATTCAAGT
TCTTCCGACTCTCTCAATGGTTTGAAGTTTGGCAAGAAAATCTACTTTGAAAATGTGGGTGGTGGG
TCATCGCCGGTGACCGGAGACGGAAACTTGCCGCCGGCGTCCAAGAGGGGGAGGGGTGGATTGGTA
CAGGGTGGTCAGCCACCTAGGTGTCAAGTTGAAGGTTGTCAGGTAGATCTGAGTGATGCTAAGGCT
TATTATTCGAGGCATAAAGTTTGTGGTATGCACTCTAAGTCTCCTACTGTTATTGTTGCTGGTCTT
GAACAGAGGTTTTGTCAACAATGTAGCAGGTTCCATCAATTGACAGAATTTGACCAAGGAAAAAGA
AGTTGCCGCAGGCGCTTGGCATGCCATAATGAGCGCCGCAGGAAGCCTCCATCTGGATCTCTTTTC
TCTACACATTACGGGAGCCTTTCTTCATCAATTTTTGAAAATAATAGCAGCAAATCTGGAAGCTTT
CTGTTAGACTTCAGCTCACACCCACATGTCAACGAGAGTTCGTGGCCAACTAGAGCATCTGAACAA
GGATGGGATCATCAATCTACTGCATCAGGGAAATTTCTTCAGCGTCCTTGGCTGAATAATTCTGAA
AACGCTACATCTGAGTTTGTTCTGCAAGGTTCAGCCACCAGGACCAATTATCCCGGTCCTGGTGTT
CCTTCAGGGGAATGTTTCTCTGGAGTCTCAGACTCCAGTACCGGTGCTCTCTCTCTTCTGTCAAAT
CGGTCCTGGGGATCAACGAATCCACCCTCAAGTCTTGGGGCTAACATTGATGGGGTACACACCCCC
ATTCAACCTTCCGATTCCCATGCTGCACCTACCAATCACTTCTCAAGCCCTTCACTGCAGTTTAAA
GGAAATGAAGCTAGCAGCAGTTTGCACGAGATGCCTGATTTGGGTTTGGGTCAAATTTCACAGGCT
GCTGACAATCAGTACCGTGGTGAGCTACGGATGGGTCATCAGAGTGGACGACAAAACATGGAATTG
GACCACTCTAACGGTTATCATCCTTCTGTTCAGAATGTGCACTGGACGCTTTGA
- The gene Nitab4.5_0002299g0030.1, belongs to family SPL13, wherein the coding region comprises the nucleotide sequence SEQ ID NO: 12:
   SEQ ID NO: 12 
ATGGAATCATCATCCTCATCCTCATCCTCGTCTAAGAGGGCAAAAGCACCTGGCAATATT
GCTCAAGTAGCTCATTGCTTGGTTGATGGATGTAATGCAGACCTTAGCCAATGCAGAGAG
TATCACCGCCGCCATAAAGTTTGTGAGGTCCATTCAAAGACTGCCAAATGTTTATCTCGA
TCTGATGGTTCCTTTTGTAGGTTTCATTCCTTGGTAGAATTTGATGATGGAAAGAGAAGC
TGTCGGAAACGTTTGGATGGACATAACAGGCGTCGAAGGAAACCTCAGCCAGATTCTATG
GCCAAAACTTCTGGATTACTTTTTACTGGCCAGCAAGGGACGAAACTTCTGTCATTTAGC
 
AGTCAACAAATTTTTCCAAGTGCAGCTGCATGGGCTGGCGTAGTCAAAACGGACAGCGAA
ATGGTATTATACAACAACCAATCGCATATAAATTGCATGGACAATCAAAACTCGTTCCCT
GATTCTTCAGCTCGTAGCTACAAAGGAGGAAATCAATTCCAGTTCATGCAAGGCAGTGAC
CGTAGTCTCCCTGAAGCTTCAATCTACCAGCCACTTGTTGATCATCCCAATTCTGCAGTA
GCAGCAGCAGTTCCTAGCAGCGGACAAAAGATCTTCTCCAGTGGATTGAACGAAATTGTG
GACTCTGATCGTGCTCTCTCTCTTCTGTCATCAGCACCCGCTGTAACTAGGGAGATTGGT
TTTAGTCACATGGTGCAGCAGCCTGCCACTATCCCCCGGCCCCAGTCACAATCTGTCGTT
CATAGCCTGCACTATGGTGGTCTAAGCCAGTACCCTTTTGCTCAAGATTTCAATAGCCAA
CCTCAAGATTCTGCTGCAGATTCACATGTTAGCAACAACAGCTCTCTGCATTTCCATGAT
ATGTTACAAAATGGAGCAGATGGATCATCTACAAGTGGTGGCTGTCAGCAAACACTAGCC
TTTATGTGGGACTAA
- The gene Nitab4.5_0001010g0010.1, belongs to family SPL13, wherein the coding region comprises the nucleotide sequence SEQ ID NO: 13:
   SEQ ID NO: 13 
ATGGAATCATCCTCCTCATCTAAGAGGGCAAAAGCACCTGGCAATATTGCTCAAGTAGCTCATTGC
TTGGTTGATGGATGTAATGCAGACCTCAGCCAATGCAGAGAGTATCACCGCCGCCATAAAGTTTGT
GAGGTCCATTCAAAGACTGCCAAAGTCACGATTGGGGGTCGAGACCAACGTTTCTGTCAGCAATGC
AGCAGGTTTCATTCCTTGGTAGAATTTGATGATGGTAAGAGAAGCTGTCGGAAACGTCTGGATGGA
CATAACAGGCGTCGAAGGAAACCTCAGCCTGATTCTATGGCCAAAACTTCTGGATTACTTTTTACT
GGCCAGCAAGGGACGAAAGTTCTGTCATTTAGCAGTCAACAAATTTTTCCAAGTGCAGTTGTGAGC
TCTGCATGGGCTGGCGTAGTCAAAACGGACAGCGACATGGTATTATACAACAATATAAATTGCATG
GACAATCAAAACTCGTTCCCTGATTCTTCGGCTCGTAGTAGCTACAAAGGAGGAAATCAATTCCAG
TTCATGCAAGGCAGTGAGCGTAGTCTCCCTGAAGCTTCAATCTGCCAGCCACTTGTTGATCATCCC
AATTCTACAGCAGCAGTTCCTAGCAGCGGACAAAAGATCTTCTCCAGTGGATTGAACGAAATTGTG
GACTCTGATCGTGCTCTCTCTCTTCTGTCATCAGCACCCGCTGTAACTAGGGAGATTGGTTTTAGT
CACATGGTGCAGCAGCCTGCCTCTATCCCCCGGCCCCGGCCCCAGTCACAATCTGTCGTACATAGC
CTGCACTATGGTGGTCTAAGCCAGTACCCTTTTGCTCAGGATTTCAATAGCCAACCTCAAGATTCT
GCTGCAGATTCACATGTTAGCAACAGCAGCTCTCTGCATTTCCATGATATGTTACAAAATGCACCA
GATGGATCATCTACAAGTGGTGGCTGTCAGCAAACACTAGCCTTTATGTGGGACTAA
- The gene Nitab4.5_0003900g0020.1, belongs to family SPL2/10/11, wherein the coding region comprises the nucleotide sequence SEQ ID NO: 14:
   SEQ ID NO: 14 
ATGGAGTGGAATGCAAAGTGGGACTGGGGAAACCTGGTAATGTTTGGTTCAGAGGCCGCCAAAAGT
CCAAAAGAACTACAATTAACAGATTGGGGAGTTGAAGAAGATGGAGAACTTGATGCTGGATCCTTC
 
AATTTGTCAAGTGGTGGTAGTGGTAGTGGTAGTGGTACCGGTGGTTATGTCTCTGATGTTGGGCAT
GGTTCTTCGATCAAGAGCTCAATATCAGCTTCTACTGACTCTTCACCCAAGGAGGGCTTGAAAGCA
TGCAACTTTTCTTTCGATGGCTCTTCTGAGGATCTTAGTAAGAAAGTGAAACTTTGCTCTAGAAAT
TCACCCCCCATGGAGGCTTCAGTAGGCTCTGTTGAACCACTTATAGGTCTGAAACTTGGTAAGCGG
ACATTTGAGACCATCGGCGGTGCTAAGGTTTCATCCTTCCCCGAGAAACACGTATCATCTGCTGCA
GCAGCAAAGAAAACAAAATCATCTACTCAGAATGCACCAACTCCTCGCTGTCAGGTTGAAGGCTGC
AGCCATGATCTTTCATCAGCTAAAGAATATTACCGGAAGCATCGAGTTTGTGACAGTCATTCCAAA
TGCCCAAAGGTCATTATAGCAGGTGTAGCACGTCGCTTTTGTCAACAGTGTAGCAGGTTCCATAGC
TTGTCTGAATTTGATGAAAAGAAGAGAAGCTGTCGCAGGAGGCTCTCTGATCACAATGCACGGCGC
CGCAAACCACAGCAGGAAACCATCCAGTTCAACTCGGCAAGGCTTTCTTCATTGTTTTATGATAGC
AGGCAACCTATGAATCTTGTGCTCAACCATTCCCAACTGGTTCAGTCTAGAGCTGCTGCAAATTCT
ACCTGGGAAAGCACTGAAGAAACTAAGTTCAGTATAACAAGAGGGTCTACTCCAAAGCCTGAAAGA
GCTGGCAGTACAAATGGGCTGTCATTATTGCCAGGGATCCAGTTGTCACGGGCCGTGGGTGCACAC
AGTAATGTATCTAGCTGCTTGCTCTTGCCCTCCAAGGGCCACACATCCGAGGTTTTCAATCGAGGT
GCCACGGAATCCATGTTCAATATGGGTACAGGACGGGAATTTCCTCGTGCTCTCTCTCTTCTGTCA
ACTAATTCATGGGGTTCATCCGAGCCTGAGACTGTTTCACTGAACAACCCAACACACGCGAATCAG
AGCAGCATGCCCGAGCAGATGATGCAGGCAATTCCACAAGGTGTGCCACTTATGTCCTGTGAATAC
TGGCAGGGTGAACAACCCTCATCCGATCCTCGTGACCATACATTGGCTGCTAATAGTCATGCTGGT
GGTAGCTTTCAGGGAATCGAGCTGTTCAAACCTCCATTTGACACTGACTTTTATCTCAATGCATTG
AACTGA
- The gene Nitab4.5_0000861g0050.1, belongs to family SPL2/10/11, wherein the coding region comprises the nucleotide sequence SEQ ID NO: 15:
   SEQ ID NO: 15 
ATGGAGTGGAATGCAAAGTGGGACTGGGGAAACCTGGTAATGTTTGGTTCAGAGGTCGCCAAAAGT
CCAAAAGAACTACAATTAACAGATTGGGGAGTTGAAGAAGATGGAGAACTTGATGCTGGATCCTTC
AATTTGTCGAGTGGTGGTAGTGGTAGTGGTACTGGTGGTTATGTCTCTGATGTTGGGTGTGGTTCT
TCGATCAAGAGCTCAATATCAGCTTCTACTGACTCTTCACCAAAGGAGGGCTTGAAAGCATCCGAC
TTTTCTTTCGATGGCTCTCCTGAGGATCTTAGTAAGAAAGTGAAACCTTGCTCTAGAAATTCACCC
CCCATGGAGGCTCCAGTAGGCTCTGTTGAACCACTTGTAGGTCTGAAACTTGGTAAGCGGACATTT
GAGACCATCGGTGGTGCTAAGGTTTCATCCTTCCCCGATAAACACGTATCATCTGCTGCAGCAGCA
AAGAAAACAAAATCATCTACTCAGAATGCACCAACTCCACGCTGTCAGGTTGAAGGCTGCAACCTT
GATCTTTCATCAGCTAAAGAATATTACCGGAAGCATCGAGTTTGTGAAAATCATTCCAAATGCCCA
AAGGTCATTATAGCAGGTGTATTACGCCGCTTTTGTCAACAGTGTAGCAGGTTCCATAGCTTGTCT
GAATTTGATGAAAAGAAGAGAAGCTGCCGCAGGAGGCTCTCTGATCACAATGCACGACGCCGCAAA
CCACAGCAGGAAACCATCCAGTTCAACTCGGCAAGGCTTTCTTCATTGTTTTATGATAGCAGGCAA
CCTATGAATCTTGTGCTCAACCATTCCAACCTGGTTCAGTGTAGAGCTGCTGCAAATTCTACCTGG
GAAAGCACTGAAGAAACTAAGTTCAGTATAACAAGAGGATTAGCTCCAAAGCCTGAAAGAGATGGC
 
AGTACAAATGGGCAGTCATTATTGCCAGGGATCCAGTTTTCACGGGCCGTGGGTGCACACGGTAAT
GTATCTAGCTGCTTGCTCTTGCCCTCCAAGGGCCACACAGCCGAGGTTTTCAATCGAGGTGCCAAG
GAATCCATGTTCAATATGGGTACAGGACCGGAATTTCCTCGTGCTCTCTCTCTTCTGTCAACTAAT
TCATGGGGTTCATCCGAGCCTGAGACTGTTTCACTGAACCACCCAACACATGCAAATCAGAGCAGC
ATGCCCGAGCAGATGATGCAGGCAATTCCACAAGGTGTGCCGCTTCTGTCCTGTGAATACTGGCAG
GGTGATGGACAACCTTCATCCGATCCTCGTGACCATACATTGGCTGCTAATAGTCATGCTGGTGGT
AGCTTTCAGGGAATCGAACTGTTCAAACCTCCATTTGACACTGATTTTTATCTCAATGCATTGAAC
TGA
- The gene Nitab4.5_0001315g0110.1, belongs to family SPL2/10/11, wherein the coding region comprises the nucleotide sequence SEQ ID NO: 16:
   SEQ ID NO: 16 
ATGCAGTTGTGTGCGCATAGCATTCTGCTGAAGTATGACAATCAAGTTTTAATATACTTCTTTGTC
TGTTACTGGAATCCAACAGCTCTGTTTGATAAAGATCCGAGTCCTACTTACTTTATTGCATTCCAT
GAGTGGGGAACTTTACAAAAGCCATCTCGTAGCTTCTTCCCCAAGATGGAGTGGAATGCAAAGTGG
GACTGGGGAAACCAGACCATCGGTGGTGCTAAGATTTCATCCTTCGCCGAGAAACCCGTATCATCT
GCTGCAGCAGCAAAGAAAACAAAATCATCTACTCAGAATGCACCAACTCCTCGCTGTCAGGTTGAA
GGCTGCAGCCTTGATCTTTCATCAGCTAAAGAATATTATCGGAAGCATAGAGTTTGTGACAGTCAT
TCCAAATGCCCAAAGGTCACTATAGCAGGTGTAGCACGCCATTTTTGTCAACAATGTAGCAGGTTC
CATAGCTTGTCTGAATTTGATGAAAAGAAGAGAAGCTGTCGCAGGAGGCTCTCTGATCACAATGCA
CGGCGCCGCAAACCACAGCAGGAAATCATCCAGCTGAACTCGGCAAGGCTTTCTTCATTGTTTTAT
GATAGCAGGAAACCTATGAATCTTACCCAACTGGTTCACTCTAGAGCTGCTGCAAATTCTACCAGG
GAAAGCACTGAAGAAACTAATTTCAGTATAACAAGAGGGTCTACTCCAAAGCCTGAAAGAGCTGGC
AGTACAAATGGGCAGTCATTACTGTCGGGGATCCAGTTTTCCTTGGCCGTGGGTGCACACAGTAAT
GTATCTAGCTGCTTGCTCTTGCCCTCCAAGGGCCACATAGACGAGGTTTTCAATCGAGATGCCAAG
GAATCCATGACCGGAATTTCCTCATGGGGTTCATCCGAGCCTGAGACTGTTTCACTGAACCACCCA
ACACATGTAAATCAGAGCAGCATGCCTGAGCAGATGATGCAGGCAATCCCACAAGGTGTGCCACTT
ATGTCCTGTGAATACTGGCAGAGAGGACAACCTTCATCCGATCCTCGTGACCATACATTGGCTGCT
AATAGTCATGCAAGT
- The gene Nitab4.5_0007217g0040.1, belongs to family SPL2/10/11, wherein the coding region comprises the nucleotide sequence SEQ ID NO: 17:
   SEQ ID NO: 17

ATGACAATCAAAGTGAGGAAACAACAAGTAGCTGAAAATGATGTGGTGGAGTTAAGGGATGCGATA
GATGGGGAGATGCATTTTGTGGCCTTGGATTGGAAGGGCTTGAGCTCTGATTCTCTTCTTCCCAAG
ACGGAGTGGAATGCAAAGTGGGACTGGGGAAACCTGATAATGTTTGGTTCAAAGGCCGCCAAAAGT
CCAAAAGAACTACAATTAACAGATTGGGGAATTGAAGAAGATGGAGAACTTGATGCTGGATCCTTC
AATTTGTCGAGTGGTGGTAGTGGTAGTGGTACAGGTGGTTATGTCTCTGATGTGGGGCGTGGTTCT

TCGATCAAGAGCTCAATATCAGCTTCTACTGACTCTTCACCAAAGGAGGGCTTGAAAGCATCCAAC
TTTTCTTTCGATGGCTCTCCTGAGGATCTTAGTAAGAAAGTGAAACCTTGGTCTAGAAATTCACCC
CCCATGGAGGCTTTGGTAGGCTCTGTTGAACCACTTATAGGTCTGAAACTTGGTAAGCGGACATTT
GAGACCATCGGTGGTGCTAAGATTTCATCCTTCCCCGAGAACCCCGTATCATCTGCTGCTGCAGCA
AAGAAAACAAAATCATCTACTCAAAATGCGCCAACTCCTCGCTGTCAGGTTGAAGGCTGCAACCTT
AATCTTTCATCAGCTTTGAATATTACTGGAAGCATCGAGTTTGTGACAGTCATTCCAAATGCCCAA
AGGCAACCTATGAATCTTGTTCTCAACCACACCCAACTGGTTCACTCTAGAGCTGCTGCAAAAAGC
ACTGAAGAAACTAAGTTCAGTATAACAAGAGGGTCTACTCCAAAGCCTGAAAGAGCTGGCAGTACA
AATGGGCAGTCATTACTGCCGGGGATCCAGTTTTCACGGGCCGTGGGTGCACACAGTAATGGCCAC
ACAGCCGAGGTTTTCAATCGACGTGCCAAGGAATCCATGCTCAAAATGGGTACAGGACCGGAATTT
CCTCGTGCTCTCTCTCTTCTGTCAACTAATTCATGGGGTTCATCCGAGCCTGAGACTGTTTCACTG
AACCACCCAACACATGCAAATCAGAGCAGCATGCCCGAGCAGATGATGCAGGCAATTCCACAAGGT
GTGCCACTTATGTCCTGTGAATACTGGCAGGGTGGACAACCTTCATCCGATCCTCGTGACCATACA
TTGGCTGCTAATAGTCATGCTTGCGTGATTCAAAGGAGTGAAAATGTAAGACAGAAGATATACCTT
CCTGAGGAGAATGGCAACGTCAGCTTGAGAGTCTTCAATGGACTGGCTGCCACACTCTGTACACCT
GACATTGTGGCTGATATTTCTTGCTCTTGCCTCTACCACCCTCTCCTTCTTCACCGAATCCTCTTC
CCCCTCCATAGATAG

In the present document, all the number identifications (number id) of the genes described herein, identified as "Nitab4.5_...", belong to the *Nicotiana tabacum* genome database "N. Tabacum v4.5 Scaffolds Edwards 2017" (https://solgenomics.net/).

The invention herein described relates to plants or part thereof, reproductive or propagating plant material or a plant cell, with an increased capacity for recombinant products (proteins, nucleic acids, or metabolites) production. A recombinant product refers to any biomolecule, either a protein, a nucleic acid, another type of biopolymer, or a metabolite, that is produced as a consequence of the transient or stable genetic modification of an organism with a recombinant nucleic acid. As used herein the expression "increased capacity for recombinant product production" refers to an increase, growth, improvement or enhancement of the production of a recombinant product in comparison to wild-type control plants. After agroinfiltration with an Agrobacterium strain carrying a plasmid for the expression of the recombinant product, the product production capacity can be determined with the following techniques: DNA or RNA extraction followed by quantitative PCR for nucleic acid quantification, extraction followed by liquid or gas chromatography or specific activity assays for metabolites quantification, or total protein extraction from the plant tissue followed by (i) two-dimensional electrophoresis and Western Blot with specific antibodies that recognize the protein of interest and densitometry, (ii) Enzyme-Linked Immunosorbent Assay (ELISA), also with specific antibodies for protein yield determination. Alternatively, the recombinant protein can be purified from the total protein extract and quantified by Bradford Assay or densitometry after two-dimensional electrophoresis followed by Coomassie staining or Western Blot.

In a preferred embodiment, the plant of the invention comprises an increased capacity for recombinant products production.

In other preferred embodiment, the plant of the invention comprises an increased capacity for recombinant products production, wherein the recombinant product is a protein, a nucleic acid, or a metabolite.

As the skilled person in the art knows, the plant of the invention could produce any metabolite if the precursor and the enzymes responsible for its biosynthesis are presented. Examples of plant metabolites include, without limitation to, terpenes (such as plant volatiles, terpene glycosides, carotenoids or sterols), phenolics (such as phenolic acids, coumarins, lignans, stilbenes, flavonoids, tannins or lignin) and nitrogen containing compounds (such as alkaloids or glucosinolates).

The plant of the invention comprises (a) at least one monoallelic heterozygous mutation, biallelic heterozygous mutation or biallelic homozygous mutation in the coding region of all SPL genes with nucleotide sequence SEQ ID NO: 1, SEQ ID NO: 2, SEQ ID NO: 4, SEQ ID NO: 6, SEQ ID NO: 7, SEQ ID NO: 8, SEQ ID NO: 9, SEQ ID NO: 12, and SEQ ID NO: 13, in combination with at least one monoallelic heterozygous mutation, biallelic heterozygous mutation or biallelic homozygous mutation in the coding region of at least one of the genes selected from the list consisting of: SEQ ID NO: 3, SEQ ID NO: 5, SEQ ID NO: 10, SEQ ID NO: 11, SEQ ID NO: 14, SEQ ID NO: 15, SEQ ID NO: 16, SEQ ID NO: 17 and any combination thereof.

The term "mutation" as used herein, refers to the insertion or deletion of one or several nucleotides in the coding region of a gene. Depending on the position and nature of the mutation they can be classified in frame-shift mutations that alter the amino acid sequence, non-sense mutations that create a stop codon, missense mutations that result in the addition, deletion or substitution of few amino acids without altering the open reading frame.

The term "monoallelic heterozygous mutation" as used herein, refers to a mutation in one allele of a single gene in diploid genome (paternal or maternal allele); only one allele carries the mutation, and the other allele is a wild type allele.

The term "biallelic heterozygous mutation" as used herein, refers to a biallelic mutations in both alleles of a single gene in diploid genome (paternal and maternal allele); the two alleles carry mutations, but the mutations are different for each of them.

The term "biallelic homozygous mutation" as used herein, refers to biallelic mutations having the same mutation in both copies (alleles) of a particular gene (both alleles carry the same mutation).

Additionally, and to further increase the yields derived from the combined mutations in SPL genes, the invention includes their combination with the gene FT5 from the Flowering-locus-T (FT) family, which is involved in the regulation of flowering time among other important biological processes. In tobacco, five different FT-like genes have been characterized: FT1, FT2 and FT3 encode flowering repressors while FT4 and FT5 encode flowering activators (Wang et al., 2018, Plant Physiol.;231:393-401. and Schmidt et al., 2020, Front Plant Sci.;10:1666).

In other preferred embodiment, the plant of the invention further comprises at least one biallelic heterozygous mutation or biallelic homozygous mutation in the genes:
- gene Nitab4.5_0000819g0010.1 (1a_FT5) comprising, or consisting of, nucleotide sequence SEQ ID NO: 18, and
- gene Nitab4.5_0000751g0180.1 (1b_FT5) comprising, or consisting of, nucleotide sequence SEQ ID NO: 19.
   SEQ ID NO: 18 
ATGCCAAGAGAACGTGAACCTCTGGTAGTTGGTCGAGTGATAGGGGATGTATTGGACCCTTTCACAAG
ATCTATTGGCCTAGGAGTTATTTATAGGGATAGAGAAGTTAATAATGGGTGTGAGCTTAGGCCTTCCC
AAGTTATTAACCAGCCCAGGGTTGAAGTTGGTGGAGATGACCTTCGTACCTTTTACACTCTGGTTATG
GTGGACCCTGATGCTCCAAGTCCAAGTGATCCAAATCTAAGAGAATACCTCCATTGGTTGGTCACTGA
TATTCCAGCTACCACAGGTGCAAGTTTTGGCCAGGAAATTGTGTGCTATGAAAGTCCAAGGCCAACAA
TGGGAATACATCGCTTTGTATTCGTATTGTTTAGACAATTGGGTCGACAGACAGTGTATGCTCCAGGA
TGGCGTCAGAATTTCAATACAAAAGATTTTGCTGAACTCTATAATCTTGGTTTACCAGTTGCTGCTGT
CTATTTTAATTGTCAAAGAGAGAGTGGCAGTGGTGGACGTAGAAGGTCTGCCGATTGA
   SEQ ID NO: 19

ATGCCAAGAGAACGTGAACCTCTGGTAGTTGGTCGAGTGATAGGGGATGTATTGGACCCTTTCACAAG
ATCTATTGGCCTAAGAGTTATTTATAGGGATAGAGAAGTTAATAATGGGTGTGAGCTTAGGCCTTCCC
AAGTTGTTAACCAGCCAAGGGTTGAAGTTGGTGGAGATGACCTTCGTACCTTTTACACTCTGGTTATG
GTGGACCCTGATGCTCCAAGTCCAAGATATCCAAATCTAAGAGAATACCTCCATTGGTTGGTCACTGA
TATTCCAGCTACCACAGGTGCAAGTTTTGGGCAAGAAATTGTGTGCTATGAAAGTCCAAGGCCAACAA
TGGGAATACATCGCTTTGTATTCGTATTGTTTAGACAATTGGGTCGACAGACAGTGTATGCTCCAGGA
TGGCGTCAGAATTTCAATACAAAAGACTTTGCTGAACTCTATAATCTTGGTTTACCGGTTGCTGCCGT
CTATTTTAATTGTCAAAGAGAGAGTGGCAGTGGTGGACGTAGAAGGTCTGCCGATTGA

### Mutations in SPL and FT genes

In other preferred embodiment, the plant of the invention comprises monoallelic and/or biallelic mutations in the coding region of SPL genes and/or FT5 gene with nucleotide sequence SEQ ID NO: 1 to SEQ ID NO: 19, preferably wherein the plant comprises an increased capacity for recombinant protein production, wherein the nucleotide "A" is adenine, "C" is cytosine, "G" is guanine and "T" is thymine, and wherein:
- the mutation in the nucleotide sequence SEQ ID NO: 1 is a deletion (del) of nucleotides ACAAC at position 353 (353delACAAC);
- the mutation in the nucleotide sequence SEQ ID NO: 2 is a deletion of nucleotides GGACACAA at position 349 (349deIGGACACAA), or a deletion of nucleotides AA at position 355 (355delAA);
- the mutation in the nucleotide sequence SEQ ID NO: 3 is an insertion (ins) of nucleotide A between position 141 and 142 (142insA), and/or a deletion of nucleotides CCATGCAAAGGCTCAA (SEQ ID NO: 131) at position 225 (225deICCATGCAAAGGCTCAA);
- the mutation in the nucleotide sequence SEQ ID NO: 4 is a deletion of the nucleotide C at position 213 (213delC), an insertion of nucleotide T between position 424 and 425 (425insT), and/or an insertion of nucleotide A between position 424 and 425 (425insA);
- the mutation in the nucleotide sequence SEQ ID NO: 5 is a deletion of the nucleotide C at position 225 (225delC), or an insertion of nucleotide A between position 224 and 225 (225insA);
- the mutation in the nucleotide sequence SEQ ID NO: 6 is a deletion of the nucleotides CA at position 204 (204deICA);
- the mutation in the nucleotide sequence SEQ ID NO: 7 is an insertion of nucleotide A between position 212 and 213 (213insA);
- the mutation in the nucleotide sequence SEQ ID NO: 8 is an insertion of nucleotide C between position 288 and 289 (289insC);
- the mutation in the nucleotide sequence SEQ ID NO: 9 is an insertion of nucleotide T between position 489 and 490 (490insT);
- the mutation in the nucleotide sequence SEQ ID NO: 10 is selected from the list consisting of:
   - a deletion of the nucleotides AAATGT at position 117 (117delAAATGT),
   - a deletion of the nucleotides GT at position 121 (121deIGT),
   - an insertion of nucleotide A between position 216 and 217 (217insA),
   - a deletion of nucleotides AG at position 217 and an insertion of nucleotide T between position 216 and 217 (217delAGinsT),
   - a deletion of nucleotides AG at position 217 (217delAG),
   - a deletion of nucleotide G at position 218 (218deIG), and
   - any combination thereof;
- the mutation in the nucleotide sequence SEQ ID NO: 11 is a deletion of nucleotide A at position 217 (217deIA), an insertion of nucleotide A between position 216 and 217 (217insA), or a deletion of nucleotides AG at position 217 (217delAG);
- the mutation in the nucleotide sequence SEQ ID NO: 12 is selected from the list consisting of:
   - a deletion of nucleotides TA at position 135 (135delTA),
   - a deletion of nucleotides TAAAGT at position 135 (135delTAAAGT),
   - a deletion of nucleotides GGCG at position 269 (269delGGCG),
   - a deletion of nucleotide G at position 272 (272deIG),
   - a deletion of nucleotide T at position 273 (273deIT),
   - an insertion of nucleotide A between position 273 and 274 (274insA),
   - an insertion of nucleotide T between position 273 and 274 (274insT), and
   - any combination thereof;
- the mutation in the nucleotide sequence SEQ ID NO: 13 is selected from the list consisting of:
   - an insertion of nucleotide T between position 122 and 123 (123insT),
   - a deletion of nucleotide T at position 123 (123deIT),
   - a deletion of nucleotides GTCGA at position 275 (275deIGTCGA),
   - an insertion of nucleotide T between position 276 and 277 (277insT),
   - an insertion of nucleotide C between position 276 and 277 (277insC),
   - an insertion of nucleotide A between position 276 and 277 (277insA),
   - an insertion of nucleotide G between position 276 and 277 (277insG), and
   - any combination thereof;
- the mutation in the nucleotide sequence SEQ ID NO: 14 is selected from the list consisting of:
   - a deletion of nucleotides GCA at position 13 (13delGCA),
   - a deletion of nucleotides CCA at position 338 (338deICCA),
   - a deletion of nucleotide C at position 598 (598delC),
   - a deletion of nucleotides CCACAGCAGGAAACCATCCAGTTCA (SEQ ID NO: 132) at position 733 and an insertion of nucleotides GC between position 732 and 733 (733deICCACAGCAGGAAACCATCCAGTTCAinsGC),
   - a deletion of nucleotides GCAGGAAACCATCCAGTTCA (SEQ ID NO: 133) at position 738 and insertion of nucleotides ATTTAGTTGCAATTTTTGTCAAGGATATCTACCTGCTAATAGCTTGCCT TTTAGCAATTTTCGCAGGAGGCTCTCTGATC (SEQ ID NO: 134) between position 737 and 738 (738delGCAGGAAACCATCCAGTTCAinsATTTAGTTGCAATTTTTGTCAA GGATATCTACCTGCTAATAGCTTGCCTTTTAGCAATTTTCGCAGGAGG CTCTCTGATC),
   - a deletion of nucleotides TTC at position 754 (754deITTC),
   - a deletion of nucleotides CA at position 756 (756deICA),
   - an insertion of nucleotide A between position 757 and 758 (758insA),
   - a deletion of nucleotide A at position 758 (758deIA), and
   - any combination thereof;
- the mutation in the nucleotide sequence SEQ ID NO: 15 is selected from the list consisting of:
   - a deletion of nucleotides GAGGCTCTCTGATCACAATGCACGACGCCGCAAACCACAGCAGGAAA CCATCCAGTTCA (SEQ ID NO: 135) at position 693 (693delGAGGCTCTCTGATCACAATGCACGACGCCGCAAACCACAGCA GGAAACCATCCAGTTCA),
   - a deletion of nucleotide G at position 693 (693deIG),
   - a deletion of nucleotides GA at position 693 (693deIGA),
   - a deletion of nucleotides GAG at position 693 (693delGAG),
   - an insertion of nucleotide T between position 692 and 693 (693insT),
   - a deletion of nucleotides AGTTCAACTCGGCA (SEQ ID NO: 136) at position 746 (746delAGTTCAACTCGGCA),
   - a deletion of nucleotides TC at position 749 (749deITC),
   - an insertion of nucleotide A between position 749 and 750 (750insA),
   - a deletion of nucleotide A at position 751 (751 delA),
   - an insertion of nucleotide A between position 751 and 752 (752insA),
   - an insertion of nucleotide G between position 751 and 752 (752insG),
   - an insertion of nucleotide T between position 751 and 752 (752insT), and
   - any combination thereof;
- the mutation in the nucleotide sequence SEQ ID NO: 16 is an insertion of nucleotide A between position 192 and 193 (193insA);
- the mutation in the nucleotide sequence SEQ ID NO: 17 is a deletion of nucleotides CACCCCCCA at position 458 (458delCACCCCCCA);
- the mutation in the nucleotide sequence SEQ ID NO: 18 is selected from the list consisting of:
   - a deletion of nucleotides TCCATTGG at position 254 (254deITCCATTGG),
   - an insertion of nucleotide T between position 257 and 258 (258insT),
   - an insertion of nucleotide A between position 257 and 258 (258insA), and
   - an insertion of nucleotide C between position 257 and 258 (258insC);
   **and/or**
- the mutation in the nucleotide sequence SEQ ID NO: 19 is selected from the list consisting of:
   - an insertion of nucleotide T between position 257 and 258 (258insT),
   - an insertion of nucleotides AT between position 257 and 258 (258insAT), and
   - an insertion of nucleotide C between position 257 and 258 (258insC).

### mSPL2 line

In other preferred embodiment, the plant of the invention (also discloses as the mSPL2 line) comprises:
**(a)** a biallelic homozygous mutation in the coding region of all following genes (the same mutation is in both alleles of the gene):
   - gene Nitab4.5_0001752g0040.1 (NtSPL3/4/5_2a) comprising, or consisting of, nucleotide sequence SEQ ID NO: 1, wherein the mutation in the nucleotide sequence SEQ ID NO: 1 is a deletion of nucleotides ACAAC at position 353 (353delACAAC);
   - gene Nitab4.5_0003348g0050.1 (NtSPL3/4/5_6a) comprising, or consisting of, nucleotide sequence SEQ ID NO: 4, wherein the mutation in the nucleotide sequence SEQ ID NO: 4 is a deletion of the nucleotide C at position 213 (213delC);
   - gene Nitab4.5_0007487g0020.1 (NtSPL3/4/5_7a) comprising, or consisting of, nucleotide sequence SEQ ID NO: 6, wherein the mutation in the nucleotide sequence SEQ ID NO: 6 is a deletion of the nucleotides CA at position 204 (204deICA);
   - gene Nitab4.5_0002219g0060.1 (NtSPL3/4/5_7b) comprising, or consisting of, nucleotide sequence SEQ ID NO: 7, wherein the mutation in the nucleotide sequence SEQ ID NO: 7 is an insertion of nucleotide A between position 212 and 213 (213insA);
   - gene Nitab4.5_0003572g0010.1 (NtSPL9/15_1a) comprising, or consisting of, nucleotide sequence SEQ ID NO: 8, wherein the mutation in the nucleotide sequence SEQ ID NO: 8 is an insertion of nucleotide C between position 288 and 289 (289insC);
   - gene Nitab4.5_0000016g0300.1 (NtSPL9/15_1b) comprising, or consisting of, nucleotide sequence SEQ ID NO: 9, wherein the mutation in the nucleotide sequence SEQ ID NO: 9 is an insertion of nucleotide T between position 489 and 490 (490insT); and
   - gene Nitab4.5_0001315g0110.1 (NtSPL2/10/11_3a) comprising, or consisting of, nucleotide sequence SEQ ID NO: 16, wherein the mutation in the nucleotide sequence SEQ ID NO: 16 is an insertion of nucleotide A between position 192 and 193 (193insA);
   and
(**b**) a biallelic heterozygous mutation in the coding region of all following genes (both alleles of the gene are mutated with different mutations):
   - gene Nitab4.5_0000638g0040.1 (NtSPL3/4/5_2b) comprising, or consisting of, nucleotide sequence SEQ ID NO: 2, wherein the mutation in the nucleotide sequence SEQ ID NO: 2 is a deletion of nucleotides AA at position 355 (355delAA) and a deletion of nucleotides GGACACAA at position 349 (349deIGGACACAA);
   - gene Nitab4.5_0001538g0080.1 (NtSPL9/15_2a) comprising, or consisting of, nucleotide sequence SEQ ID NO: 10, wherein the mutation in the nucleotide sequence SEQ ID NO: 10 is a deletion of the nucleotides AAATGT at position 117 (117delAAATGT); a deletion of nucleotides AG at position 217 and an insertion of nucleotide T between position 216 and 217 (217delAGinsT); and a deletion of nucleotides AG at position 217 (217delAG);
   - gene Nitab4.5_0002299g0030.1 (NtSPL13_1a) comprising, or consisting of, nucleotide sequence SEQ ID NO: 12, wherein the mutation in the nucleotide sequence SEQ ID NO: 12 is an insertion of nucleotide A between position 273 and 274 (274insA) and an insertion of nucleotide T between position 273 and 274 (274insT);
   - gene Nitab4.5_0001010g0010.1 (NtSPL13_1b) comprising, or consisting of, nucleotide sequence SEQ ID NO: 13, wherein the mutation in the nucleotide sequence SEQ ID NO: 13 is an insertion of nucleotide T between position 276 and 277 (277insT) and an insertion of nucleotide C between position 276 and 277 (277insC);
   - gene Nitab4.5_0003900g0020.1 (NtSPL2/10/11_1a) comprising, or consisting of, nucleotide sequence SEQ ID NO: 14, wherein the mutation in the nucleotide sequence SEQ ID NO: 14 is a deletion of nucleotides TTC at position 754 (754delTTC) and a deletion of nucleotides CA at position 756 (756deICA);
   - gene Nitab4.5_0000861g0050.1 (NtSPL2/10/11_1b) comprising, or consisting of, nucleotide sequence SEQ ID NO: 15, wherein the mutation in the nucleotide sequence SEQ ID NO: 15 is a deletion of nucleotides GAGGCTCTCTGATCACAATGCACGACGCCGCAAACCACAGCAGGAAACCAT CCAGTTCA at position 693 (693delGAGGCTCTCTGATCACAATGCACGACGCCGCAAACCACAGCAGGAA ACCATCCAGTTCA); a deletion of nucleotides GAG at position 693 (693delGAG); and a deletion of nucleotide A at position 751 (751deIA);
   and
**(c)** a monoallelic heterozygous mutation in the coding region of the following genes (the mutation is in one of the alleles of the gene):
   - gene Nitab4.5_0000991g0020.1 (2b_SPL9/15) comprising, or consisting of, nucleotide sequence SEQ ID NO: 11, wherein the mutation in the nucleotide sequence SEQ ID NO: 11 is an insertion of nucleotide A between position 216 and 217 (217insA); and
   - gene Nitab4.5_0007217g0040.1 (NtSPL2/10/11_3b) comprising, or consisting of, nucleotide sequence SEQ ID NO: 17, wherein the mutation in the nucleotide sequence SEQ ID NO: 17 is a deletion of nucleotides CACCCCCCA at position 458 (458delCACCCCCCA);
   **and**
**(d)** a monoallelic heterozygous mutation or a biallelic heterozygous mutation, wherein the mutation is in the coding region or in the region out of the coding region of the gene:
   - gene Nitab4.5_0003942g0050.1 (NtSPL3/4/5_6b) comprising, or consisting of, nucleotide sequence SEQ ID NO: 5, wherein the mutation in the nucleotide sequence SEQ ID NO: 5 is a deletion of the nucleotide C at position 225 (225delC) and/or a mutation which is an insertion of nucleotide T between position 436 and 437 (437insT).

In other more preferred embodiment of the plant of the invention (the mSPL2 line), the gene Nitab4.5_0003942g0050.1 (NtSPL3/4/5_6b) comprising, or consisting of, nucleotide sequence SEQ ID NO: 5, wherein the monoallelic heterozygous mutation in the nucleotide sequence SEQ ID NO: 5 is a deletion of the nucleotide C at position 225 (225delC) and a mutation which is an insertion of nucleotide T between position 436 and 437 (437insT).

In other more preferred embodiment of the plant of the invention (the mSPL2 line), the gene Nitab4.5_0003942g0050.1 (NtSPL3/4/5_6b) comprising, or consisting of, nucleotide sequence SEQ ID NO: 5, wherein the biallelic heterozygous mutation in the nucleotide sequence SEQ ID NO: 5 is a deletion of the nucleotide C at position 225 (225delC) in one of the alleles and a mutation which is an insertion of nucleotide T between position 436 and 437 (437insT) in the other allele.

In other more preferred embodiment, the plant of the invention (mSPL2 line) comprising, or consisting of:
- the two alleles of the gene Nitab4.5_0001752g0040.1 (NtSPL3/4/5_2a) comprising, or consisting of, nucleotide sequence SEQ ID NO: 20 (sequence with the mutation 353delACAAC) that generates an early stop codon at position 418;
- one allele of the gene Nitab4.5_0000638g0040.1 (NtSPL3/4/5_2b) comprising, or consisting of, nucleotide sequence SEQ ID NO: 22 (sequence with the mutation 355delAA) that generates an early stop codon at position 397, and the other allele comprising, or consisting of, nucleotide sequence SEQ ID NO: 21 (sequence with the mutation 349deIGGACACAA) that generates an early stop codon at position 391;
- the two alleles of the gene Nitab4.5_0003348g0050.1 (NtSPL3/4/5_6a) comprising, or consisting of, nucleotide sequence SEQ ID NO: 25 (sequence with the mutation 213delC) that results in a frameshift and a delayed stop codon;
- one allele of the gene Nitab4.5_0003942g0050.1 (NtSPL3/4/5_6b) comprising, or consisting of, nucleotide sequence SEQ ID NO: 28 (sequence with the mutation 437insT) not affecting the coding sequence and the other allele comprising, or consisting of, nucleotide sequence SEQ ID NO: 29 (sequence with the mutation 225deIC) that results in a frameshift and a delayed stop codon; or one allele of the gene Nitab4.5_0003942g0050.1 (NtSPL3/4/5_6b) comprising, or consisting of, nucleotide sequence SEQ ID NO: 5 and other allele comprising, or consisting of, nucleotide sequence SEQ ID NO: 31 (sequence with the mutation 225delC and 437insT) that results in a frameshift and a delayed stop codon,
- the two alleles of the gene Nitab4.5_0007487g0020.1 (NtSPL3/4/5_7a) comprising, or consisting of, nucleotide sequence SEQ ID NO: 32 (sequence with the mutation 204deICA) that generates an early stop codon at position 244;
- the two alleles of the gene Nitab4.5_0002219g0060.1 (NtSPL3/4/5_7b) comprising, or consisting of, nucleotide sequence SEQ ID NO: 33 (sequence with the mutation 213insA) that generates an early stop codon at position 256;
- the two alleles of the gene Nitab4.5_0003572g0010.1 (NtSPL9/15_1a) comprising, or consisting of, nucleotide sequence SEQ ID NO: 34 (sequence with the mutation 289insC) that generates an early stop codon at position 301;
- the two alleles of the gene Nitab4.5_0000016g0300.1 (NtSPL9/15_1b) comprising, or consisting of, nucleotide sequence SEQ ID NO: 35 (sequence with the mutation 490insT) that generates an early stop codon at position 502;
- one allele of the gene Nitab4.5_0001538g0080.1 (2a_SPL9/15) comprising, or consisting of, nucleotide sequence SEQ ID NO: 37 (sequence with the mutation 217delAGinsT) that generates an early stop codon at position 241, and the other allele comprising, or consisting of, nucleotide sequence SEQ ID NO: 38 (sequence with the mutations 117delAAATGT and 217deIAG) that generates an early stop codon at position 220;
- one allele of the gene Nitab4.5_0000991g0020.1 (2b_SPL9/15) comprising, or consisting of, nucleotide sequence SEQ ID NO: 11, and the other allele comprising, or consisting of, nucleotide sequence SEQ ID NO: 42 (sequence with the mutation 217insA) that generates an early stop codon at position 229;
- one allele of the gene Nitab4.5_0002299g0030.1 (1a_SPL13) comprising, or consisting of, nucleotide sequence SEQ ID NO: 44 (sequence with the mutation 274insA) that generates an early stop codon at position 358, and the other allele comprising, or consisting of, nucleotide sequence SEQ ID NO: 45 (sequence with the mutation 274insT) that generates an early stop codon at position 358;
- one allele of the gene Nitab4.5_0001010g0010.1 (1b_SPL13) comprising, or consisting of, nucleotide sequence SEQ ID NO: 51 (sequence with the mutation 277insT) that generates an early stop codon at position 295 and the other allele comprising, or consisting of, nucleotide sequence SEQ ID NO: 52 (sequence with the mutation 277insC) that generates an early stop codon at position 295;
- one allele of the gene Nitab4.5_0003900g0020.1 (1a_SPL2/10/11) comprising, or consisting of, nucleotide sequence SEQ ID NO: 60 (sequence with the mutation 754deITTC) that results in the loss of one amino acid and the other allele comprising, or consisting of, nucleotide sequence SEQ ID NO: 61 (sequence with the mutation 756deICA) that generates an early stop codon at position 784;
- one allele of the gene Nitab4.5_0000861g0050.1 (1b_SPL2/10/11) comprising, or consisting of, nucleotide sequence SEQ ID NO: 71 (sequence with the mutation
   693delGAGGCTCTCTGATCACAATGCACGACGCCGCAAACCACAGCAGGA AACCATCCAGTTCA) that generates an early stop codon at position 721, and the other allele comprising, or consisting of, nucleotide sequence SEQ ID NO: 72 (sequence with the mutations 693delGAG and 751delA) that generates an early stop codon at position 793;
- the two alleles of the gene Nitab4.5_0001315g0110.1 (NtSPL2/10/11_3a) comprising, or consisting of, nucleotide sequence SEQ ID NO: 83 (sequence with the mutation 193insA) that generates an early stop codon at position 229; and
- one allele of the gene Nitab4.5_0007217g0040.1 (NtSPL2/10/11_3b) comprising, or consisting of, nucleotide sequence SEQ ID NO: 17 and the other allele comprising, or consisting of, nucleotide sequence SEQ ID NO: 84 (sequence with the mutation 458delCACCCCCCA) that results in the loss of three amino acids.

### mSPL3 line

In other preferred embodiment, the plant of the invention (also discloses as the mSPL3 line) comprises:
**(a)** a biallelic homozygous mutation in the coding region of all following genes (the same mutation is in both alleles of the gene):
   - gene Nitab4.5_0001752g0040.1 (NtSPL3/4/5_2a) comprising, or consisting of, nucleotide sequence SEQ ID NO: 1, wherein the mutation in the nucleotide sequence SEQ ID NO: 1 is a deletion of nucleotides ACAAC at position 353 (353delACAAC);
   - gene Nitab4.5_0003348g0050.1 (NtSPL3/4/5_6a) comprising, or consisting of, nucleotide sequence SEQ ID NO: 4, wherein the mutation in the nucleotide sequence SEQ ID NO: 4 is a deletion of the nucleotide C at position 213 (213delC);
   - gene Nitab4.5_0007487g0020.1 (NtSPL3/4/5_7a) comprising, or consisting of, nucleotide sequence SEQ ID NO: 6, wherein the mutation in the nucleotide sequence SEQ ID NO: 6 is a deletion of the nucleotides CA at position 204 (204deICA);
   - gene Nitab4.5_0002219g0060.1 (NtSPL3/4/5_7b) comprising, or consisting of, nucleotide sequence SEQ ID NO: 7, wherein the mutation in the nucleotide sequence SEQ ID NO: 7 is an insertion of nucleotide A between position 212 and 213 (213insA);
   - gene Nitab4.5_0003572g0010.1 (NtSPL9/15_1a) comprising, or consisting of, nucleotide sequence SEQ ID NO: 8, wherein the mutation in the nucleotide sequence SEQ ID NO: 8 is an insertion of nucleotide C between position 288 and 289 (289insC);
   - gene Nitab4.5_0000016g0300.1 (NtSPL9/15_1b) comprising, or consisting of, nucleotide sequence SEQ ID NO: 9, wherein the mutation in the nucleotide sequence SEQ ID NO: 9 is an insertion of nucleotide T between position 489 and 490 (490insT);
   - gene Nitab4.5_0001010g0010.1 (NtSPL13_1b) comprising, or consisting of, nucleotide sequence SEQ ID NO: 13, wherein the mutation in the nucleotide sequence SEQ ID NO: 13 is an insertion of nucleotide A between position 276 and 277 (277insA); and
   - gene Nitab4.5_0000861g0050.1 (NtSPL2/10/11_1b) comprising, or consisting of, nucleotide sequence SEQ ID NO: 15, wherein the mutation in the nucleotide sequence SEQ ID NO: 15 is an insertion of nucleotide T between position 692 and 693 (693insT);
   and
**(b)** a biallelic heterozygous mutation in the coding region of all following genes (both alleles of the gene are mutated with different mutations):
   - gene Nitab4.5_0000638g0040.1 (NtSPL3/4/5_2b) comprising, or consisting of, nucleotide sequence SEQ ID NO: 2, wherein the mutation in the nucleotide sequence SEQ ID NO: 2 is a deletion of nucleotides AA at position 355 (355delAA) and a deletion of nucleotides GGACACAA at position 349 (349deIGGACACAA); and
   - gene Nitab4.5_0002299g0030.1 (NtSPL13_1a) comprising, or consisting of, nucleotide sequence SEQ ID NO: 12, wherein the mutation in the nucleotide sequence SEQ ID NO: 12 is an insertion of nucleotide A between position 273 and 274 (274insA) and an insertion of nucleotide T between position 273 and 274 (274insT);
   **and**
**(c)** a monoallelic heterozygous mutation in the coding region of the following gene (the mutation is in one of the alleles of the gene):
   - gene Nitab4.5_0004959g0040.1 (NtSPL3/4/5_4b) comprising, or consisting of, nucleotide sequence SEQ ID NO: 3, wherein the mutation in the nucleotide sequence SEQ ID NO: 3 is an insertion (ins) of nucleotide A between position 141 and 142 (142insA).

In other more preferred embodiment, the plant of the invention (mSPL3 line) comprising, or consisting of:
- the two alleles of the gene Nitab4.5_0001752g0040.1 (NtSPL3/4/5_2a) comprising, or consisting of, nucleotide sequence SEQ ID NO: 20 (sequence with the mutation 353delACAAC) that generates an early stop codon at position 418;
- one allele of the gene Nitab4.5_0000638g0040.1 (NtSPL3/4/5_2b) comprising, or consisting of, nucleotide sequence SEQ ID NO: 22 (sequence with the mutation 355delAA) that generates an early stop codon at position 397, and the other allele comprising, or consisting of, nucleotide sequence SEQ ID NO: 21 (sequence with the mutation 349deIGGACACAA) that generates an early stop codon at position 391;
- one allele of the gene Nitab4.5_0004959g0040.1 (NtSPL3/4/5_4b) comprising, or consisting of, nucleotide sequence SEQ ID NO: 3, and the other allele comprising, or consisting of, nucleotide sequence SEQ ID NO: 23 (sequence with the mutation 142insA) that generates an early stop codon at position 160;
- the two alleles of the gene Nitab4.5_0003348g0050.1 (NtSPL3/4/5_6a) comprising, or consisting of, nucleotide sequence SEQ ID NO: 25 (sequence with the mutation 213delC) that results in a frameshift and a delayed stop codon;
- the two alleles of the gene Nitab4.5_0007487g0020.1 (NtSPL3/4/5_7a) comprising, or consisting of, nucleotide sequence SEQ ID NO: 32 (sequence with the mutation 204deICA) that generates an early stop codon at position 244;
- the two alleles of the gene Nitab4.5_0002219g0060.1 (NtSPL3/4/5_7b) comprising, or consisting of, nucleotide sequence SEQ ID NO: 33 (sequence with the mutation 213insA) that generates an early stop codon at position 256;
- the two alleles of the gene Nitab4.5_0003572g0010.1 (NtSPL9/15_1a) comprising, or consisting of, nucleotide sequence SEQ ID NO: 34 (sequence with the mutation 289insC) that generates an early stop codon at position 301;
- the two alleles of the gene Nitab4.5_0000016g0300.1 (NtSPL9/15_1b) comprising, or consisting of, nucleotide sequence SEQ ID NO: 35 (sequence with the mutation 490insT) that generates an early stop codon at position 502;
- one allele of the gene Nitab4.5_0002299g0030.1 (1a_SPL13) comprising, or consisting of, nucleotide sequence SEQ ID NO: 44 (sequence with the mutation 274insA) that generates an early stop codon at position 358, and the other allele comprising, or consisting of, nucleotide sequence SEQ ID NO: 45 (sequence with the mutation 274insT) that generates an early stop codon at position 358;
- the two alleles of the gene Nitab4.5_0001010g0010.1 (1b_SPL13) comprising, or consisting of, nucleotide sequence SEQ ID NO: 53 (sequence with the mutation 277insA) that generates an early stop codon at position 295;
   and
- the two alleles of the gene Nitab4.5_0000861g0050.1 (1b_SPL2/10/11) comprising, or consisting of, nucleotide sequence SEQ ID NO: 73 (sequence with the mutations 693insT) that generates an early stop codon at position 703.

### mSPL4 line

In other preferred embodiment, the plant of the invention (also discloses as the mSPL4 line) comprises:
**(a)** a biallelic homozygous mutation in the coding region of all following genes (the same mutation is in both alleles of the gene):
   - gene Nitab4.5_0001752g0040.1 (NtSPL3/4/5_2a) comprising, or consisting of, nucleotide sequence SEQ ID NO: 1, wherein the mutation in the nucleotide sequence SEQ ID NO: 1 is a deletion of nucleotides ACAAC at position 353 (353delACAAC);
   - gene Nitab4.5_0000638g0040.1 (NtSPL3/4/5_2b) comprising, or consisting of, nucleotide sequence SEQ ID NO: 2, wherein the mutation in the nucleotide sequence SEQ ID NO: 2 is a deletion of nucleotides AA at position 355 (355delAA);
   - gene Nitab4.5_0003348g0050.1 (NtSPL3/4/5_6a) comprising, or consisting of, nucleotide sequence SEQ ID NO: 4, wherein the mutation in the nucleotide sequence SEQ ID NO: 4 is a deletion of the nucleotide C at position 213 (213delC);
   - gene Nitab4.5_0007487g0020.1 (NtSPL3/4/5_7a) comprising, or consisting of, nucleotide sequence SEQ ID NO: 6, wherein the mutation in the nucleotide sequence SEQ ID NO: 6 is a deletion of the nucleotides CA at position 204 (204deICA);
   - gene Nitab4.5_0002219g0060.1 (NtSPL3/4/5_7b) comprising, or consisting of, nucleotide sequence SEQ ID NO: 7, wherein the mutation in the nucleotide sequence SEQ ID NO: 7 is an insertion of nucleotide A between position 212 and 213 (213insA);
   - gene Nitab4.5_0003572g0010.1 (NtSPL9/15_1a) comprising, or consisting of, nucleotide sequence SEQ ID NO: 8, wherein the mutation in the nucleotide sequence SEQ ID NO: 8 is an insertion of nucleotide C between position 288 and 289 (289insC);
   - gene Nitab4.5_0000016g0300.1 (NtSPL9/15_1b) comprising, or consisting of, nucleotide sequence SEQ ID NO: 9, wherein the mutation in the nucleotide sequence SEQ ID NO: 9 is an insertion of nucleotide T between position 489 and 490 (490insT); and
   - gene Nitab4.5_0001010g0010.1 (NtSPL13_1b) comprising, or consisting of, nucleotide sequence SEQ ID NO: 13, wherein the mutation in the nucleotide sequence SEQ ID NO: 13 is an insertion of nucleotide A between position 276 and 277 (277insA);
   and
**(b)** a biallelic heterozygous mutation in the coding region of all following genes (both alleles of the gene are mutated with different mutations):
   - gene Nitab4.5_0002299g0030.1 (NtSPL13_1a) comprising, or consisting of, nucleotide sequence SEQ ID NO: 12, wherein the mutation in the nucleotide sequence SEQ ID NO: 12 is an insertion of nucleotide A between position 273 and 274 (274insA) is in one allele and a deletion of nucleotide G at position 272 (272deIG) in the second allele; and
   - gene Nitab4.5_0003900g0020.1 (NtSPL2/10/11_1a) comprising, or consisting of, nucleotide sequence SEQ ID NO: 14, wherein the mutation in the nucleotide sequence SEQ ID NO: 14 is a deletion of nucleotides GCA at position 13 (13delGCA), a deletion of nucleotide C at position 598 (598delC), and an insertion of nucleotide A between position 757 and 758 (758insA);
   **and**
**(c)** a monoallelic heterozygous mutation in the coding region of the following genes (the mutation is in one of the alleles of the gene):
   - gene Nitab4.5_0004959g0040.1 (NtSPL3/4/5_4b) comprising, or consisting of, nucleotide sequence SEQ ID NO: 3, wherein the mutation in the nucleotide sequence SEQ ID NO: 3 is an insertion (ins) of nucleotide A between position 141 and 142 (142insA) and a deletion of nucleotides CCATGCAAAGGCTCAA at position 225 (225deICCATGCAAAGGCTCAA);
   - gene Nitab4.5_0003348g0050.1 (NtSPL3/4/5_6a) comprising, or consisting of, nucleotide sequence SEQ ID NO: 4, wherein the mutation in the nucleotide sequence SEQ ID NO: 4 is an insertion of nucleotide A between position 424 and 425 (425insA);
   - gene Nitab4.5_0003942g0050.1 (NtSPL3/4/5_6b) comprising, or consisting of, nucleotide sequence SEQ ID NO: 5, wherein the mutation in the nucleotide sequence SEQ ID NO: 5 is a deletion of the nucleotide C at position 225 (225delC);
   - gene Nitab4.5_0001538g0080.1 (NtSPL9/15_2a) comprising, or consisting of, nucleotide sequence SEQ ID NO: 10, wherein the mutation in the nucleotide sequence SEQ ID NO: 10 is an insertion of nucleotide A between position 216 and 217 (217insA);
      and
   - gene Nitab4.5_0000861g0050.1 (NtSPL2/10/11_1b) comprising, or consisting of, nucleotide sequence SEQ ID NO: 15, wherein the mutation in the nucleotide sequence SEQ ID NO: 15 is an insertion of nucleotide A between position 751 and 752 (752insA).

In other more preferred embodiment of the plant of the invention (also discloses as the mSPL4 line), the gene Nitab4.5_0003900g0020.1 (NtSPL2/10/11_1a) comprising, or consisting of, nucleotide sequence SEQ ID NO: 14, wherein the biallelic heterozygous mutation in the nucleotide sequence SEQ ID NO: 14 is:
(i) a deletion of nucleotides GCA at position 13 (13delGCA) and a deletion of nucleotide C at position 598 (598deIC) in one of the alleles of the gene and an insertion of nucleotide A between position 757 and 758 (758insA) in the other of the alleles of the gene; or
(ii) a deletion of nucleotides GCA at position 13 (13delGCA) and an insertion of nucleotide A between position 757 and 758 (758insA) in one of the alleles of the gene and a deletion of nucleotide C at position 598 (598delC) in the other of the alleles of the gene.

In other more preferred embodiment, the plant of the invention (mSPL4 line) comprising, or consisting of:
- the two alleles of the gene Nitab4.5_0001752g0040.1 (NtSPL3/4/5_2a) comprising, or consisting of, nucleotide sequence SEQ ID NO: 20 (sequence with the mutation 353delACAAC) that generates an early stop codon at position 418;
- the two alleles of the gene Nitab4.5_0000638g0040.1 (NtSPL3/4/5_2b) comprising, or consisting of, nucleotide sequence SEQ ID NO: 22 (sequence with the mutation 355delAA) that generates an early stop codon at position 397;
- one allele of the gene Nitab4.5_0004959g0040.1 (NtSPL3/4/5_4b) comprising, or consisting of, nucleotide sequence SEQ ID NO: 3, and the other allele comprising, or consisting of, nucleotide sequence SEQ ID NO: 24 (sequence with the mutation 142insA+225deICCATGCAAAGGCTCAA) that generates an early stop codon at position 160;
- one allele of the gene Nitab4.5_0003348g0050.1 (NtSPL3/4/5_6a) comprising, or consisting of, nucleotide sequence SEQ ID NO: 25 (sequence with the mutation 213delC) that results in a frameshift and a delayed stop codon and the other allele comprising, or consisting of, nucleotide sequence SEQ ID NO: 27 (sequence with the mutation 213delC + 425insA) that results in a frameshift and a delayed stop codon;
- one allele of the gene Nitab4.5_0003942g0050.1 (NtSPL3/4/5_6b) comprising, or consisting of, nucleotide sequence SEQ ID NO: 5, and the other allele comprising, or consisting of, nucleotide sequence SEQ ID NO: 29 (sequence with the mutation 225deIC) that results in a frameshift and a delayed stop codon;
- the two alleles of the gene Nitab4.5_0007487g0020.1 (NtSPL3/4/5_7a) comprising, or consisting of, nucleotide sequence SEQ ID NO: 32 (sequence with the mutation 204deICA) that generates an early stop codon at position 244;
- the two alleles of the gene Nitab4.5_0002219g0060.1 (NtSPL3/4/5_7b) comprising, or consisting of, nucleotide sequence SEQ ID NO: 33 (sequence with the mutation 213insA) that generates an early stop codon at position 256;
- the two alleles of the gene Nitab4.5_0003572g0010.1 (NtSPL9/15_1a) comprising, or consisting of, nucleotide sequence SEQ ID NO: 34 (sequence with the mutation 289insC) that generates an early stop codon at position 301;
- the two alleles of the gene Nitab4.5_0000016g0300.1 (NtSPL9/15_1b) comprising, or consisting of, nucleotide sequence SEQ ID NO: 35 (sequence with the mutation 490insT) that generates an early stop codon at position 502;
- one allele of the gene Nitab4.5_0001538g0080.1 (2a_SPL9/15) comprising, or consisting of, nucleotide sequence SEQ ID NO: 10, and the other allele comprising, or consisting of, nucleotide sequence SEQ ID NO: 36 (sequence with the mutation 217insA) that generates an early stop codon at position 229;
- one allele of the gene Nitab4.5_0002299g0030.1 (1a_SPL13) comprising, or consisting of, nucleotide sequence SEQ ID NO: 44 (sequence with the mutation 274insA) that generates an early stop codon at position 358, and the other allele comprising, or consisting of, nucleotide sequence SEQ ID NO: 46 (sequence with the mutation 272deIG) that generates an early stop codon at position 400;
- the two alleles of the gene Nitab4.5_0001010g0010.1 (1b_SPL13) comprising, or consisting of, nucleotide sequence SEQ ID NO: 53 (sequence with the mutation 277insA) that generates an early stop codon at position 295;
- the two alleles gene Nitab4.5_0003900g0020.1 (1a_SPL2/10/11) comprising, or consisting of, nucleotide sequence selected from de list consisting of: SEQ ID NO: 62 (sequence with the mutation 13deIGCA+598deIC) that generates an early stop codon at position 607, SEQ ID NO: 63 (sequence with the mutation 13deIGCA+758insA) that generates an early stop codon at position 784, SEQ ID NO: 64 (sequence with the mutation 598delC) that generates an early stop codon at position 610, SEQ ID NO: 65 (sequence with the mutation 758insA) that generates an early stop codon at position 787 and any combination thereof; preferably comprising, or consisting of, SEQ ID NO: 62 and 65, or, comprising, or consisting of, SEQ ID NO: 63 and 64;
   and
- one allele of the gene Nitab4.5_0000861g0050.1 (1b_SPL2/10/11) comprising, or consisting of, nucleotide sequence SEQ ID NO: 15, and the other allele comprising, or consisting of, nucleotide sequence SEQ ID NO: 74 (sequence with the mutation 752insA) that generates an early stop codon at position 781.

### mFTSPLa line

In other preferred embodiment, the plant of the invention (also discloses as the mFTSPLa line) comprises:
**(a)** a biallelic homozygous mutation in the coding region of all following genes (the same mutation is in both alleles of the gene):
   - gene Nitab4.5_0001752g0040.1 (2a_SPL3/4/5) comprising, or consisting of, nucleotide sequence SEQ ID NO: 1, wherein the mutation in the nucleotide sequence SEQ ID NO: 1 is a deletion of nucleotides ACAAC at position 353 (353delACAAC);
   - gene Nitab4.5_0003348g0050.1 (6a_SPL3/4/5) comprising, or consisting of, nucleotide sequence SEQ ID NO: 4, wherein the mutation in the nucleotide sequence SEQ ID NO: 4 is a deletion of the nucleotide C at position 213 (213delC);
   - gene Nitab4.5_0007487g0020.1 (7a_SPL3/4/5) comprising, or consisting of, nucleotide sequence SEQ ID NO: 6, wherein the mutation in the nucleotide sequence SEQ ID NO: 6 is a deletion of the nucleotides CA at position 204 (204deICA);
   - gene Nitab4.5_0002219g0060.1 (SPL3/4/5) comprising, or consisting of, nucleotide sequence SEQ ID NO: 7, wherein the mutation in the nucleotide sequence SEQ ID NO: 7 is an insertion of nucleotide A between position 212 and 213 (213insA);
   - gene Nitab4.5_0003572g0010.1 (1a_SPL9/15) comprising, or consisting of, nucleotide sequence SEQ ID NO: 8, wherein the mutation in the nucleotide sequence SEQ ID NO: 8 is an insertion of nucleotide C between position 288 and 289 (289insC);
   - gene Nitab4.5_0000016g0300.1 (1b_SPL9/15) comprising, or consisting of, nucleotide sequence SEQ ID NO: 9, wherein the mutation in the nucleotide sequence SEQ ID NO: 9 is an insertion of nucleotide T between position 489 and 490 (490insT);
   - gene Nitab4.5_0001010g0010.1 (1b_SPL13) comprising, or consisting of, nucleotide sequence SEQ ID NO: 13, wherein the mutation in the nucleotide sequence SEQ ID NO: 13 is an insertion of nucleotide T between position 276 and 277 (277insT);
      and
   - gene Nitab4.5_0000861g0050.1 (1b_SPL2/10/11) comprising, or consisting of, nucleotide sequence SEQ ID NO: 15, wherein the mutation in the nucleotide sequence SEQ ID NO: 15 is a deletion of nucleotide G at position 693 (693deIG) and an insertion of nucleotide A between position 751 and 752 (752insA);
   and
**(b)** a biallelic heterozygous mutation in the coding region of all following genes (both alleles of the gene are mutated with different mutations):
   - gene Nitab4.5_0000638g0040.1 (2b_SPL3/4/5) comprising, or consisting of, nucleotide sequence SEQ ID NO: 2, wherein the mutation in the nucleotide sequence SEQ ID NO: 2 is a deletion of nucleotides AA at position 355 (355delAA) or a deletion of nucleotides GGACACAA at position 349 (349deIGGACACAA);
   - gene Nitab4.5_0002299g0030.1 (1a_SPL13) comprising, or consisting of, nucleotide sequence SEQ ID NO: 12, wherein the mutation in the nucleotide sequence SEQ ID NO: 12 is a deletion of nucleotides G at position 272 (272deIG) or a deletion of nucleotides GGCG at position 269 (269delGGCG);
   - gene Nitab4.5_0000819g0010.1 (1a_FT5) comprising, or consisting of, nucleotide sequence SEQ ID NO: 18, wherein the mutation in the nucleotide sequence SEQ ID NO: 18 is an insertion of nucleotide A between position 257 and 258 (258insA) or an insertion of nucleotide C between position 257 and 258 (258insC);
      and
   - gene Nitab4.5_0000751g0180.1 (1b_FT5) comprising, or consisting of, nucleotide sequence SEQ ID NO: 19, wherein the mutation in the nucleotide sequence SEQ ID NO: 19 is an insertion of nucleotide T between position 257 and 258 (258insT) or an insertion of nucleotide AT between position 257 and 258 (258insAT).
   **and**
**(c)** a monoallelic heterozygous mutation or a biallelic heterozygous mutation, wherein the mutation is in the coding region or in the region out of the coding region of the gene:
   - gene Nitab4.5_0003900g0020.1 (1a_SPL2/10/11) comprising, or consisting of, nucleotide sequence SEQ ID NO: 14, wherein the mutation in the nucleotide sequence SEQ ID NO: 14 is a deletion of nucleotides CCA at position 338 (338deICCA) and/or a deletion of nucleotides GCAGGAAACCATCCAGTTCA at position 738 and insertion of nucleotides ATTTAGTTGCAATTTTTGTCAAGGATATCTACCTGCTAATAGCTTGCCTTTTA GCAATTTTCGCAGGAGGCTCTCTGATC between position 737 and 738 (738delGCAGGAAACCATCCAGTTCAinsATTTAGTTGCAATTTTTGTCAAGGAT ATCTACCTGCTAATAGCTTGCCTTTTAGCAATTTTCGCAGGAGGCTCTCTGA TC).

In other more preferred embodiment of the plant of the invention (the mFTSPLa line), the gene Nitab4.5_0003900g0020.1 (1a_SPL2/10/11) comprising, or consisting of, nucleotide sequence SEQ ID NO: 14, wherein the biallelic heterozygous mutation in the nucleotide sequence SEQ ID NO: 14 is a deletion of nucleotides CCA at position 338 (338deICCA) in one of the alleles of the gene; and a deletion of nucleotides GCAGGAAACCATCCAGTTCA at position 738 and insertion of nucleotides ATTTAGTTGCAATTTTTGTCAAGGATATCTACCTGCTAATAGCTTGCCTTTTAGC AATTTTCGCAGGAGGCTCTCTGATC between position 737 and 738 (738delGCAGGAAACCATCCAGTTCAinsATTTAGTTGCAATTTTTGTCAAGGATAT CTACCTGCTAATAGCTTGCCTTTTAGCAATTTTCGCAGGAGGCTCTCTGATC) in the other of the alleles of the gene.

In other more preferred embodiment of the plant of the invention (the mFTSPLa line), the gene Nitab4.5_0003900g0020.1 (1a_SPL2/10/11) comprising, or consisting of, nucleotide sequence SEQ ID NO: 14, wherein the monoallelic heterozygous mutation in the nucleotide sequence SEQ ID NO: 14 is a deletion of nucleotides CCA at position 338 (338deICCA) and a deletion of nucleotides GCAGGAAACCATCCAGTTCA at position 738 and insertion of nucleotides ATTTAGTTGCAATTTTTGTCAAGGATATCTACCTGCTAATAGCTTGCCTTTTAGC AATTTTCGCAGGAGGCTCTCTGATC between position 737 and 738 (738delGCAGGAAACCATCCAGTTCAinsATTTAGTTGCAATTTTTGTCAAGGATAT CTACCTGCTAATAGCTTGCCTTTTAGCAATTTTCGCAGGAGGCTCTCTGATC) in one of the alleles of the gene (the other allele of the gene is wild type).

In other more preferred embodiment, the plant of the invention (mFTSPLa line) comprising, or consisting of:
- the two alleles of the gene Nitab4.5_0001752g0040.1 (NtSPL3/4/5_2a) comprising, or consisting of, nucleotide sequence SEQ ID NO: 20 (sequence with the mutation 353delACAAC) that generates an early stop codon at position 418;
- one allele of the gene Nitab4.5_0000638g0040.1 (NtSPL3/4/5_2b) comprising, or consisting of, nucleotide sequence SEQ ID NO: 22 (sequence with the mutation 355delAA) that generates an early stop codon at position 397, and the other allele comprising, or consisting of, nucleotide sequence SEQ ID NO: 21 (sequence with the mutation 349deIGGACACAA) that generates an early stop codon at position 391;
- the two alleles of the gene Nitab4.5_0003348g0050.1 (NtSPL3/4/5_6a) comprising, or consisting of, nucleotide sequence SEQ ID NO: 25 (sequence with the mutation 213delC) that results in a frameshift and a delayed stop codon;
- the two alleles of the gene Nitab4.5_0007487g0020.1 (NtSPL3/4/5_7a) comprising, or consisting of, nucleotide sequence SEQ ID NO: 32 (sequence with the mutation 204deICA) that generates an early stop codon at position 244;
- the two alleles of the gene Nitab4.5_0002219g0060.1 (NtSPL3/4/5_7b) comprising, or consisting of, nucleotide sequence SEQ ID NO: 33 (sequence with the mutation 213insA) that generates an early stop codon at position 256;
- the two alleles of the gene Nitab4.5_0003572g0010.1 (NtSPL9/15_1a) comprising, or consisting of, nucleotide sequence SEQ ID NO: 34 (sequence with the mutation 289insC) that generates an early stop codon at position 301;
- the two alleles of the gene Nitab4.5_0000016g0300.1 (NtSPL9/15_1b) comprising, or consisting of, nucleotide sequence SEQ ID NO: 35 (sequence with the mutation 490insT) that generates an early stop codon at position 502;
- one allele of the gene Nitab4.5_0002299g0030.1 (1a_SPL13) comprising, or consisting of, nucleotide sequence SEQ ID NO: 46 (sequence with the mutation 272deIG) that generates an early stop codon at position 400, and the other allele comprising, or consisting of, nucleotide sequence SEQ ID NO: 47 (sequence with the mutation 269delGGCG) that generates an early stop codon at position 397;
- the two alleles of the gene Nitab4.5_0001010g0010.1 (1b_SPL13) comprising, or consisting of, nucleotide sequence SEQ ID NO: 51 (sequence with the mutation 277insT) that generates an early stop codon at position 295;
- one allele of the gene Nitab4.5_0003900g0020.1 (1a_SPL2/10/11) comprising, or consisting of, nucleotide sequence SEQ ID NO: 66 (sequence with the mutation738delGCAGGAAACCATCCAGTTCAinsATTTAGTTGCAATTTTTGTCA AGGATATCTACCTGCTAATAGCTTGCCTTTTAGCAATTTTCGCAGGAGGCTC TCTGATC) that results in a substitution of a sequence of seven aminoacids by another sequence of 27 aminoacids in a second downstream position and the other allele comprising, or consisting of, nucleotide sequence SEQ ID NO: 67 (sequence with the mutation 338deICCA) that results in the loss of one amino acid; or one allele of the gene Nitab4.5_0003900g0020.1 (1a_SPL2/10/11) comprising, or consisting of, nucleotide SEQ ID NO: 14 and the other allele comprising, or consisting of, nucleotide sequence SEQ ID NO: 68 (sequence with the mutations 338delCCA + 738delGCAGGAAACCATCCAGTTCAinsATTTAGTTGCAATTTTTGTCAAGGAT ATCTACCTGCTAATAGCTTGCCTTTTAGCAATTTTCGCAGGAGGCTCTCTGA TC) that results in a loss of one aminoacid in one upstream position, and a substitution of a sequence of seven aminoacids by another sequence of 27 aminoacids in a second downstream position;
- two alleles of the gene Nitab4.5_0000861g0050.1 (1b_SPL2/10/11) comprising, or consisting of, nucleotide sequence SEQ ID NO: 75 (sequence with the mutations 693deIG + 752insA) that results in a substitution of nineteen amino acids;
- one allele of the gene Nitab4.5_0000819g0010.1 (1a_FT5) comprising, or consisting of, nucleotide sequence SEQ ID NO: 87 (sequence with the mutation 258insA) that generates an early stop codon at position 271, and the other allele comprising, or consisting of, nucleotide sequence SEQ ID NO: 88 (sequence with the mutation 258insC) that generates an early stop codon at position 271;
   and
- one allele of the gene Nitab4.5_0000751g0180.1 (1b_FT5) comprising, or consisting of, nucleotide sequence SEQ ID NO: 89 (sequence with the mutation 258insT) that generates an early stop codon at position 271, and the other allele comprising, or consisting of, nucleotide sequence SEQ ID NO: 90 (sequence with the mutation 258insAT) that generates a frameshift and a delayed stop codon.

### mFTSPLb line

In other preferred embodiment, the plant of the invention (also discloses as the mFTSPLb line) comprises:
**(a)** a biallelic homozygous mutation in the coding region of all following genes (the same mutation is in both alleles of the gene):
   - gene Nitab4.5_0001752g0040.1 (2a_SPL3/4/5) comprising, or consisting of, nucleotide sequence SEQ ID NO: 1, wherein the mutation in the nucleotide sequence SEQ ID NO: 1 is a deletion of nucleotides ACAAC at position 353 (353delACAAC);
   - gene Nitab4.5_0000638g0040.1 (2b_SPL3/4/5) comprising, or consisting of, nucleotide sequence SEQ ID NO: 2, wherein the mutation in the nucleotide sequence SEQ ID NO: 2 is a deletion of nucleotides AA at position 355 (355delAA);
   - gene Nitab4.5_0004959g0040.1 (4b_SPL3/4/5) comprising, or consisting of, nucleotide sequence SEQ ID NO: 3, wherein the mutation in the nucleotide sequence SEQ ID NO: 3 is an insertion (of nucleotide A between position 141 and 142 (142insA);
   - gene Nitab4.5_0003348g0050.1 (6a_SPL3/4/5) comprising, or consisting of, nucleotide sequence SEQ ID NO: 4, wherein the mutation in the nucleotide sequence SEQ ID NO: 4 is a deletion of the nucleotide C at position 213 (213delC);
   - gene Nitab4.5_0003942g0050.1 (6b_SPL3/4/5) comprising, or consisting of, nucleotide sequence SEQ ID NO: 5, wherein the mutation in the nucleotide sequence SEQ ID NO: 5 is an insertion of nucleotide A between position 224 and 225 (225insA);
   - gene Nitab4.5_0007487g0020.1 (7a_SPL3/4/5) comprising, or consisting of, nucleotide sequence SEQ ID NO: 6, wherein the mutation in the nucleotide sequence SEQ ID NO: 6 is a deletion of the nucleotides CA at position 204 (204deICA);
   - gene Nitab4.5_0002219g0060.1 (SPL3/4/5) comprising, or consisting of, nucleotide sequence SEQ ID NO: 7, wherein the mutation in the nucleotide sequence SEQ ID NO: 7 is an insertion of nucleotide A between position 212 and 213 (213insA);
   - gene Nitab4.5_0003572g0010.1 (1a_SPL9/15) comprising, or consisting of, nucleotide sequence SEQ ID NO: 8, wherein the mutation in the nucleotide sequence SEQ ID NO: 8 is an insertion of nucleotide C between position 288 and 289 (289insC);
   - gene Nitab4.5_0000016g0300.1 (1b_SPL9/15) comprising, or consisting of, nucleotide sequence SEQ ID NO: 9, wherein the mutation in the nucleotide sequence SEQ ID NO: 9 is an insertion of nucleotide T between position 489 and 490 (490insT);
   - gene Nitab4.5_0001538g0080.1 (2a_SPL9/15) comprising, or consisting of, nucleotide sequence SEQ ID NO: 10, wherein the mutation in the nucleotide sequence SEQ ID NO: 10 is an insertion of nucleotide A between position 216 and 217 (217insA);
   - gene Nitab4.5_0000991g0020.1 (2b_SPL9/15) comprising, or consisting of, nucleotide sequence SEQ ID NO: 11, wherein the mutation in the nucleotide sequence SEQ ID NO: 11 is a deletion of nucleotide A at position 217 (217deIA);
   - gene Nitab4.5_0002299g0030.1 (1a_SPL13) comprising, or consisting of, nucleotide sequence SEQ ID NO: 12, wherein the mutation in the nucleotide sequence SEQ ID NO: 12 is a deletion of nucleotides TA at position 135 (135deITA) and an insertion of nucleotide A between position 273 and 274 (274insA);
   - gene Nitab4.5_0003900g0020.1 (1a_SPL2/10/11) comprising, or consisting of, nucleotide sequence SEQ ID NO: 14, wherein the mutation in the nucleotide sequence SEQ ID NO: 14 is an insertion of nucleotide A between position 757 and 758 (758insA);
   - gene Nitab4.5_0000861g0050.1 (1b_SPL2/10/11) comprising, or consisting of, nucleotide sequence SEQ ID NO: 15, wherein the mutation in the nucleotide sequence SEQ ID NO: 15 is a deletion of nucleotide G at position 693 (693deIG);
   - gene Nitab4.5_0000819g0010.1 (1a_FT5) comprising, or consisting of, nucleotide sequence SEQ ID NO: 18, wherein the mutation in the nucleotide sequence SEQ ID NO: 18 is a deletion of nucleotides TCCATTGG at position 254 (254deITCCATTGG); and
   - gene Nitab4.5_0000751g0180.1 (1b_FT5) comprising, or consisting of, nucleotide sequence SEQ ID NO: 19, wherein the mutation in the nucleotide sequence SEQ ID NO: 19 is an insertion of nucleotide T between position 257 and 258 (258insT);
   **and**
**(b)** a biallelic heterozygous mutation in the coding region of all following genes (both alleles of the gene are mutated with different mutations):
   - gene Nitab4.5_0000861g0050.1 (1b_SPL2/10/11) comprising, or consisting of, nucleotide sequence SEQ ID NO: 15, wherein the mutation in the nucleotide sequence SEQ ID NO: 15 is a deletion of nucleotides TC at position 749 (749deITC) and an insertion of nucleotide A between position 749 and 750 (750insA); and
   - gene Nitab4.5_0001010g0010.1 (1b_SPL13) comprising, or consisting of, nucleotide sequence SEQ ID NO: 13, wherein the mutation in the nucleotide sequence SEQ ID NO: 13 is an insertion of nucleotide T between position 122 and 123 (123insT), an insertion of nucleotide A between position 276 and 277 (277insA) and an insertion of nucleotide G between position 276 and 277 (277insG);
   **and**
**(c)** at least one monoallelic heterozygous mutation in the coding region of the following gene:
   - gene Nitab4.5_0003348g0050.1 (6a_SPL3/4/5) comprising, or consisting of, nucleotide sequence SEQ ID NO: 4, wherein the mutation in the nucleotide sequence SEQ ID NO: 4 is an insertion of nucleotide T between position 424 and 425 (425insT).

In other more preferred embodiment of the plant of the invention (the mFTSPLb line), the gene Nitab4.5_0001010g0010.1 (1b_SPL13) comprising, or consisting of, nucleotide sequence SEQ ID NO: 13, wherein the biallelic heterozygous mutation in the nucleotide sequence SEQ ID NO: 13 is:
(i) an insertion of nucleotide A between position 276 and 277 (277insA) in one of the alleles of the gene and an insertion of nucleotide T between position 122 and 123 (123insT) and an insertion of nucleotide G between position 276 and 277 (277insG) in the other of the alleles of the gene; or
(ii) an insertion of nucleotide G between position 276 and 277 (277insG) in one of the alleles of the gene and an insertion of nucleotide T between position 122 and 123 (123insT) and an insertion of nucleotide A between position 276 and 277 (277insA) in the other of the alleles of the gene.

In other more preferred embodiment, the plant of the invention (mFTSPLb line) comprising, or consisting of:
- the two alleles of the gene Nitab4.5_0001752g0040.1 (NtSPL3/4/5_2a) comprising, or consisting of, nucleotide sequence SEQ ID NO: 20 (sequence with the mutation 353delACAAC) that generates an early stop codon at position 418;
- the two alleles of the gene Nitab4.5_0000638g0040.1 (NtSPL3/4/5_2b) comprising, or consisting of, nucleotide sequence SEQ ID NO: 22 (sequence with the mutation 355deIAA) that generates an early stop codon at position 397;
- the two alleles of the gene Nitab4.5_0004959g0040.1 (NtSPL3/4/5_4b) comprising, or consisting of, nucleotide sequence SEQ ID NO: 23 (sequence with the mutation 142insA) that generates an early stop codon at position 160;
- one allele of the gene Nitab4.5_0003348g0050.1 (NtSPL3/4/5_6a) comprising, or consisting of, nucleotide sequence SEQ ID NO: 25 (sequence with the mutation 213delC) that results in a frameshift and a delayed stop codon, and the other allele comprising, or consisting of, nucleotide sequence SEQ ID NO: 26 (sequence with the mutation 213delC + 425insT) that results in a frameshift and a delayed stop codon;
- two alleles of the gene Nitab4.5_0003942g0050.1 (NtSPL3/4/5_6b) comprising, or consisting of, nucleotide sequence SEQ ID NO: 30 (sequence with the mutation 225insA) that generates an early stop codon at position 235;
- the two alleles of the gene Nitab4.5_0007487g0020.1 (NtSPL3/4/5_7a) comprising, or consisting of, nucleotide sequence SEQ ID NO: 32 (sequence with the mutation 204deICA) that generates an early stop codon at position 244;
- the two alleles of the gene Nitab4.5_0002219g0060.1 (NtSPL3/4/5_7b) comprising, or consisting of, nucleotide sequence SEQ ID NO: 33 (sequence with the mutation 213insA) that generates an early stop codon at position 256;
- the two alleles of the gene Nitab4.5_0003572g0010.1 (NtSPL9/15_1a) comprising, or consisting of, nucleotide sequence SEQ ID NO: 34 (sequence with the mutation 289insC) that generates an early stop codon at position 301;
- the two alleles of the gene Nitab4.5_0000016g0300.1 (NtSPL9/15_1b) comprising, or consisting of, nucleotide sequence SEQ ID NO: 35 (sequence with the mutation 490insT) that generates an early stop codon at position 502;
- the two alleles of the gene Nitab4.5_0001538g0080.1 (2a_SPL9/15) comprising, or consisting of, nucleotide sequence SEQ ID NO: 36 (sequence with the mutation 217insA) that generates an early stop codon at position 229;
- the two alleles of the gene Nitab4.5_0000991g0020.1 (2b_SPL9/15) comprising, or consisting of, nucleotide sequence SEQ ID NO: 41 (sequence with the mutation 217deIA) that generates an early stop codon at position 241;
- the two alleles of the gene Nitab4.5_0002299g0030.1 (1a_SPL13) comprising, or consisting of, nucleotide sequence SEQ ID NO: 48 (sequence with the mutation 135delTA + 274insA) that generates an early stop codon at position 181;
- one allele of the gene Nitab4.5_0001010g0010.1 (1b_SPL13) comprising, or consisting of, nucleotide sequence SEQ ID NO: 53 (sequence with the mutation 277insA) that generates an early stop codon at position 295, and the other allele comprising, or consisting of, nucleotide sequence SEQ ID NO: 54 (sequence with the mutation 123insT + 277insG) that generates an early stop codon at position 124; or one allele of the gene Nitab4.5_0001010g0010.1 (1b_SPL13) comprising, or consisting of, nucleotide sequence SEQ ID NO: 55 (sequence with the mutation 123insT + 277insA) that generates an early stop codon at position 124, and the other allele comprising, or consisting of, nucleotide sequence SEQ ID NO: 56 (sequence with the mutation 277insG) that generates an early stop codon at position 295;
- the two alleles of the gene Nitab4.5_0003900g0020.1 (1a_SPL2/10/11) comprising, or consisting of, nucleotide sequence SEQ ID NO: 65 (sequence with the mutation 758insA) that generates an early stop codon at position 787;
- one allele of the gene Nitab4.5_0000861g0050.1 (1b_SPL2/10/11) comprising, or consisting of, nucleotide sequence SEQ ID NO: 76 (sequence with the mutations 693deIG + 749deITC) that results in the substitution of eighteen amino acids and the deletion of one, and the other allele comprising, or consisting of, nucleotide sequence SEQ ID NO: 77 (sequence with the mutation 693deIG + 750insA) that results in the substitution of nineteen amino acids;
- the two alleles of the gene Nitab4.5_0000819g0010.1 (1a_FT5) comprising, or consisting of, nucleotide sequence SEQ ID NO: 85 (sequence with the mutation 254deITCCATTGG) that results in frameshift and a delayed stop codon;
   and
- the two alleles of the gene Nitab4.5_0000751g0180.1 (1b_FT5) comprising, or consisting of, nucleotide sequence SEQ ID NO: 89 (sequence with the mutation 258insT) that generates an early stop codon at position 271.

### mFTSPLc line

In other preferred embodiment, the plant of the invention (also discloses as the mFTSPLc line) comprises:
**(a)** a biallelic homozygous mutation in the coding region of all following genes (the same mutation is in both alleles of the gene):
   - gene Nitab4.5_0001752g0040.1 (2a_SPL3/4/5) comprising, or consisting of, nucleotide sequence SEQ ID NO: 1, wherein the mutation in the nucleotide sequence SEQ ID NO: 1 is a deletion of nucleotides ACAAC at position 353 (353delACAAC);
   - gene Nitab4.5_0003348g0050.1 (6a_SPL3/4/5) comprising, or consisting of, nucleotide sequence SEQ ID NO: 4, wherein the mutation in the nucleotide sequence SEQ ID NO: 4 is a deletion of the nucleotide C at position 213 (213delC);
   - gene Nitab4.5_0007487g0020.1 (7a_SPL3/4/5) comprising, or consisting of, nucleotide sequence SEQ ID NO: 6, wherein the mutation in the nucleotide sequence SEQ ID NO: 6 is a deletion of the nucleotides CA at position 204 (204deICA);
   - gene Nitab4.5_0002219g0060.1 (SPL3/4/5) comprising, or consisting of, nucleotide sequence SEQ ID NO: 7, wherein the mutation in the nucleotide sequence SEQ ID NO: 7 is an insertion of nucleotide A between position 212 and 213 (213insA);
   - gene Nitab4.5_0003572g0010.1 (1a_SPL9/15) comprising, or consisting of, nucleotide sequence SEQ ID NO: 8, wherein the mutation in the nucleotide sequence SEQ ID NO: 8 is an insertion of nucleotide C between position 288 and 289 (289insC);
   - gene Nitab4.5_0000016g0300.1 (1b_SPL9/15) comprising, or consisting of, nucleotide sequence SEQ ID NO: 9, wherein the mutation in the nucleotide sequence SEQ ID NO: 9 is an insertion of nucleotide T between position 489 and 490 (490insT); and
   - gene Nitab4.5_0000751g0180.1 (1b_FT5) comprising, or consisting of, nucleotide sequence SEQ ID NO: 19, wherein the mutation in the nucleotide sequence SEQ ID NO: 19 is an insertion of nucleotide T between position 257 and 258 (258insT);
   **and**
**(b)** a biallelic heterozygous mutation in the coding region of all following genes (both alleles of the gene are mutated with different mutations):
   - gene Nitab4.5_0000638g0040.1 (2b_SPL3/4/5) comprising, or consisting of, nucleotide sequence SEQ ID NO: 2, wherein the mutation in the nucleotide sequence SEQ ID NO: 2 is a deletion of nucleotides AA at position 355 (355delAA) or a deletion of nucleotides GGACACAA at position 349 (349deIGGACACAA);
   - gene Nitab4.5_0001010g0010.1 (1b_SPL13) comprising, or consisting of, nucleotide sequence SEQ ID NO: 13, wherein the mutation in the nucleotide sequence SEQ ID NO: 13 is an insertion of nucleotide A between position 276 and 277 (277insA) and an insertion of nucleotide T between position 276 and 277 (277insT);
   - gene Nitab4.5_0000861g0050.1 (1b_SPL2/10/11) comprising, or consisting of, nucleotide sequence SEQ ID NO: 15, wherein the mutation in the nucleotide sequence SEQ ID NO: 15 is a deletion of nucleotides GA at position 693 (693deIGA), an insertion of nucleotide T between position 692 and 693 (693insT), an insertion of nucleotide G between position 751 and 752 (752insG), and/or an insertion of nucleotide T between position 751 and 752 (752insT); and
   - gene Nitab4.5_0000819g0010.1 (1a_FT5) comprising, or consisting of, nucleotide sequence SEQ ID NO: 18, wherein the mutation in the nucleotide sequence SEQ ID NO: 18 is an insertion of nucleotide T between position 257 and 258 (258insT) or an insertion of nucleotide A between position 257 and 258 (258insA);
   **and**
**(c)** a monoallelic heterozygous mutation in the coding region of the following genes:
   - gene Nitab4.5_0003942g0050.1 (6b_SPL3/4/5) comprising, or consisting of, nucleotide sequence SEQ ID NO: 5, wherein the mutation in the nucleotide sequence SEQ ID NO: 5 is an insertion of nucleotide A between position 224 and 225 (225insA);
   - gene Nitab4.5_0001538g0080.1 (2a_SPL9/15) comprising, or consisting of, nucleotide sequence SEQ ID NO: 10, wherein the mutation in the nucleotide sequence SEQ ID NO: 10 is a deletion of nucleotides GT at position 121 and a deletion of nucleotide G at position 218 (121deIGT+218deIG);
   - gene Nitab4.5_0000991g0020.1 (2b_SPL9/15) comprising, or consisting of, nucleotide sequence SEQ ID NO: 11, wherein the mutation in the nucleotide sequence SEQ ID NO: 11 is a deletion of nucleotides AG at position 217 (217delAG);
   - gene Nitab4.5_0002299g0030.1 (1a_SPL13) comprising, or consisting of, nucleotide sequence SEQ ID NO: 12, wherein the mutation in the nucleotide sequence SEQ ID NO: 12 is a deletion of nucleotide T at position 273 (273deIT); and
   - gene Nitab4.5_0003900g0020.1 (1a_SPL2/10/11) comprising, or consisting of, nucleotide sequence SEQ ID NO: 14, wherein the mutation in the nucleotide sequence SEQ ID NO: 14 is a deletion of nucleotide A at position 758 (758deIA).

In other more preferred embodiment of the plant of the invention (the mFTSPLc line), the gene Nitab4.5_0000861g0050.1 (1b_SPL2/10/11) comprising, or consisting of, nucleotide sequence SEQ ID NO: 15, wherein the biallelic heterozygous mutation monoallelic mutation in the nucleotide sequence SEQ ID NO: 15 is:
(i) an insertion of nucleotide T between position 692 and 693 (693insT) and an insertion of nucleotide G between position 751 and 752 (752insG) in one of the alleles of the gene, and a deletion of nucleotides GA at position 693 (693deIGA) and an insertion of nucleotide T between position 751 and 752 (752insT) in the other of the alleles of the gene; or
(ii) an insertion of nucleotide T between position 692 and 693 (693insT) and an insertion of nucleotide T between position 751 and 752 (752insT) in one of the alleles of the gene, and a deletion of nucleotides GA at position 693 (693deIGA) and an insertion of nucleotide G between position 751 and 752 (752insG) in the other of the alleles of the gene.

In other more preferred embodiment, the plant of the invention (mFTSPLc line) comprising, or consisting of:
- the two alleles of the gene Nitab4.5_0001752g0040.1 (NtSPL3/4/5_2a) comprising, or consisting of, nucleotide sequence SEQ ID NO: 20 (sequence with the mutation 353delACAAC) that generates an early stop codon at position 418;
- one of the allele of the gene Nitab4.5_0000638g0040.1 (NtSPL3/4/5_2b) comprising, or consisting of, nucleotide sequence SEQ ID NO: 21 (sequence with the mutation 349deIGGACACAA) that generates an early stop codon at position 391; and the other allele comprising, or consisting of, nucleotide sequence SEQ ID NO: 22 (sequence with the mutation 355delAA) that generates an early stop codon at position 397;
- the two alleles of the gene Nitab4.5_0003348g0050.1 (NtSPL3/4/5_6a) comprising, or consisting of, nucleotide sequence SEQ ID NO: 25 (sequence with the mutation 213delC) that results in a frameshift and a delayed stop codon;
- one allele of the gene Nitab4.5_0003942g0050.1 (NtSPL3/4/5_6b) comprising, or consisting of, nucleotide sequence SEQ ID NO: 5, and other allele comprising, or consisting of, nucleotide sequence SEQ ID NO: 30 (sequence with the mutation 225insA) that generates an early stop codon at position 235;
- the two alleles of the gene Nitab4.5_0007487g0020.1 (NtSPL3/4/5_7a) comprising, or consisting of, nucleotide sequence SEQ ID NO: 32 (sequence with the mutation 204deICA) that generates an early stop codon at position 244;
- the two alleles of the gene Nitab4.5_0002219g0060.1 (NtSPL3/4/5_7b) comprising, or consisting of, nucleotide sequence SEQ ID NO: 33 (sequence with the mutation 213insA) that generates an early stop codon at position 256;
- the two alleles of the gene Nitab4.5_0003572g0010.1 (NtSPL9/15_1a) comprising, or consisting of, nucleotide sequence SEQ ID NO: 34 (sequence with the mutation 289insC) that generates an early stop codon at position 301;
- the two alleles of the gene Nitab4.5_0000016g0300.1 (NtSPL9/15_1b) comprising, or consisting of, nucleotide sequence SEQ ID NO: 35 (sequence with the mutation 490insT) that generates an early stop codon at position 502;
- one allele of the gene Nitab4.5_0001538g0080.1 (2a_SPL9/15) comprising, or consisting of, nucleotide sequence SEQ ID NO: 10, and other allele comprising, or consisting of, nucleotide sequence SEQ ID NO: 39 (sequence with the mutation 121delGT + 218deIG) that generates an early stop codon at position 214;
- one allele of the gene Nitab4.5_0000991g0020.1 (2b_SPL9/15) comprising, or consisting of, nucleotide sequence SEQ ID NO: 11, and other allele comprising, or consisting of, nucleotide sequence SEQ ID NO: 43 (sequence with the mutation 217delAG) that generates an early stop codon at position 226;
- one allele of the gene Nitab4.5_0002299g0030.1 (1a_SPL13) comprising, or consisting of, nucleotide sequence SEQ ID NO: 12, and other allele comprising, or consisting of, nucleotide sequence SEQ ID NO: 50 (sequence with the mutation 273deIT) that generates an early stop codon at position 400;
- one allele of the gene Nitab4.5_0001010g0010.1 (1b_SPL13) comprising, or consisting of, nucleotide sequence SEQ ID NO: 53 (sequence with the mutation 277insA) that generates an early stop codon at position 295, and the other allele comprising, or consisting of, nucleotide sequence SEQ ID NO: 51 (sequence with the mutation 277insT) that generates an early stop codon at position 295;
- one allele of the gene Nitab4.5_0003900g0020.1 (1a_SPL2/10/11) comprising, or consisting of, nucleotide sequence SEQ ID NO: 14, and the other allele comprising, or consisting of, nucleotide sequence SEQ ID NO: 69 (sequence with the mutation 758deIA) that generates an early stop codon at position 802;
- one allele of the gene Nitab4.5_0000861g0050.1 (1b_SPL2/10/11) comprising, or consisting of, nucleotide sequence SEQ ID NO: 78 (sequence with the mutations 693insT + 752insG) that generates an early stop codon at position 703, and the other allele comprising, or consisting of, nucleotide sequence SEQ ID NO: 79 (sequence with the mutation 693insT + 752insT) that generates an early stop codon at position 703; or one allele of the gene Nitab4.5_0000861g0050.1 (1b_SPL2/10/11) comprising, or consisting of, nucleotide sequence SEQ ID NO: 80 (sequence with the mutations 693delGA + 752insG) that generates an early stop codon at position 700, and the other allele comprising, or consisting of, nucleotide sequence SEQ ID NO: 81 (sequence with the mutation 693delGA + 752insT) that generates an early stop codon at position 700;
- one allele of the gene Nitab4.5_0000819g0010.1 (1a_FT5) comprising, or consisting of, nucleotide sequence SEQ ID NO: 86 (sequence with the mutation 258insT) that generates an early stop codon at position 271, and the other allele comprising, or consisting of, nucleotide sequence SEQ ID NO: 87 (sequence with the mutation 258insA) that generates an early stop codon at position 271;
   and
- the two alleles of the gene Nitab4.5_0000751g0180.1 (1b_FT5) comprising, or consisting of, nucleotide sequence SEQ ID NO: 89 (sequence with the mutation 258insT) that generates an early stop codon at position 271.

### mFTSPLd line

In other preferred embodiment, the plant of the invention (also discloses as the mFTSPLd line) comprises:
(a) a biallelic homozygous mutation in the coding region of all following genes (the same mutation is in both alleles of the gene):
   - gene Nitab4.5_0001752g0040.1 (2a_SPL3/4/5) comprising, or consisting of, nucleotide sequence SEQ ID NO: 1, wherein the mutation in the nucleotide sequence SEQ ID NO: 1 is a deletion of nucleotides ACAAC at position 353 (353delACAAC);
   - gene Nitab4.5_0003348g0050.1 (6a_SPL3/4/5) comprising, or consisting of, nucleotide sequence SEQ ID NO: 4, wherein the mutation in the nucleotide sequence SEQ ID NO: 4 is a deletion of the nucleotide C at position 213 (213delC);
   - gene Nitab4.5_0003942g0050.1 (6b_SPL3/4/5) comprising, or consisting of, nucleotide sequence SEQ ID NO: 5, wherein the mutation in the nucleotide sequence SEQ ID NO: 5 is an insertion of nucleotide A between position 224 and 225 (225insA);
   - gene Nitab4.5_0007487g0020.1 (7a_SPL3/4/5) comprising, or consisting of, nucleotide sequence SEQ ID NO: 6, wherein the mutation in the nucleotide sequence SEQ ID NO: 6 is a deletion of the nucleotides CA at position 204 (204deICA);
   - gene Nitab4.5_0002219g0060.1 (SPL3/4/5) comprising, or consisting of, nucleotide sequence SEQ ID NO: 7, wherein the mutation in the nucleotide sequence SEQ ID NO: 7 is an insertion of nucleotide A between position 212 and 213 (213insA);
   - gene Nitab4.5_0003572g0010.1 (1a_SPL9/15) comprising, or consisting of, nucleotide sequence SEQ ID NO: 8, wherein the mutation in the nucleotide sequence SEQ ID NO: 8 is an insertion of nucleotide C between position 288 and 289 (289insC);
   - gene Nitab4.5_0000016g0300.1 (1b_SPL9/15) comprising, or consisting of, nucleotide sequence SEQ ID NO: 9, wherein the mutation in the nucleotide sequence SEQ ID NO: 9 is an insertion of nucleotide T between position 489 and 490 (490insT);
   - gene Nitab4.5_0001538g0080.1 (2a_SPL9/15) comprising, or consisting of, nucleotide sequence SEQ ID NO: 10, wherein the mutation in the nucleotide sequence SEQ ID NO: 10 is a deletion of nucleotides AG at position 217 (217delAG);
   - gene Nitab4.5_0002299g0030.1 (1a_SPL13) comprising, or consisting of, nucleotide sequence SEQ ID NO: 12, wherein the mutation in the nucleotide sequence SEQ ID NO: 12 is a deletion of nucleotides TAAAGT at position 135 and an insertion of nucleotide A between position 273 and 274 (135delTAAAGT + 274insA);
   - gene Nitab4.5_0000819g0010.1 (1a_FT5) comprising, or consisting of, nucleotide sequence SEQ ID NO: 18, wherein the mutation in the nucleotide sequence SEQ ID NO: 18 is an insertion of nucleotide T between position 257 and 258 (258insT); and
   - gene Nitab4.5_0000751g0180.1 (1b_FT5) comprising, or consisting of, nucleotide sequence SEQ ID NO: 19, wherein the mutation in the nucleotide sequence SEQ ID NO: 19 is an insertion of nucleotide C between position 257 and 258 (258insC);
   **and**
**(b)** a biallelic heterozygous mutation in the coding region of all following genes (both alleles of the gene are mutated with different mutations):
   - gene Nitab4.5_0000638g0040.1 (2b_SPL3/4/5) comprising, or consisting of, nucleotide sequence SEQ ID NO: 2, wherein the mutation in the nucleotide sequence SEQ ID NO: 2 is a deletion of nucleotides AA at position 355 (355delAA) or a deletion of nucleotides GGACACAA at position 349 (349deIGGACACAA);
      and
   - gene Nitab4.5_0000861g0050.1 (1b_SPL2/10/11) comprising, or consisting of, nucleotide sequence SEQ ID NO: 15, wherein the mutation in the nucleotide sequence SEQ ID NO: 15 is an insertion of nucleotide A between position 751 and 752 (752insA), an insertion of nucleotide T between position 692 and 693 (693insT), and/or a deletion of nucleotides AGTTCAACTCGGCA at position 746 (746delAGTTCAACTCGGCA);
   **and**
**(c)** a monoallelic heterozygous mutation or a biallelic heterozygous mutation, wherein the mutation is in the coding region or in the region out of the coding region of the gene:
   - gene Nitab4.5_0001010g0010.1 (1b_SPL13) comprising, or consisting of, nucleotide sequence SEQ ID NO: 13, wherein the mutation in the nucleotide sequence SEQ ID NO: 13 is a deletion of nucleotide T at position 123 (123deIT) and/or a deletion of nucleotides GTCGA at position 275 (275delGTCGA).

In other more preferred embodiment of the plant of the invention (the mFTSPLd line), the gene Nitab4.5_0001010g0010.1 (1b_SPL13) comprising, or consisting of, nucleotide sequence SEQ ID NO: 13, wherein the monoallelic heterozygous mutation in the nucleotide sequence SEQ ID NO: 13 is a deletion of nucleotide T at position 123 (123delT) and a deletion of nucleotides GTCGA at position 275 (275deIGTCGA) in one of the alleles and the other allele is wild type.

In other more preferred embodiment of the plant of the invention (the mFTSPLd line), the gene Nitab4.5_0001010g0010.1 (1b_SPL13) comprising, or consisting of, nucleotide sequence SEQ ID NO: 13, wherein the biallelic heterozygous mutation in the nucleotide sequence SEQ ID NO: 13 is a deletion of nucleotide T at position 123 (123delT) in one of the alleles and a deletion of nucleotides GTCGA at position 275 (275deIGTCGA) in the other allele.

In other more preferred embodiment, the plant of the invention (mFTSPLd line) comprising, or consisting of:
- the two alleles of the gene Nitab4.5_0001752g0040.1 (NtSPL3/4/5_2a) comprising, or consisting of, nucleotide sequence SEQ ID NO: 20 (sequence with the mutation 353delACAAC) that generates an early stop codon at position 418;
- one allele of the gene Nitab4.5_0000638g0040.1 (NtSPL3/4/5_2b) comprising, or consisting of, nucleotide sequence SEQ ID NO: 21 (sequence with the mutation 349deIGGACACAA) that generates an early stop codon at position 391; and the other allele comprising, or consisting of, nucleotide sequence SEQ ID NO: 22 (sequence with the mutation 355delAA) that generates an early stop codon at position 397;
- the two alleles of the gene Nitab4.5_0003348g0050.1 (NtSPL3/4/5_6a) comprising, or consisting of, nucleotide sequence SEQ ID NO: 25 (sequence with the mutation 213delC) that results in a frameshift and a delayed stop codon;
- the two alleles of the gene Nitab4.5_0003942g0050.1 (NtSPL3/4/5_6b) comprising, or consisting of, nucleotide sequence SEQ ID NO: 30 (sequence with the mutation 225insA) that generates an early stop codon at position 235;
- the two alleles of the gene Nitab4.5_0007487g0020.1 (NtSPL3/4/5_7a) comprising, or consisting of, nucleotide sequence SEQ ID NO: 32 (sequence with the mutation 204deICA) that generates an early stop codon at position 244;
- the two alleles of the gene Nitab4.5_0002219g0060.1 (NtSPL3/4/5_7b) comprising, or consisting of, nucleotide sequence SEQ ID NO: 33 (sequence with the mutation 213insA) that generates an early stop codon at position 256;
- the two alleles of the gene Nitab4.5_0003572g0010.1 (NtSPL9/15_1a) comprising, or consisting of, nucleotide sequence SEQ ID NO: 34 (sequence with the mutation 289insC) that generates an early stop codon at position 301;
- the two alleles of the gene Nitab4.5_0000016g0300.1 (NtSPL9/15_1b) comprising, or consisting of, nucleotide sequence SEQ ID NO: 35 (sequence with the mutation 490insT) that generates an early stop codon at position 502;
- the two alleles of the gene Nitab4.5_0001538g0080.1 (2a_SPL9/15) comprising, or consisting of, nucleotide sequence SEQ ID NO: 40 (sequence with the mutation 217delAG) that generates an early stop codon at position 226;
- the two alleles of the gene Nitab4.5_0002299g0030.1 (1a_SPL13) comprising, or consisting of, nucleotide sequence SEQ ID NO: 49 (sequence with the mutations 135delTAAAGT + 274insA) that generates a deletion of two amino acids at position 133 and an early stop codon at position 352;
- one allele of the gene Nitab4.5_0001010g0010.1 (1b_SPL13) comprising, or consisting of, nucleotide sequence SEQ ID NO: 13 and the other allele comprising, or consisting of, nucleotide sequence SEQ ID NO: 57 (sequence with the mutations 123delT + 275deIGTCGA) that generates an early stop codon at position 217; or one allele of the gene Nitab4.5_0001010g0010.1 (1b_SPL13) comprising, or consisting of, nucleotide sequence SEQ ID NO: 58 (sequence with the mutation 123delT) that generates an early stop codon at position 217, and the other allele comprising, or consisting of, nucleotide sequence SEQ ID NO: 59 (sequence with the mutation 275delGTCGA) that generates an early stop codon at position 289;
- one allele of the gene Nitab4.5_0000861g0050.1 (1b_SPL2/10/11) comprising, or consisting of, nucleotide sequence SEQ ID NO: 74 (sequence with the mutations 752insA) that generates an early stop codon at position 781, and the other allele comprising, or consisting of, nucleotide sequence SEQ ID NO: 82 (sequence with the mutation 693insT + 746delAGTTCAACTCGGCA) that generates an early stop codon at position 703;
- the two alleles of the gene Nitab4.5_0000819g0010.1 (1a_FT5) comprising, or consisting of, nucleotide sequence SEQ ID NO: 86 (sequence with the mutation 258insT) that generates an early stop codon at position 271;
   and
- the two alleles of the gene Nitab4.5_0000751g0180.1 (1b_FT5) comprising, or consisting of, nucleotide sequence SEQ ID NO: 91 (sequence with the mutation 258insC) that generates an early stop codon at position 271.

### mFTSPLe line

In other preferred embodiment, the plant of the invention (also discloses as the mFTSPLe line) comprises:
**(a)** a biallelic homozygous mutation in the coding region of all following genes (the same mutation is in both alleles of the gene):
   - gene Nitab4.5_0001752g0040.1 (2a_SPL3/4/5) comprising, or consisting of, nucleotide sequence SEQ ID NO: 1, wherein the mutation in the nucleotide sequence SEQ ID NO: 1 is a deletion of nucleotides ACAAC at position 353 (353delACAAC);
   - gene Nitab4.5_0003348g0050.1 (6a_SPL3/4/5) comprising, or consisting of, nucleotide sequence SEQ ID NO: 4, wherein the mutation in the nucleotide sequence SEQ ID NO: 4 is a deletion of the nucleotide C at position 213 (213delC);
   - gene Nitab4.5_0003942g0050.1 (6b_SPL3/4/5) comprising, or consisting of, nucleotide sequence SEQ ID NO: 5, wherein the mutation in the nucleotide sequence SEQ ID NO: 5 is an insertion of nucleotide T between position 436 and 437 and a deletion of nucleotide C at position 225 (225deIC + 437insT);
   - gene Nitab4.5_0007487g0020.1 (7a_SPL3/4/5) comprising, or consisting of, nucleotide sequence SEQ ID NO: 6, wherein the mutation in the nucleotide sequence SEQ ID NO: 6 is a deletion of the nucleotides CA at position 204 (204deICA);
   - gene Nitab4.5_0002219g0060.1 (SPL3/4/5) comprising, or consisting of, nucleotide sequence SEQ ID NO: 7, wherein the mutation in the nucleotide sequence SEQ ID NO: 7 is an insertion of nucleotide A between position 212 and 213 (213insA);
   - gene Nitab4.5_0003572g0010.1 (1a_SPL9/15) comprising, or consisting of, nucleotide sequence SEQ ID NO: 8, wherein the mutation in the nucleotide sequence SEQ ID NO: 8 is an insertion of nucleotide C between position 288 and 289 (289insC);
   - gene Nitab4.5_0000016g0300.1 (1b_SPL9/15) comprising, or consisting of, nucleotide sequence SEQ ID NO: 9, wherein the mutation in the nucleotide sequence SEQ ID NO: 9 is an insertion of nucleotide T between position 489 and 490 (490insT);
   - gene Nitab4.5_0002299g0030.1 (1a_SPL13) comprising, or consisting of, nucleotide sequence SEQ ID NO: 12, wherein the mutation in the nucleotide sequence SEQ ID NO: 12 is an insertion of nucleotide T between position 273 and 274 (274insT);
   - gene Nitab4.5_0001010g0010.1 (1b_SPL13) comprising, or consisting of, nucleotide sequence SEQ ID NO: 13, wherein the mutation in the nucleotide sequence SEQ ID NO: 13 is an insertion of nucleotide T between position 276 and 277 (277insT);
   - gene Nitab4.5_0003900g0020.1 (1a_SPL2/10/11) comprising, or consisting of, nucleotide sequence SEQ ID NO: 14, wherein the mutation in the nucleotide sequence SEQ ID NO: 14 is a deletion of nucleotides CCACAGCAGGAAACCATCCAGTTCA at position 733 and an insertion of nucleotides GC between position 732 and 733 (733deICCACAGCAGGAAACCATCCAGTTCAinsGC);
   - gene Nitab4.5_0000819g0010.1 (1a_FT5) comprising, or consisting of, nucleotide sequence SEQ ID NO: 18, wherein the mutation in the nucleotide sequence SEQ ID NO: 18 is an insertion of nucleotide T between position 257 and 258 (258insT); and
   - gene Nitab4.5_0000751g0180.1 (1b_FT5) comprising, or consisting of, nucleotide sequence SEQ ID NO: 19, wherein the mutation in the nucleotide sequence SEQ ID NO: 19 is an insertion of nucleotide C between position 257 and 258 (258insC);
   **and**
**(b)** a biallelic heterozygous mutation in the coding region of the following genes:
   - gene Nitab4.5_0000638g0040.1 (2b_SPL3/4/5) comprising, or consisting of, nucleotide sequence SEQ ID NO: 2, wherein the mutation in the nucleotide sequence SEQ ID NO: 2 is a deletion of nucleotides AA at position 355 (355delAA) or a deletion of nucleotides GGACACAA at position 349 (349deIGGACACAA).

In other more preferred embodiment, the plant of the invention (mFTSPLe line) comprising, or consisting of:
- the two alleles of the gene Nitab4.5_0001752g0040.1 (NtSPL3/4/5_2a) comprising, or consisting of, nucleotide sequence SEQ ID NO: 20 (sequence with the mutation 353delACAAC) that generates an early stop codon at position 418;
- one allele of the gene Nitab4.5_0000638g0040.1 (NtSPL3/4/5_2b) comprising, or consisting of, nucleotide sequence SEQ ID NO: 21 (sequence with the mutation 349delGGACACAA) that generates an early stop codon at position 391; and the other allele comprising, or consisting of, nucleotide sequence SEQ ID NO: 22 (sequence with the mutation 355delAA) that generates an early stop codon at position 397;
- the two alleles of the gene Nitab4.5_0003348g0050.1 (NtSPL3/4/5_6a) comprising, or consisting of, nucleotide sequence SEQ ID NO: 25 (sequence with the mutation 213delC) that results in a frameshift and a delayed stop codon;
- the two alleles of the gene Nitab4.5_0003942g0050.1 (NtSPL3/4/5_6b) comprising, or consisting of, nucleotide sequence SEQ ID NO: 31 (sequence with the mutation 225delC + 437insT) that results in a frameshift and a delayed stop codon;
- the two alleles of the gene Nitab4.5_0007487g0020.1 (NtSPL3/4/5_7a) comprising, or consisting of, nucleotide sequence SEQ ID NO: 32 (sequence with the mutation 204delCA) that generates an early stop codon at position 244;
- the two alleles of the gene Nitab4.5_0002219g0060.1 (NtSPL3/4/5_7b) comprising, or consisting of, nucleotide sequence SEQ ID NO: 33 (sequence with the mutation 213insA) that generates an early stop codon at position 256;
- the two alleles of the gene Nitab4.5_0003572g0010.1 (NtSPL9/15_1a) comprising, or consisting of, nucleotide sequence SEQ ID NO: 34 (sequence with the mutation 289insC) that generates an early stop codon at position 301;
- the two alleles of the gene Nitab4.5_0000016g0300.1 (NtSPL9/15_1b) comprising, or consisting of, nucleotide sequence SEQ ID NO: 35 (sequence with the mutation 490insT) that generates an early stop codon at position 502;
- the two alleles of the gene Nitab4.5_0002299g0030.1 (1a_SPL13) comprising, or consisting of, nucleotide sequence SEQ ID NO: 45 (sequence with the mutations 274insT) that generates an early stop codon at position 358;
- the two alleles of the gene Nitab4.5_0001010g0010.1 (1b_SPL13) comprising, or consisting of, nucleotide sequence SEQ ID NO: 51 (sequence with the mutations 277insT) that generates an early stop codon at position 295;
- the two alleles of the gene Nitab4.5_0003900g0020.1 (1a_SPL2/10/11) comprising, or consisting of, nucleotide sequence SEQ ID NO: 70 (sequence with the mutations 733deICCACAGCAGGAAACCATCCAGTTCAinsGC) that generates an early stop codon at position 763;
- the two alleles of the gene Nitab4.5_0000819g0010.1 (1a_FT5) comprising, or consisting of, nucleotide sequence SEQ ID NO: 86 (sequence with the mutation 258insT) that generates an early stop codon at position 271;
   and
- the two alleles of the gene Nitab4.5_0000751g0180.1 (1b_FT5) comprising, or consisting of, nucleotide sequence SEQ ID NO: 91 (sequence with the mutation 258insC) that generates an early stop codon at position 271.

### Method for producing the plant of the invention

Other aspect of the present invention relates to a method for producing the plant of the invention (all the SPL and FTSPL lines), comprising subjecting the plant belonging to *Nicotiana tabacum* or part thereof, the reproductive or propagating plant material, or the plant cell, hereinafter "the method of the invention", to directed mutagenesis introducing:
(a) at least one monoallelic heterozygous mutation, biallelic heterozygous mutation or biallelic homozygous mutation in the coding region of the following genes (all SPL genes in (a) comprising at least one mutation):
   - gene Nitab4.5_0001752g0040.1 (2a_SPL3/4/5) comprising, or consisting of, nucleotide sequence SEQ ID NO: 1,
   - gene Nitab4.5_0000638g0040.1 (2b_SPL3/4/5) comprising, or consisting of, nucleotide sequence SEQ ID NO: 2,
   - gene Nitab4.5_0003348g0050.1 (6a_SPL3/4/5) comprising, or consisting of, nucleotide sequence SEQ ID NO: 4,
   - gene Nitab4.5_0007487g0020.1 (7a_SPL3/4/5) comprising, or consisting of, nucleotide sequence SEQ ID NO: 6,
   - gene Nitab4.5_0002219g0060.1 (7b_SPL3/4/5) comprising, or consisting of, nucleotide sequence SEQ ID NO: 7,
   - gene Nitab4.5_0003572g0010.1 (1a_SPL9/15) comprising, or consisting of, nucleotide sequence SEQ ID NO: 8, and
   - gene Nitab4.5_0000016g0300.1 (1b_SPL9/15) comprising, or consisting of, nucleotide sequence SEQ ID NO: 9,
   - gene Nitab4.5_0002299g0030.1 (1a_SPL13) comprising, or consisting of, nucleotide sequence SEQ ID NO: 12, and
   - gene Nitab4.5_0001010g0010.1 (1b_SPL13) comprising, or consisting of, nucleotide sequence SEQ ID NO: 13,
   and
(b) at least one monoallelic heterozygous mutation, biallelic heterozygous mutation or biallelic homozygous mutation in the coding region of at least one of the genes selected from the list consisting of (at least one SPL gene in (b) comprising at least one mutation):
   - gene Nitab4.5_0004959g0040.1 (4b_SPL3/4/5) comprising, or consisting of, nucleotide sequence SEQ ID NO: 3,
   - gene Nitab4.5_0003942g0050.1 (6b_SPL3/4/5) comprising, or consisting of, nucleotide sequence SEQ ID NO: 5,
   - gene Nitab4.5_0001538g0080.1 (2a_SPL9/15) comprising, or consisting of, nucleotide sequence SEQ ID NO: 10,
   - gene Nitab4.5_0000991g0020.1 (2b_SPL9/15) comprising, or consisting of, nucleotide sequence SEQ ID NO: 11,
   - gene Nitab4.5_0003900g0020.1 (1a_SPL2/10/11) comprising, or consisting of, nucleotide sequence SEQ ID NO: 14,
   - gene Nitab4.5_0000861g0050.1 (1b_SPL2/10/11) comprising, or consisting of, nucleotide sequence SEQ ID NO: 15,
   - gene Nitab4.5_0001315g0110.1 (3a_SPL2/10/11) comprising, or consisting of, nucleotide sequence SEQ ID NO: 16, and
   - gene Nitab4.5_0007217g0040.1 (3b_SPL2/10/11) comprising, or consisting of, nucleotide sequence SEQ ID NO: 17, and any combination thereof,
wherein the plant or part thereof, reproductive or propagating plant material, or a plant cell, is not obtained by means of an essentially biological process.

The "mSPL and mFTSPL lines" have been previously described and said plant lines is equally applicable for the present aspect as well as its embodiments.

The term "directed mutagenesis" or "site-directed mutagenesis", as used herein, refers to a molecular biology method that is used to make specific and intentional mutating changes to the DNA sequence of a gene and any gene products.

There are numerous methods for achieving site-directed mutagenesis. Examples of methods for achieving directed mutagenesis include, without limitation to, homologous recombination-dependent gene targeting, antisense RNA, directed transposon insertion, virus induced gene silencing or genome editing techniques.

Thus, in a preferred embodiment of the method of the invention the directed mutagenesis is achieved by homologous recombination-dependent gene targeting, directed transposon insertion, or genome editing techniques.

In other preferred embodiment of the method of the invention, the genome editing technique is CRISPR/Cas technique. The system is carried out by using the vector comprising a guide RNA (gRNA) that targets a specific DNA sequence, and a Cas enzyme that cleaves the DNA at the targeted site. The resulting double-stranded break can be repaired by the plant cell's DNA repair machinery, either through non-homologous end joining (NHEJ) or homology-directed repair (HDR), being NHEJ the preferred pathway for DNA reparation in plants. However, NHEJ can sometimes result in errors, such as small insertions or deletions (indels), that can disrupt the function of the targeted gene. CRISPR/Cas ability to target several loci simultaneously (multiplexing) by delivering multiple gRNAs accelerates plant breeding, especially for traits hidden by functional redundancy, such as SPL genes, in polyploid crops, such as tobacco.

In other more preferred embodiment of the method of the invention, the CRISPR/Cas technique is carried out by using the vector comprising, or consisting of, the nucleotide sequence SEQ ID NO: 92, SEQ ID NO: 93, SEQ ID NO: 94 or SEQ ID NO: 95.

As has been described in the present document, the *N*. *tabacum* lines were obtained by editing multiple genes of the K326 tobacco variety, preferably with two sequential rounds (multiSPL and multiFTSPL), of Agrobacterium-mediated genetic transformation with CRISPR/Cas9 constructs.

Thus, in other preferred embodiment, the method of the invention further comprises Agrobacterium-mediated genetic transformation, preferably in combination with CRISPR/Cas technique. Agrobacterium-mediated transformation is widely used for introducing DNA into plant cells, including CRISPR/Cas components for genome editing. The method involves the use of a natural plant pathogen, *Agrobacterium tumefaciens,* which transfers a portion of its DNA (T-DNA) into the host plant genome. The T-DNA can be engineered to carry the CRISPR/Cas components, such as one or multiple gRNAs and the Cas enzyme, in addition to a selectable marker cassette. Upon infection of tobacco leaf discs, the T-DNA is integrated into the plant genome, resulting in the expression of the CRISPR/Cas system and the selectable marker. Tobacco leaf discs are cultured on a selective regeneration medium containing (i) antibiotics or herbicides that kill the untransformed cells and select for the transformed cells that carry the selectable marker gene, such as 100 mg/L Kanamycin and (ii) plant growth regulators, 0.1 mg/L naphthaleneacetic acid (NAA) and 1 mg/L benzylaminopurine (BAP). The transformed cells that survive on the selective medium form callus tissue from which shoot-like structures regenerate. These shoot structures develop into shoots that can be transferred to a rooting medium (selective medium without plant growth regulators) for root induction and complete plant regeneration.

Regenerated plants can be analysed for editions at the targeted loci by total genomic DNA extraction followed by Polymerase Chain Reaction amplification of the targeted sites and Sanger sequencing.

### Method for producing the recombinant products

As has been described in the present document, for determining the recombinant production capacity, the mSPL and mFTSPL *N*. *tabacum* lines of the invention were agroinfiltrated with a gene construct containing the gene that codifies a recombinant products, particularly GFP, preferably under the control of a constitutive promoter (35SCaMV).

So, another aspect of the invention relates to a method for producing recombinant products, hereinafter "the second method of the invention", comprising:
- transforming the plant of the invention with a strain belongs to genus *Agrobacterium,* comprising a vector, wherein said vector comprising an expression cassette and wherein the expression cassette comprising a gene encoding at least one recombinant product.

In other preferred embodiment of the second method of the invention, the strain belongs to genus *Agrobacterium* is *Agrobacterium tumefaciens.*

In other preferred embodiment of the second method of the invention, the strain of *Agrobacterium tumefaciens* is strain LBA4404, GV3101, AGL1 or EHA105.

As used herein, the term "expression cassette" has its general meaning in the art. It refers to a nucleic acid construct, which, when present in a given cell under suitable condition, enables the expression of a gene of interest. According to the present invention, said gene of interest is gene encoding a recombinant product which one wishes to produce and collect.

The expression cassette can be contained in any suitable expression vector. Typically, the expression may be a binary vector, such as the pCAMBIA, pLXB, pGREEN or pLXZ vector, which has been modified to include the gene encoding the recombinant product of interest.

In other preferred embodiment of the second method of the invention, the expression cassette comprises a promoter, a signal peptide, a terminator region, a polyadenylation sequence and/or an infective viral clone and a gene encoding at least one recombinant product.

In other preferred embodiment of the second method of the invention, the promoter is a promoter derived from a Brassicaceae plant-infecting virus (Cauliflower Mosaic Virus).

In other more preferred embodiment of the second method of the invention, the promoter is the cauliflower mosaic virus 358 (35SCaMV) promoter.

As knows the skilled person in the art, the infective clones derived from DNA viruses can be used in the field of biofactories, and in particular of vectors derived from viruses of the geminivirus family. These DNA viruses present, among other favorable characteristics, a greater cargo capacity to carry foreign DNA and a greater ease of producing several recombinant products simultaneously.

So, in other preferred embodiment of the second method of the invention, the vector is a geminivirus vector, preferably wherein the virus is the Bean Yellow Dwarf Virus (BeYDV).

In other more preferred embodiment of the second method of the invention, the vector, preferably the BeYDV vector, comprises a gene encoding an antibody against the spike protein of the SARSCov2 virus, wherein the vector comprises the nucleotide sequence SEQ ID NO: 98.

In other more preferred embodiment of the second method of the invention, the recombinant product is a recombinant protein, a recombinant nucleic acid, or a recombinant metabolite.

### Use of the plant of the invention

As has been described in the present document, increased recombinant protein production has been obtained with mSPL and mFTSPL lines, preferably with mSPL4 and mFTSPLb (Figure 3 and 6). In addition, the present invention defines combinations of mutations in tobacco SPL and FT genes which confer to the tobacco plant a maximum capacity of recombinant products (proteins, nucleic acids, or metabolites) production, in particular by means of the geminivirus-mediated agroinfiltration technique, and also extends indefinitely over time the production capacity of recombinant product in the leaves.

So, another aspect of the present invention relates to the use of the plant of the invention for producing recombinant products, preferably the recombinant product is a protein, a nucleic acid, or a metabolite.

As used, the expression "recombinant protein" or "heterologous protein" or "protein of interest" are used interchangeably to refer to a protein which is usually not expressed by the plant belonging to the *N*. *tabacum,* or not expressed at significant levels. Suitable recombinant proteins according to the invention include, but are not limited to, allergens, vaccines, enzymes, enzyme inhibitors, antibodies, antibody fragments, antigens, toxins, anti-microbial peptides, hormones, growth factors, blood proteins (such as albumin, coagulation factors, transferrin), receptors and signaling proteins, components of gene and cell therapy vectors, protein component of biomedical standards, protein component of cell culture media, fusion or tagged proteins, cystein (disulfide bridges)-rich peptides and proteins, and glycosylated plant proteins (such as lectins, papain .. ).

### DESCRIPTION OF THE DRAWINGS

**Figure 1****.** Mutations of the mSPL, mFTSPL and mFT T1 lines.
**Figure 2****.** Phenotype of the mSPL, mFTSPL and mFT T2 mutants. **A)** Length/width ratio of leaf 3-4 measured at 49 days after sowing. **B)** Length/width ratio of leaf 13-14 measured at 77 days after sowing. **C)** Picture of a selection of leaves collected at 49 and 77 days after sowing (DAS). **D)** Days from sowing to anthesis. For the plants that did not flower (mFTSPL a-e and mFT), 168 days (the final time of the experiment) was used. **E)** Number of juvenile leaves. For lines mSPL and mFTSPL this number corresponds to the total number of leaves at flowering or final time of the experiment. For A), B), D) and E), error bars indicate standard deviation (n=3). Statistical analyses were performed using One-way ANOVA (Tukey's multiple comparisons test, P-Value ≤ 0.05). Variables within the same statistical groups are marked with the same letters.
**Figure 3****.** Production of recombinant protein (GFP) (measured as fluorescence) in the tobacco mSPL, mFTSPL and mFT lines after agroinfiltration with 35s:eGFP:tNos. Error bars indicate standard deviation (n=4). Statistical analyses were performed using One-way ANOVA (Tukey's multiple comparisons test, P-Value ≤ 0.05). Variables within the same statistical groups are marked with the same letters.
**Figure 4****.** Regeneration capacity of mFTSPLb. Explants of WT and mFTSPLb at 7 weeks after transformation with plasmid GB_UA_7118.

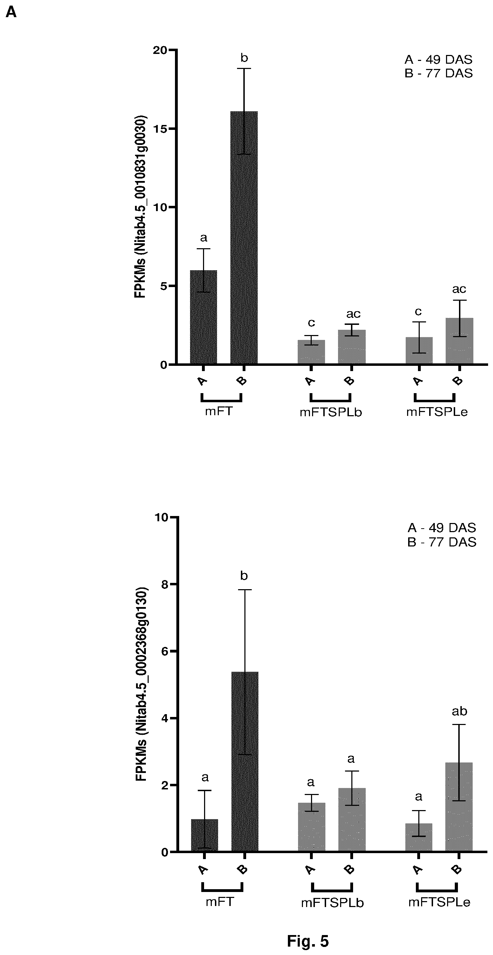

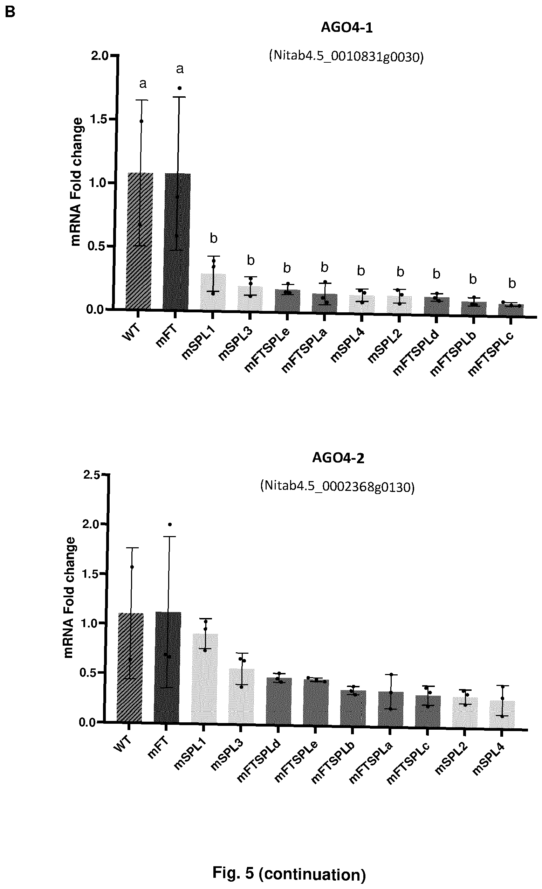

**Figure 5****.** AGO4 expression in mFT, mSPL and mFTSPL lines. **A)** Fragments per kilobase of exon per million mapped fragments (FPKMs) for Nitab4.5_0010831g0030 and Nitab4.5_0002368g0130 in RNA samples obtained from leaf 3-4 (collected at 49 days after sowing "A-49 DAS") and leaf 13-14 (collected at 77 days after sowing, "B-77DAS") for lines mFT, mFTSPLb and mFTSPLe. Error bars indicate standard deviation (n=3). Statistical analyses were performed using One-way ANOVA (Tukey's multiple comparisons test, P-Value ≤ 0.05). Variables within the same statistical groups are marked with the same letters. **B)** Nitab4.5_0010831g0030 and Nitab4.5_0002368g0130 mRNA fold change (compared to WT) for samples obtained from leaf 13-14 (collected at 77 days after sowing) for all mSPL, mFTSPL and mFT lines. Error bars indicate standard deviation. Statistical analyses were performed using One-way ANOVA (Tukey's multiple comparisons test, P-Value ≤ 0.05). Variables within the same statistical groups are marked with the same letters.
**Figure 6****.** Production of recombinant protein in the tobacco mSPL, mFTSPL and mFT lines using geminivirus derived vectors. **A)** Fluorescence levels after agroinfiltration with LIR:35s:eGFP:tNos:SIR:LIR and pNos:BeYDVRep/RepA:tNos. Error bars indicate standard deviation (n=4). Statistical analyses were performed using One-way ANOVA (Tukey's multiple comparisons test, P-Value ≤ 0.05). Variables within the same statistical groups are marked with the same letters. **B)** Image showing the differential fluorescence levels between WT and mFTSPLb after agroinfiltration with LIR:35s:eGFP:tNos:SIR:LIR and pNos:BeYDVRep/RepA:tNos. **C)** Recombinant expression of a single chain antibody against SARS-Cov-2 RBD domain in WT and mFTSPLb using a BeYDV derived vector.

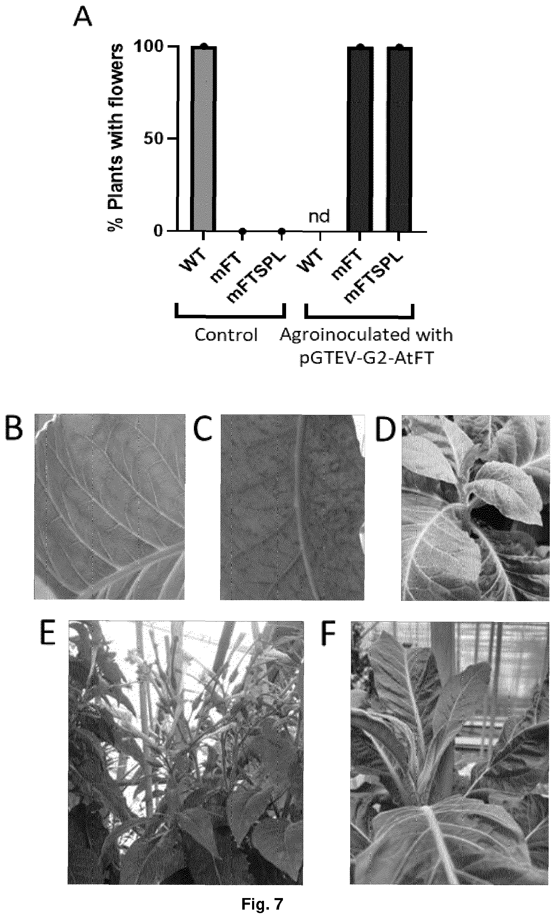

**Figure 7****.** Reversion of flowering in mFT and mFTSPL tobacco plants by delivering Arabidopsis thaliana FT via agroinoculation with a Tobacco Etch Virus (TEV) based vector. **A)** % of plants that flowered after agroinfiltration with pGTEV-G2-AtFT. Control plants were not agroinfiltrated (n=25). "nd" indicates no data. **B)** Photograph of the abaxial side of a control leaf. **C)** Photograph of the abaxial side of a pGTEV-G2-AtFT infected leaf showing viral infection symptoms (mottling). Photographs were taken 15 days after agroinoculated with TEV-AtFT. **D)** Photograph of a plant infected with TEV-AtFT showing viral infection symptoms (vein clearing). Photograph was taken 20 days after agroinoculation with TEV-AtFT. **E)** mFT and mFTSPL plants agroinoculated with pGTEV-G2-AtFT. **F)** Non agroinoculated mFT plant.

### EXAMPLES

### A. Breeding multiSPL, multiFT and multiFTSPL N. tabacum lines

The improved *N. tabacum* lines were obtained by editing multiple genes of the K326 tobacco variety. Gene edited lines were obtained by either one (multiFT) or two sequential rounds (multiSPL and multiFTSPL) of Agrobacterium-mediated genetic transformation with CRISPR/Cas9 constructs and subsequent segregation of transgenes.

### A.1. Parental line for breeding mSPL and mFTSPL lines: mSPL1

mSPL1 is a non-transgenic line obtained by gene editing (CRISPR/Cas9) of a wild type plant of the commercial variety K326 using the GB2138 (SEQ ID NO: 92) construct that contains gRNAs targeting several SPL genes (Table 1).

**Table 1. List of gRNAs used to generate the edited lines.**

| **gRNA short name** | **gRNA sequence (5'-3')** | **SEQ ID NO** | **Targeted genes** | **Plasmid including the gRNA** |
|---|---|---|---|---|
| gFT1 | AGGGAAGTCAACAA TGCATG | SEQ ID NO: 99 | *Nitab4.5_0010534g0010.1* | GB2710, GB2713 |
| | | | *Nitab4.5_0000971g0160.1* | |
| gFT2 | ATCAGGATCCACCA TAACCT | SEQ ID NO: 100 | *Nitab4.5_0010534g0010.1* | GB2710, GB2713 |
| | | | *Nitab4.5_0000971g0160.1* | |
| gFT3 | AGTTTGTATCTGTAG TTGCT | SEQ ID NO: 101 | *Nitab4.5_0008339g0030.1* | GB2710, GB2713 |
| gFT4 | AAGGAAGTCAACAA TGCATG | SEQ ID NO: 102 | *Nitab4.5_0008339g0010.1* | GB2710, GB2713 |
| | | | *Nitab4.5_0011525g0020.1* | |
| gFT5 | CCAGAGTGTAAAAG GTACGA | SEQ ID NO: 103 | *Nitab4.5_0000751g0180.1* | GB2710, GB2713 |
| | | | *Nitab4.5_0000819g0010.1* | |
| gFT6 | AGGACGATACTAAC CAATGG | SEQ ID NO: 104 | *Nitab4.5_0000751g0180.1* | GB2710, GB2713 |
| | | | *Nitab4.5_0000819g0010.1* | |
| gSPL2 | CTTCTGTTCAGAATG TGCAC | SEQ ID NO: 105 | *Nitab4.5_0000991g0020.1* | GB2138 |
| | | | *Nitab4.5_0001538g0080.1* | |
| | | | *Nitab4.5_0000016g0300.1* | |
| | | | *Nitab4.5_0003572g0010.1* | |
| gSPL5 | AAGGAAAAAGAAGT TGCCGC | SEQ ID NO: 106 | *Nitab4.5_0000991g0020.1* | GB2138 |
| | | | *Nitab4.5_0001538g0080.1* | |
| gSPL1 | CGATTAGACAAAAG AGATCC | SEQ ID NO: 107 | *Nitab4.5_0000016g0300.1* | GB2138 |
| | | | *Nitab4.5_0003572g0010.1* | |
| gSPL7 | CATGTGATTCTCCTG GAGAA | SEQ ID NO: 108 | *Nitab4.5_0003348g0050.1* | GB2138 |
| | | | *Nitab4.5_0003942g0050.1* | |
| gSPL6 | GAAATTCACAGACC TTGTGG | SEQ ID NO: 109 | *Nitab4.5_0003348g0050.1* | GB2138 |
| | | | *Nitab4.5_0003942g0050.1* | |
| gSPL8 | GAAACTCGCACACC TTGTGG | SEQ ID NO: 110 | *Nitab4.5_0002219g0060.1* | GB2138 |
| | | | *Nitab4.5_0007487g0020.1* | |
| gSPL9 | | SEQ ID NO: 111 | *Nitab4.5_0002219g0060.1* | GB2138 |
| | AATCATATGAAGTTT TACGG | | *Nitab4.5_0007487g0020.1* | |
| gSPL4 | GAGGGTCAAGTACT ACTCAA | SEQ ID NO: 112 | *Nitab4.5_0000638g0040.1* | GB2138 |
| gSPL3 | GTTTGGCTGGACAC AACGAA | SEQ ID NO: 113 | *Nitab4.5_0000638g0040.1* | GB2138 |
| | | | *Nitab4.5_0001752g0040.1* | |
| gSPL1.1 | CGACGGCTTGAGAC TTTGCA | SEQ ID NO: 114 | *Nitab4.5_0006721g0040.1* | GB2713, GB2714 |
| gSPL1.2 | AATCGAAAAGGAGT TGCAGG | SEQ ID NO: 115 | *Nitab4.5_0006721g0040.1* | GB2713, GB2714 |
| gSPL1.3 | AAGGGTCAAGTACT TCAATG | SEQ ID NO: 116 | *Nitab4.5_0004959g0040.1* | GB2713, GB2714 |
| gSPL1.4 | CGACGGCTTGAGCC TTTGCA | SEQ ID NO: 117 | *Nitab4.5_0004959g0040.1* | GB2713, GB2714 |
| gSPL1.5 | GGACCTCACAAACT TTATGG | SEQ ID NO: 118 | *Nitab4.5_0002299g0030.1* | GB2713, GB2714 |
| | | | *Nitab4.5_0001010g0010.1* | |
| gSPL1.6 | ATGGACATAACAGG CGTCGA | SEQ ID NO: 119 | *Nitab4.5_0002299g0030.1* | GB2713, GB2714 |
| | | | *Nitab4.5_0001010g0010.1* | |
| gSPL2.1 | CGAACAGTTGAAAG CAACAG | SEQ ID NO: 120 | *Nitab4.5_0000067g0130.1* | GB2713, GB2714 |
| | | | *Nitab4.5_0002558g0020.1* | |
| gSPL2.2 | TCATCATAGGAGGT GCCGAG | SEQ ID NO: 121 | *Nitab4.5_0000067g0130.1* | GB2713, GB2714 |
| | | | *Nitab4.5_0002558g0020.1* | |
| gSPL2.3 | TAGAAATTCACCCC CCATGG | SEQ ID NO: 122 | *Nitab4.5_0000861g0050.1* | GB2713, GB2714 |
| | | | *Nitab4.5_0003900g0020.1* | |
| | | | *Nitab4.5_0007217g0040.1* | |
| gSPL2.4 | TGTGATCAGAGAGC CTCCTG | SEQ ID NO: 123 | *Nitab4.5_0000861g0050.1* | GB2713, GB2714 |
| gSPL2.5 | GGAAACCATCCAGT TCAACT | SEQ ID NO: 124 | *Nitab4.5_0003900g0020.1* | GB2713, GB2714 |
| | | | *Nitab4.5_0000861g0050.1* | |
| gSPL3.1 | TTAAAGGGGCATCA GTCCAA | SEQ ID NO: 125 | *Nitab4.5_0003348g0050.1* | GB2713, GB2714 |
| | | | *Nitab4.5_0003942g0050.1* | |
| gSPL3.2 | ATCTACTTTGAAAAT GTGGG | SEQ ID NO: 126 | *Nitab4.5_0000991g0020.1* | GB2713, GB2714 |
| | | | *Nitab4.5_0001538g0080.1* | |
| gSPL3.3 | | SEQ ID NO: 127 | *Nitab4.5_0003348g0050.1* | |
| | GAAATTCACAGACC TTGTGG | | *Nitab4.5_0003942g0050.1* | GB2713, GB2714 |
| gSPL3.4 | CAGTCATTCCAAATG CCCAA | SEQ ID NO: 128 | *Nitab4.5_0001315g0110.1* | GB2713, GB2714 |
| | | | *Nitab4.5_0007217g0040.1* | |
| | | | *Nitab4.5_0003900g0020.1* | |
| | | | *Nitab4.5_0000067g0130.1* | |
| | | | *Nitab4.5_0002558g0020.1* | |
| gSPL3.5 | AGATGGAGTGGAAT GCAAAG | SEQ ID NO: 129 | *Nitab4.5_0001315g0110.1* | GB2713, GB2714 |
| | | | *Nitab4.5_0003900g0020.1* | |
| | | | *Nitab4.5_0000861g0050.1* | |
| gSPL3.6 | CCTTCAACTTGACAC CTAGG | SEQ ID NO: 130 | *Nitab4.5_0000991g0020.1* | *GB2713, GB2714* |
| | | | *Nitab4.5_0001538g0080.1* | |

After self-pollination of primary transformants, T-DNA free T1 plants were screened for maximum number of mutations and mSPL1 was selected. mSPL1 contains mutations in seven genes of the SPLs family (Figure 1 and Table 2). The mSPL1 line, used as the parental line of the multiSPL and multiFTSPL lines, has mutations in 7 SPLs belonging to groups SPL3/4/5 and SPL9/15. The mSPL1 line showed only some slight phenotypic differences in flowering time from the wild type line K326.

### A.2. mSPL lines

The modifications introduced in the multiSPL (mSPL) lines consist of mutations (deletions or insertions) in genes belonging to the family of SPL (SQUAMOSA promoter-binding protein-like) transcription factors. These lines were obtained by genetic editing (CRISPR/Cas9) of the tobacco line cv. K326 mSPL1 using the GB2714 construct (SEQ ID NO: 93). As previously explained, mSPL1 is a non-transgenic line generated with CRISPR/Cas9 in a previous round of Agrobacterium-mediated transformation using the GB2138 construct and containing mutations in seven genes of the SPLs family (Table 2). In addition to the mSPL1 mutations, the mSPL lines present mutations in several combinations of the previously described SPL genes.

After Agrobacterium mediated transformation with GB2714, 20 T0 lines containing mutations in genes of the SPL family were selected. These T0 plants were self-pollinated giving rise to a T1 generation in which the transgenic construct containing the CRISPR/Cas9 nuclease was segregated. Four transgene-free T1 plants (mSPL 2, 3 and 4) were selected and their T2 offspring was phenotypically analyzed in the greenhouse. The genotype of the T1 mSPL2, mSPL3 and mSPL4 plants is shown in Table 2.

**Table 2. Genotype of the T1 mSPL1, mSPL2, mSPL3 and mSPL4 lines. ^{(a)} Position and nature of the mutation. The number indicates the position (with respect to the coding sequence) of the first nucleotide of the mutation; the sequence corresponds to the inserted or deleted nucleotides; ins: insertion; del: deletion. ^{(b)} +/+: biallelic mutation - both alleles of the gene have the same mutation; +/-: monoallelic mutation - the mutation is in one of the alleles of the gene.**

| **Edited genes** | **SEQ ID NO (WT)** | **Mutation** | **SEQ ID NO** | **SPL 1** | **SPL2** | **SPL3** | **SPL4** |
|---|---|---|---|---|---|---|---|
| Nitab4.5_0001752g 0040.1 | SEQ ID NO: 1 | 353 delACAAC | SEQ ID NO: 20 | +/+ | +/+ | +/+ | +/+ |
| **NtSPL3/4/5_2a** | | | | | | | |
| Nitab4.5_0000638g 0040.1 | SEQ ID NO: 2 | 349delGGACAC AA | SEQ ID NO: 21 | +/- | +/- | +/- | |
| **NtSPL3/4/5_2b** | | 355delAA | SEQ ID NO: 22 | +/- | +/- | +/- | +/+ |
| Nitab4.5_0004959g0040.1 | SEQ ID NO: 3 | 142 insA | SEQ ID NO: 23 | | | +/- | |
| **NtSPL3/4/5_4b** | | 142insA+225del CCATGCAAAG GCTCAA | SEQ ID NO: 24 | | | | +/- |
| Nitab4.5_0003348g 0050.1 | SEQ ID NO: 4 | 213delC | SEQ ID NO: 25 | +/+ | +/+ | +/+ | +/- |
| | | 213delC + 425insT | SEQ ID NO: 26 | | | | |
| **NtSPL3/4/5_6a** | | 213delC + 425insA | SEQ ID NO: 27 | | | | +/- |
| Nitab4.5_0003942g 0050.1 | SEQ ID NO: 5 | 437insT (out of CDS) | SEQ ID NO: 28 | | +/- | | |
| | | 225delC | SEQ ID NO: 29 | | +/- | | +/- |
| **NtSPL3/4/5_6b** | | 225insA | SEQ ID NO: 30 | | | | |
| | | 225delC + 437insT | SEQ ID NO: 31 | | +/- | | |
| Nitab4.5_0007487g 0020.1 | SEQ ID NO: 6 | 204delCA | SEQ ID NO: 32 | +/+ | +/+ | +/+ | +/+ |
| **NtSPL3/4/5 7a** | | | | | | | |
| Nitab4.5_0002219g 0060.1 | SEQ ID NO: 7 | 212insA | SEQ ID NO: 33 | +/+ | +/+ | +/+ | +/+ |
| **NtSPL3/4/5_7b** | | | | | | | |
| Nitab4.5_0003572g 0010.1 | SEQ ID NO: 8 | 289insC | SEQ ID NO: 34 | +/+ | +/+ | +/+ | +/+ |
| **NtSPL9/15_1a** | | | | | | | |
| Nitab4.5_0000016g 0300.1 | SEQ ID NO: 9 | 490insT | SEQ ID NO: 35 | +/+ | +/+ | +/+ | +/+ |
| **NtSPL9/15_1b** | | | | | | | |
| Nitab4.5_0001538g 0080.1 | SEQ ID NO: 10 | 217insA | SEQ ID NO: 36 | | | | +/- |
| | | 217delAGinsT | SEQ ID NO: 37 | | +/- | | |
| | | 117delAAATGT +217delAG | SEQ ID NO: 38 | | +/- | | |
| **NtSPL9/15_2a** | | 121delGT + 218delG | SEQ ID NO: 39 | | | | |
| | | 217delAG | SEQ ID NO: 40 | | | | |
| Nitab4.5_0000991g 0020.1 | SEQ ID NO: 11 | 217delA | SEQ ID NO: 41 | | | | |
| | | 217insA | SEQ ID NO: 42 | | +/- | | |
| **NtSPL9/15_2b** | | 217delAG | SEQ ID NO: 43 | | | | |
| Nitab4.5_0002299g 0030.1 | SEQ ID NO: 12 | 274insA | SEQ ID NO: 44 | | +/- | +/- | +/- |
| | | 274insT | SEQ ID NO: 45 | | +/- | +/- | |
| **NtSPL13_1a** | | 272delG | SEQ ID NO: 46 | | | | +/- |
| | | 269delGGCG | SEQ ID NO: 47 | | | | |
| | | 135delTA + 274insA | SEQ ID NO: 48 | | | | |
| | | 135delTAAAGT + 274insA | SEQ ID NO: 49 | | | | |
| | | 273delT | SEQ ID NO: 50 | | | | |
| Nitab4.5_0001010g 0010.1 | SEQ ID NO: 13 | 277insT | SEQ ID NO: 51 | | +/- | | |
| | | 277insC | SEQ ID NO: 52 | | +/- | | |
| | | 277insA | SEQ ID NO: 53 | | | +/+ | +/+ |
| | | 123insT+277ins G | SEQ ID NO: 54 | | | | |
| | | 123insT+277ins A | SEQ ID NO: 55 | | | | |
| **NtSPL13_1b** | | 277insG | SEQ ID NO: 56 | | | | |
| | | 123delT+275del GTCGA | SEQ ID NO: 57 | | | | |
| | | 123delT | SEQ ID NO: 58 | | | | |
| | | 275delGTCGA | SEQ ID NO: 59 | | | | |
| Nitab4.5_0003900g 0020.1 | SEQ ID NO: 14 | 754delTTC | SEQ ID NO: 60 | | +/- | | |
| | | 756delCA | SEQ ID NO: 61 | | +/- | | |
| | | 13delGCA+598d elC | SEQ ID NO: 62 | | | | +/- |
| | | 13delGCA+758i nsA | SEQ ID NO: 63 | | | | +/- |
| **NtSPL2/10/11_1a** | | 598delC | SEQ ID NO: 64 | | | | +/- |
| | | 758insA | SEQ ID NO: 65 | | | | +/- |
| | | 738delGCAGG AAACCATCCA GTTCAinsATTT AGTTGCAATTT TTGTCAAGGA | SEQ ID NO: 66 | | | | |
| | | TATCTACCTGC TAATAGCTTGC CTTTTAGCAAT TTTCGCAGGA GGCTCTCTGA TC | | | | | |
| | | 338delCCA | SEQ ID NO: 67 | | | | |
| | | 338delCCA+738 delGCAGGAAA CCATCCAGTT CAinsATTTAGT TGCAATTTTTG TCAAGGATAT CTACCTGCTA ATAGCTTGCC TTTTAGCAATT TTCGCAGGAG GCTCTCTGAT C | SEQ ID NO: 68 | | | | |
| | | 758delA | SEQ ID NO: 69 | | | | |
| | | 733delCCACAG CAGGAAACCA TCCAGTTCAins GC | SEQ ID NO: 70 | | | | |
| Nitab4.5_0000861g 0050.1 | SEQ ID NO: 15 | 693delGAGGCT CTCTGATCAC AATGCACGAC GCCGCAAACC ACAGCAGGAA ACCATCCAGT TCA | SEQ ID NO: 71 | | +/- | | |
| **NtSPL2/10/11_1b** | | 693delGAG+75 1delA | SEQ ID NO: 72 | | +/- | | |
| | | 693insT | SEQ ID NO: 73 | | | +/+ | |
| | | 752insA | SEQ ID NO: 74 | | | | +/- |
| | | 693delG+752ins A | SEQ ID NO: 75 | | | | |
| | | 693delG+749del TC | SEQ ID NO: 76 | | | | |
| | | 693delG+750ins A | SEQ ID NO: 77 | | | | |
| | | 693insT+752ins G | SEQ ID NO: 78 | | | | |
| | | 693insT+752ins T | SEQ ID NO: 79 | | | | |
| | | 693delGA+752i nsG | SEQ ID NO: 80 | | | | |
| | | 693delGA+752i nsT | SEQ ID NO: 81 | | | | |
| | | 693insT+746del AGTTCAACTC GGCA | SEQ ID NO: 82 | | | | |
| Nitab4.5_0001315g 0110.1 | SEQ ID NO: 16 | 193insA | SEQ ID NO: 83 | | +/+ | | |
| **NtSPL2/10/11_3a** | | | | | | | |
| Nitab4.5_0007217g 0040.1 | SEQ ID NO: 17 | 458delCACCCC CCA | SEQ ID NO: 84 | | +/- | | |
| **NtSPL2/10/11_3b** | | | | | | | |

### A.3. mFT line

In parallel, two lines with mutations only in the NtFT5 genes were generated. The mFT lines carry biallelic mutations in the homeologous genes NtFT5a and NtFT5b. NtFT5 is a photoperiod-independent activator of flowering belonging to the FT gene family (flowering locus T) and two FT5 genes were identified in K326 [Nitab4.5_0000751g0180.1 (NtFT5b), Nitab4.5_0000819g0010.1 (NtFT5a)].

These mFT lines were obtained by gene editing (CRISPR/Cas9) of a wild type plant of the commercial variety K326 using the GB2710 construct (SEQ ID NO: 94) that contains gRNAs targeting NtFT5a and NtFT5b genes (Table 3). In the primary transformants with biallelic edition in NtFT5a and NtFT5b genes, flowering was induced (see mechanism of flowering induction below) and they were self-pollinated obtaining the T1 generation. T1 plants were screened for the absence of the T-DNA and one plant for each line lacking the T-DNA was selected for flowering induction. Five plants of T2 lines derived from one of the lines (mFT) were analysed. The genotype of the mFT T1 plant is displayed in Figure 1 and Table 3.

**Table 3. Genotype of the T1 mFT line. ^{(a)} Position and nature of the mutation. The number indicates the position (with respect to the coding sequence) of the first nucleotide of the mutation; the sequence corresponds to the inserted or deleted nucleotides; ins: insertion; del: deletion. ^{(b)} +/+: biallelic mutation - both alleles of the gene have the same mutation; +/-: monoallelic mutation - the mutation is in one of the alleles of the gene.**

| **T1 Line** | **Edited genes** | **Gene short name** | **gRNA** | **Mutation^{a}** | **Mono/Biallelic^{b}** |
|---|---|---|---|---|---|
| **mFT** | *Nitab4.5_0000819g0010.1* | NtFT5_1a | gFT6 | 258insT | +/+ |
| | *Nitab4.5_0000751g0180.1* | NtFT5_1b | gFT6 | 258insT | +/+ |

### A.4. mFTSPL lines

The modifications introduced in the multiFTSPL (mFTSPL) lines consist of mutations (deletions or insertions) in genes belonging to both the NtSPL2/3/4/5/9/10/11/13/15 and the NtFT5 subfamilies. The mFTSPL lines were obtained by genetic editing (CRISPR/Cas9) of the tobacco line cv. K326 mSPL1 using the GB2713 construct (SEQ ID NO: 95). mSPL1, as described above, is a non-transgenic line generated by CRISPR/Cas9 using the GB2138 construct and containing mutations in seven genes of the SPLs family (Table 4). The mFTSPL lines present, in addition to the mSPL1 mutations, mutations in the NtFT5a and NtFT5b genes and in various combinations of the previously described SPL genes.

After Agrobacterium mediated transformation with GB2713, biallelic T0 lines for NtFT5a and NtFT5b were selected that also contained additional mutations in genes of the SPL family. These T0 plants, after flowering induction (see mechanism of flowering induction below), were self-pollinated giving rise to a T1 generation in which the transgenic construct containing the CRISPR/Cas9 nuclease was segregated. Five transgene-free T1 plants (mFTSPLa-e) were selected and their T2 offspring was phenotypically analyzed in the greenhouse. The genotype of the T1 mFTSPL plants is shown in Table 4.

**Table 4. Genotype of the T1 mFTSPL lines. ^{(a)} Position and nature of the mutation. The number indicates the position (with respect to the coding sequence) of the first nucleotide of the mutation; the sequence corresponds to the inserted or deleted nucleotides; ins: insertion; del: deletion. ^{(b)} +/+: biallelic mutation - both alleles of the gene have the same mutation; +/-: monoallelic mutation - the mutation is in one of the alleles of the gene.**

| **Edited genes** | **SEQ ID NO (WT)** | **Mutation** | **SEQ ID NO** | **FTSPL a** | **FTSPL b** | **FTSPL c** | **FTSPL d** | **FTSPL e** |
|---|---|---|---|---|---|---|---|---|
| Nitab4.5_0 001752g00 40.1 | SEQ ID NO: 1 | 353 delACAAC | SEQ ID NO: 20 | +/+ | +/+ | +/+ | +/+ | +/+ |
| NtSPL3/4/ 5_2a | | | | | | | | |
| Nitab4.5_0 000638g00 40.1 | SEQ ID NO: 2 | 349delGGA CACAA | SEQ ID NO: 21 | +/- | | +/- | +/- | +/- |
| NtSPL3/4/ 5_2b | | 355delAA | SEQ ID NO: 22 | +/- | +/+ | +/- | +/- | +/- |
| Nitab4.5_0 004959g00 40.1 | SEQ ID NO: 3 | 142 insA | SEQ ID NO: 23 | | +/+ | | | |
| NtSPL3/4/ 5_4b | | 142insA+225 delCCATGC AAAGGCTC AA | SEQ ID NO: 24 | | | | | |
| Nitab4.5_0 003348g00 50.1 | SEQ ID NO: 4 | 213delC | SEQ ID NO: 25 | +/+ | +/- | +/+ | +/+ | +/+ |
| | | 213delC + 425insT | SEQ ID NO: 26 | | +/- | | | |
| NtSPL3/4/5_6a | | 213delC + 425insA | SEQ ID NO: 27 | | | | | |
| Nitab4.5_0 003942g00 50.1 | SEQ ID NO: 5 | 437insT (out of CDS) | SEQ ID NO: 28 | | | | | |
| | | 225delC | SEQ ID NO: 29 | | | | | |
| NtSPL3/4/ 5_6b | | 225insA | SEQ ID NO: 30 | | +/+ | +/- | +/+ | |
| | | 225delC + 437insT | SEQ ID NO: 31 | | | | | +/+ |
| Nitab4.5_0 007487g00 20.1 | SEQ ID NO: 6 | 204delCA | SEQ ID NO: 32 | +/+ | +/+ | +/+ | +/+ | +/+ |
| NtSPL3/4/ 5_7a | | | | | | | | |
| Nitab4.5_0 002219g00 60.1 | SEQ ID NO: 7 | 212insA | SEQ ID NO: 33 | +/+ | +/+ | +/+ | +/+ | +/+ |
| NtSPL3/4/ 5_7b | | | | | | | | |
| Nitab4.5_0 003572g00 10.1 | SEQ ID NO: 8 | 289insC | SEQ ID NO: 34 | +/+ | +/+ | +/+ | +/+ | +/+ |
| NtSPL9/15 _1a | | | | | | | | |
| Nitab4.5_0 000016g03 00.1 | SEQ ID NO: 9 | 490insT | SEQ ID NO: 35 | +/+ | +/+ | +/+ | +/+ | +/+ |
| NtSPL9/15 _1b | | | | | | | | |
| Nitab4.5_0 001538g00 80.1 | SEQ ID NO: 10 | 217insA | SEQ ID NO: 36 | | +/+ | | | |
| | | 217delAGins T | SEQ ID NO: 37 | | | | | |
| | | 117delAAAT GT +217delAG | SEQ ID NO: 38 | | | | | |
| NtSPL9/15_2a | | 121delGT + 218delG | SEQ ID NO: 39 | | | +/- | | |
| | | 217delAG | SEQ ID NO: 40 | | | | +/+ | |
| Nitab4.5_0 000991g00 20.1 | SEQ ID NO: 11 | 217delA | SEQ ID NO: 41 | | +/+ | | | |
| | | 217insA | SEQ ID NO: 42 | | | | | |
| NtSPL9/15_2b | | 217delAG | SEQ ID NO: 43 | | | +/- | | |
| Nitab4.5_0 002299g00 30.1 | SEQ ID NO: 12 | 274insA | SEQ ID NO: 44 | | | | | |
| | | 274insT | SEQ ID NO: 45 | | | | | +/+ |
| | | 272delG | SEQ ID NO: 46 | +/- | | | | |
| | | 269delGGC G | SEQ ID NO: 47 | +/- | | | | |
| NtSPL13_ 1a | | 135delTA + 274insA | SEQ ID NO: 48 | | +/+ | | | |
| | | 135delTAAA GT + 274insA | SEQ ID NO: 49 | | | | +/+ | |
| | | 273delT | SEQ ID NO: 50 | | | +/- | | |
| Nitab4.5_0 001010g00 10.1 | SEQ ID NO: 13 | 277insT | SEQ ID NO: 51 | +/+ | | +/- | | +/+ |
| | | 277insC | SEQ ID NO: 52 | | | | | |
| | | 277insA | SEQ ID NO: 53 | | +/- | +/- | | |
| | | 123insT+277 insG | SEQ ID NO:54 | | +/- | | | |
| | | 123insT+277 insA | SEQ ID NO: 55 | | +/- | | | |
| NtSPL13_1b | | 277insG | SEQ ID NO: 56 | | +/- | | | |
| | | 123delT+275 delGTCGA | SEQ ID NO: 57 | | | | +/- | |
| | | 123delT | SEQ ID NO: 58 | | | | +/- | |
| | | 275delGTC GA | SEQ ID NO: 59 | | | | +/- | |
| Nitab4.5_0 003900g00 20.1 | SEQ ID NO: 14 | 754delTTC | SEQ ID NO: 60 | | | | | |
| | | 756delCA | SEQ ID NO: 61 | | | | | |
| NtSPL2/10/11_1a | | 13delGCA+5 98delC | SEQ ID NO: 62 | | | | | |
| | | 13delGCA+7 58insA | SEQ ID NO: 63 | | | | | |
| | | 598delC | SEQ ID NO: 64 | | | | | |
| | | 758insA | SEQ ID NO: 65 | | +/+ | | | |
| | | 738delGCA GGAAACCA TCCAGTTC AinsATTTA GTTGCAAT TTTTGTCA AGGATATC TACCTGCT AATAGCTT GCCTTTTA GCAATTTT CGCAGGA GGCTCTCT GATC | SEQ ID NO: 66 | +/- | | | | |
| | | 338delCCA | SEQ ID NO: 67 | +/- | | | | |
| | | 338delCCA+ 738delGCA GGAAACCA TCCAGTTC AinsATTTA GTTGCAAT TTTTGTCA AGGATATC TACCTGCT AATAGCTT GCCTTTTA GCAATTTT CGCAGGA GGCTCTCT GATC | SEQ ID NO: 68 | +/- | | | | |
| | | 758delA | SEQ ID NO: 69 | | | +/- | | |
| | | 733delCCAC AGCAGGAA ACCATCCA GTTCAinsG C | SEQ ID NO: 70 | | | | | +/+ |
| Nitab4.5_0 000861g00 50.1 | | 693delGAG GCTCTCTG ATCACAAT GCACGACG CCGCAAAC CACAGCAG GAAACCAT CCAGTTCA | SEQ ID NO: 71 | | | | | |
| | | 693delGAG+ 751delA | SEQ ID NO: 72 | | | | | |
| | | 693insT | SEQ ID NO: 73 | | | | | |
| | | 752insA | SEQ ID NO: 74 | | | | +/- | |
| | | 693delG+75 2insA | SEQ ID NO: 75 | +/+ | | | | |
| | SEQ ID NO: 15 | 693delG+74 9delTC | SEQ ID NO: 76 | | +/- | | | |
| NtSPL2/10/11_1b | | 693delG+75 0insA | SEQ ID NO: 77 | | +/- | | | |
| | | 693insT+752 insG | SEQ ID NO: 78 | | | +/- | | |
| | | 693insT+752 insT | SEQ ID NO: 79 | | | +/- | | |
| | | 693delGA+7 52insG | SEQ ID NO: 80 | | | +/- | | |
| | | 693delGA+7 52insT | SEQ ID NO: 81 | | | +/- | | |
| | | 693insT+746 delAGTTCA ACTCGGCA | SEQ ID NO: 82 | | | | +/- | |
| Nitab4.5_0 001315g01 10.1 NtSPL2/10 /11_3a | SEQ ID NO: 16 | 193insA | SEQ ID NO: 83 | | | | | |
| Nitab4.5_0 007217g00 40.1 | SEQ ID NO: 17 | 458delCACC CCCCA | SEQ ID NO: 84 | | | | | |
| NtSPL2/10 /11_3b | | | | | | | | |
| Nitab4.5_0 000819g00 10.1 | SEQ ID NO: 18 | 254delTCCA TTGG | SEQ ID NO: 85 | | +/+ | | | |
| | | 258insT | SEQ ID NO: 86 | | | +/- | +/+ | +/+ |
| NtFT5_1a | | 258insA | SEQ ID NO: 87 | +/- | | +/- | | |
| | | 258insC | SEQ ID NO: 88 | +/- | | | | |
| Nitab4.5_0 000751g01 80.1 | SEQ ID NO: 19 | 258insT | SEQ ID NO: 89 | +/- | +/+ | +/+ | | |
| | | 258insAT | SEQ ID NO: 90 | +/- | | | | |
| NtFT5_1b | | 258insC | SEQ ID NO: 91 | | | | +/+ | +/+ |

### B. Characterization of the mSPL, mFTSPL and mFT lines under greenhouse conditions

The T2 offspring of the lines mFT, mSPL1-4 and mFTSPLa-e described above (five plants per line) was studied under greenhouse conditions.

### B.1. mSPL lines

As described above, mSPL plants were generated in two successive rounds of gene editing. In a first round, the mSPL1 line was generated, whose genotype is shown in Figure 1. Later, in a second round, new mutations were added, giving rise to the mSPL2, mSPL3 and mSPL4 lines. Pooled analysis of mSPL mutants revealed that only the lines that accumulated the mutations in SPL genes gave rise to plants whose leaves had prevented the transition from the juvenile stage (rounded, with a length/width ratio value lower than 2) (Figure 2A and 2C), to the adult stage (lanceolated in the wild type, length/width ratio greater than 2) (Figure 2B and 2C).

Specifically, the combination of mutations present in the mSPL1 line was insufficient to block the transition from juvenile to adult leaf, while the rest of the second-round lines (mSPL2-4) analysed showed this block. Similarly, all mSPL lines except the mSPL1 line showed significant delays in the days to anthesis and, thus, in flowering time (Figure 2D).

Next, it was decided to study whether extended juvenility was accompanied by increased recombinant production capacity. To this end, leaves of adult plants (three months after sowing) of the different lines were agroinfiltrated with a gene construct containing the GFP gene under the control of a constitutive promoter (35SCaMV), (construct ID GB4293, SEQ ID NO: 96). It was observed that the lines mSPL2 and mSPL4, which presented homozygous loss-of-function mutations in all the genes of the SPL13 and SPL4 families and at least in half of the genes of the SPL3 and SPL9/15 families, presented also higher yields of GFP, which were on average 2.7 times higher than in the wild type line (Figure 3).

### B.2. mFT lines

Analysis of the multiFT mutants generated in parallel, showed that biallelic or homozygous loss-of-function combinations in two homeologous genes of the FT family (NtFT5a and NtFT5b) ensured complete abolition of flowering under all tested conditions. The mFT mutants (represented as mFT in Figure 1), biallelic NtFT5a and NtFT5b mutants, exhibited a juvenile-adult transition similar to wild-type plants (represented as mFT in Figure 2A, 2B and 2C). The number of leaves with juvenile characteristics produced by the mFT mutants was similar to that generated by wild plants (Figure 2E). The mFT plants did not flower in the period of time analysed (Figure 2D), nor later throughout the three years of study.

### B.3. mFTSPL lines

As previously described, starting from the mSPL1 mutant, a collection of multiFTSPL mutants was also generated (Figure 1) that combined the NtFT5a and NtFT5b mutations with mutations in SPL genes. These lines were developed with the objective of adding the non-flowering trait to the juvenility previously obtained by combining SPL mutations. Equivalent to the multiSPL mutants, the leaves of the 77-day-old multiFTSPL adult plants showed juvenile morphology (Figure 2C). For its part, and equivalent to the mFT mutants, no mFTSPL mutant flowered throughout the study (168 days, Figure 2D). Unexpectedly, the new combination of mutations was found not only to delay the juvenile-to-adult transition, but to abolish it completely and indefinitely, regardless of plant age. As a consequence of this indefinite production of leaves with a juvenile phenotype, at harvesting time (168 days) the number of juvenile leaves produced by the mFTSPL plants was higher on average than that of the mSPL lines and up to 10 times higher than that of WT plants (Figure 2E).

Surprisingly, the resulting plants retained and even increased the ability to produce higher average yields of recombinant protein compared to the control lines and the best of the mSPL lines when agroinfiltrated with plasmid GB4293 (SEQ ID NO: 96).

Remarkably, the mFTSPLb line presented the highest recombinant GFP production efficiencies observed among all the lines analyzed, 3.4 times higher than the wild type on average (Figure 3). The genotype of this line had mutations in all the genes of the SPL13, SPL4 and SPL9/15 families, 4 out of the 6 genes of the SPL3 family, one of the two genes of the SPL5 family, two of the 8 genes of the SPL2/10/11 family, and finally the mutated FT5a and FT5b genes, all homozygous.

In addition, in genetic transformation experiments, it was observed that the mFTSPLb line also had higher explant growth rates (Figure 4). For studying the regeneration capacity, leaves of equivalent ages were taken from WT plants and from the mFTSPLb mutant, (the mFTSPL line with more pronounced juvenile traits) and a stable transformation process was carried out with them using the plasmid GB_UA_7118 (SEQ ID NO: 137). As shown in the transformation experiment (Table 5), the number of shoots regenerated after 7 weeks from mFTSPLb leaves quadrupled those of the control.

**Table 5. Transformation experiment. Number of transgenic shoots at 4 and 7 weeks after transformation experiment with mFTSPLb.**

| **Genetic background** | **Selection marker** | **Plasmid ID** | **Backbone** | **Plasmid size (kb)** | **N° of transformed discs** | **N° of shoots after 4 weeks** | **N° of shoots after 7 weeks** |
|---|---|---|---|---|---|---|---|
| WT | nptlI | GB_UA_7 118 | pCambia | 10.3 | 105 | 0 | 19 |
| mFTSPLb | | | | | 105 | 7 | 91 |

Moreover, the early appearance of adventitious shoots was recorded, observing that at 4 weeks post-transformation only shoots appeared in the mFTSPLb genotype. As shown in Figure 4, the differences observed do not result from changes in the amount or rate of proliferation of undifferentiated calli, whose number is similar in the mutant and WT. On the contrary, it is the process of organogenesis, which gives rise to the formation of differentiated shoots, which is much faster (about 2-3 weeks) in mFTSPLb than in the WT line.

In summary, the addition of the non-flowering trait to the juvenile trait served to generate a set of more biosecure biofactory plant lines (by eliminating the possibility of unintended gene transmission by cross-pollination or seed). The production of juvenile leaves in the plant turned out to occur indefinitely over time in all the lines analyzed. In addition, in the mFTSPLb line, juvenility was associated to a maximum capacity of recombinant GFP protein production by agroinfiltration.

### C. Mechanisms behind high recombinant protein production in the mSPL and mFTSPL lines

To investigate the molecular mechanisms responsible for the greater production of recombinant protein (GFP) in leaves with the juvenile phenotype of adult plants of the multiFTSPL lines, compared to leaves with an adult phenotype of WT plants of the same age, a comparative transcriptomic analysis between leaves of the same age of the mFT and mFTSPLb and mFTSPLe genotypes was performed. Total RNA was extracted from leaves collected at two developmental stages (stage A and stage B) for three T2 plants of lines mFT, mFTSPLb and mFTSPLe and sequenced to analyse expression across the transcriptome. Stage A corresponded to 49 days after sowing, a timepoint at which the control wild type plants did not go through the juvenile-to-adult transition yet (Figure 2A). Stage B corresponded to 77 days after sowing, a timepoint at which the control wild type and mFT plants already showed an increased length/width ratio on their leaves (Figure 2B). Within the set of differentially expressed genes at stage B between genotypes mFT and mFTSPLb (1066 genes in total), the gene encoding protein argonaute 4 (AGO4) stood out. AGO4 is involved in the regulation of gene silencing mediated by epigenetic modifications of chromatin and in particular in DNA methylation.

Interestingly, Nitab4.5_0010831g0030 and Nitab4.5_0002368g0130 (the two AGO4 homeologue genes present in tobacco) were found to be differentially repressed in juvenile leaves of mFTSPLb compared to age-matched adult leaves of mFT (Figure 5A, stage B). A similar repression of AGO4 in adult leaves was observed for mFTSPLe compared to mFT (Figure 5A, stage B). Levels of transcripts at stage A were similar in the three genotypes for Nitab4.5_0002368g0130 and decreased in mFTSPLb and mFTSPLe compared to mFT for Nitab4.5_0010831g0030. mRNA levels of AGO4 were also determined by RT-qPCR in samples obtained from equivalent leaves for all mSPL and mFTSPL lines with similar results (Figure 5B). This observation led to the formulation of the hypothesis that the higher yields of recombinant protein in plants with accumulation of SPL mutations is due to a lower capacity for DNA methylation of the transgenes introduced transiently by agroinfiltration and therefore a lower capacity to repress their expression.

The transient expression of transgenes in biofactory plants by using the agroinfiltration technique makes possible to introduce one or several copies of linear DNA fragments (T-DNA) containing the gene of interest, usually flanked by a promoter and a terminator regions that control gene expression, into the nucleus of the leaf cells. In other types of strategies, the T-DNA contains an infective viral clone which in turn contains the transgene of interest. The expression and subsequent replication of the infectious viral clone allows the number of copies of the transgene of interest (gene dose) to be amplified and, as a consequence, to increase the yields of the resulting recombinant protein. The most commonly used infective clones correspond to RNA viruses such as Tobacco Mosaic Virus or Potato Virus X.

However, there is growing interest in the use of infective clones derived from DNA viruses in the field of biofactories, and in particular of vectors derived from viruses of the geminivirus family. These DNA viruses present, among other favorable characteristics, a greater cargo capacity to carry foreign DNA and a greater ease of producing several recombinant proteins simultaneously.

It is known that transcriptional silencing based on DNA methylation is one of the main mechanisms by which plants defend themselves against infection by DNA viruses of the geminivirus family. This opened the possibility that the extended juvenility of the multiSPL and multiFTSPL mutants had effects on the yield of geminivirus-mediated recombinant production, and that these positive effects were even greater than in the case of non-replicative vectors such as those studied before. To verify this possibility, the yields of recombinant GFP obtained by agroinfiltration of a recombinant geminivirus vector based on the Bean Yellow Dwarf virus (BeYDV) (GB4312, SEQ ID NO: 97) along with the BeYDV Rep/RepA (GB3598, SEQ ID NO: 138) were tested in multiSPL and multiFTSPL plants compared with their WT controls. Strikingly, it was observed that the GFP yields were up to 11.6 times higher in the mFTSPLb line compared to the WT (Figure 6A and 6B), and that in general, the GFP yields varied accordingly to the juvenility of the different lines tested.

Beyond reporter proteins such as GFP, the interest of biofactory plants lies in the possibility of producing proteins with high added value such as vaccines and therapeutic antibodies. To verify the superior capacity of the improved line mFTSPLb as a recombinant protein biofactory, a single chain nano-antibody (nano72) against the spike protein of the SARSCov2 virus was expressed in this line. For this, the antibody sequence was cloned in the BeYDV geminivirus vector and the resulting construct (GB4419, SEQ ID NO: 98) along with the BeYDV Rep/RepA (GB3598, SEQ ID NO: 138) were used to agroinfiltrate leaves of WT tobacco and of the mFTSPLb mutant. Western blot analysis of the resulting leaf extracts is shown in Figure 6C. As can be seen, the relative yield obtained in leaves of mFTSPLb plants was much higher than in the WT, consistent with those observed in the case of GFP. This result confirms the improvement of mFTSPLb as a biofactory.

### D. Mechanisms for flowering reversibility in the mFTSPL and mFT lines

In order to obtain successive generations of mFTSPL and mFT plants with a non-flowering phenotype, it was necessary to devise an effective flowering reversion system. The present invention also provides mechanisms for reversing the non-flowering phenotype so that plants with flowering abolished by mutations in the FT5a and FT5b genes can be propagated by seed. Flowering of a double allelic mutant genotype in the two NtFT5 homeologs, could not be reverted with changes in photoperiod or with any other reversion mechanism based on growth conditions. FT is a mobile factor that acts downstream of SPLs in floral signaling. Therefore, flowering of plants with NtFT5a/b-KO mutations, including multiFTSPL plants, can be reversed by exogenous supplementation of FT mRNA or FT protein. This can be implemented by genetic transformation (stable or transient), grafting with a WT stock, or infecting the plant with a viral vector containing a recombinant FT sequence.

In particular, it was shown that the agroinoculation of a recombinant viral vector derived from the tobacco etch virus (TEV) that incorporates the *Arabidopsis thaliana* FT (AtFT) sequence in its genome (pGTEV-G2-AtFT, SEQ ID NO: 139), reverses flowering in 100% of the lines with NtFT5a/b-KO mutations, including the multiFTSPL plants analysed (Figure 7A). Approximately ten days after agroinoculation of mFT and mFTSPL plants with *Agrobacterium tumefaciens* strain GV3101 carrying the plasmid pGTEV-G2-AtFT, typical symptoms associated with TEV infection including mottling (Figure 7B and 7C) or vein clearing (Figure 7D) appeared on the leaves. And roughly two weeks later, floral shoots appeared on the inoculated plants, which subsequently generated flowers and seeds (Figure 7E and 7F).

In summary, the present invention defines a minimal combination of mutations in tobacco SPL genes (particularly the lines SPL 2, 3 and 4), which confer to the tobacco plant an increased capacity for recombinant protein production, in particular by the agroinfiltration technique, and more specifically by the agroinfiltration technique mediated by a DNA virus of the geminivirus family. In addition, the present invention defines another ideal combination of mutations in tobacco SPL and FT genes (the line mFTSPLb with 4xSPL3, 2xSPL4, 1xSPL5, 4x SPL9/15, 2x SPL2/10/11, 2xSPL13 and 2xFT5 mutations), which confer to the tobacco plant a maximum capacity of recombinant protein production, in particular by means of the geminivirus-mediated agroinfiltration technique. This ideal combination also extends indefinitely over time the maximum production capacity of recombinant protein in the leaves and confers as an added advantage a biosafety level increased by the genetic containment that the absence of flowering implies, which can be reversed in a controlled manner for seed production.

## Claims

1. A plant belonging to *Nicotiana tabacum* or part thereof, reproductive or propagating plant material or a plant cell, **characterized in that** it comprises:
(a) one monoallelic heterozygous mutation, biallelic heterozygous mutation or biallelic homozygous mutation in the coding region of the following SPL genes:
- gene comprising, or consisting of, nucleotide sequence SEQ ID NO: 1,
- gene comprising, or consisting of, nucleotide sequence SEQ ID NO: 2,
- gene comprising, or consisting of, nucleotide sequence SEQ ID NO: 4,
- gene comprising, or consisting of, nucleotide sequence SEQ ID NO: 6,
- gene comprising, or consisting of, nucleotide sequence SEQ ID NO: 7,
- gene comprising, or consisting of, nucleotide sequence SEQ ID NO: 8,
- gene comprising, or consisting of, nucleotide sequence SEQ ID NO: 9,
- gene comprising, or consisting of, nucleotide sequence SEQ ID NO: 12, and
- gene comprising, or consisting of, nucleotide sequence SEQ ID NO: 13, and
(b) at least one monoallelic heterozygous mutation, biallelic heterozygous mutation or biallelic homozygous mutation in the coding region of at least one of the genes selected from the list consisting of:
- gene comprising, or consisting of, nucleotide sequence SEQ ID NO: 3,
- gene comprising, or consisting of, nucleotide sequence SEQ ID NO: 5,
- gene comprising, or consisting of, nucleotide sequence SEQ ID NO: 10,
- gene comprising, or consisting of, nucleotide sequence SEQ ID NO: 11,
- gene comprising, or consisting of, nucleotide sequence SEQ ID NO: 14,
- gene comprising, or consisting of, nucleotide sequence SEQ ID NO: 15,
- gene comprising, or consisting of, nucleotide sequence SEQ ID NO: 16, and
- gene comprising, or consisting of, nucleotide sequence SEQ ID NO: 17, and any combination thereof,
wherein the plant or part thereof, reproductive or propagating plant material, or a plant cell, is not obtained by means of an essentially biological process.

2. The plant or part thereof, reproductive or propagating plant material or a plant cell, according to claim 1, wherein the plant comprises an increased capacity for recombinant product production, preferably the recombinant product is a recombinant protein, a recombinant nucleic acid, or a recombinant metabolite.

3. The plant or part thereof, reproductive or propagating plant material or a plant cell, according to claim 1 or 2, wherein the plant further comprises at least one biallelic heterozygous mutation or biallelic homozygous mutation in the genes:
- gene comprising or consisting of nucleotide sequence SEQ ID NO: 18, and
- gene comprising or consisting of nucleotide sequence SEQ ID NO: 19.

4. The plant or part thereof, reproductive or propagating plant material or a plant cell, according to any one of the claims 1 to 3, wherein:
- the mutation in the nucleotide sequence SEQ ID NO: 1 is 353delACAAC;
- the mutation in the nucleotide sequence SEQ ID NO: 2 is 349delGGACACAA or 355delAA;
- the mutation in the nucleotide sequence SEQ ID NO: 3 is 142insA and/or 225deICCATGCAAAGGCTCAA;
- the mutation in the nucleotide sequence SEQ ID NO: 4 is 213delC, 425insT and/or 425insA;
- the mutation in the nucleotide sequence SEQ ID NO: 5 is 225delC or 225insA;
- the mutation in the nucleotide sequence SEQ ID NO: 6 is 204delCA;
- the mutation in the nucleotide sequence SEQ ID NO: 7 is 213insA;
- the mutation in the nucleotide sequence SEQ ID NO: 8 is 289insC;
- the mutation in the nucleotide sequence SEQ ID NO: 9 is 490insT;
- the mutation in the nucleotide sequence SEQ ID NO: 10 is selected from the list consisting of: 117delAAATGT, 121delGT, 217insA, 217delAGinsT, 217delAG, 218deIG and any combination thereof;
- the mutation in the nucleotide sequence SEQ ID NO: 11 is 217delA, 217insA or 217delAG;
- the mutation in the nucleotide sequence SEQ ID NO: 12 is selected from the list consisting of: 135delTA, 135delTAAAGT, 269delGGCG, 272delG, 273delT, 274insA, 274insT and any combination thereof;
- the mutation in the nucleotide sequence SEQ ID NO: 13 is selected from the list consisting of: 123insT, 123delT, 275deIGTCGA, 277insT, 277insC, 277insA, 277insG and any combination thereof;
- the mutation in the nucleotide sequence SEQ ID NO: 14 is selected from the list consisting of: 13delGCA, 338delCCA, 598delC, 733deICCACAGCAGGAAACCATCCAGTTCAinsGC, 738delGCAGGAAACCATCCAGTTCAinsATTTAGTTGCAATTTTTGTCAA GGATATCTACCTGCTAATAGCTTGCCTTTTAGCAATTTTCGCAGGAGG CTCTCTGATC, 754delTTC, 756delCA, 758insA, 758delA, and any combination thereof;
- the mutation in the nucleotide sequence SEQ ID NO: 15 is selected from the list consisting of: 693delGAGGCTCTCTGATCACAATGCACGACGCCGCAAACCACAGCAG GAAACCATCCAGTTCA, 693delG, 693delGA, 693delGAG, 693insT, 746delAGTTCAACTCGGCA, 749delTC, 750insA, 751delA, 752insA, 752insG, 752insT and any combination thereof;
- the mutation in the nucleotide sequence SEQ ID NO: 16 is 193insA;
- the mutation in the nucleotide sequence SEQ ID NO: 17 is 458delCACCCCCCA;
- the mutation in the nucleotide sequence SEQ ID NO: 18 is selected from the list consisting of: 254deITCCATTGG, 258insT, 258insA and 258insC;
and/or
- the mutation in the nucleotide sequence SEQ ID NO: 19 is 258insT, 258insAT or 258insC.

5. The plant or part thereof, reproductive or propagating plant material or a plant cell, according to any one of the claims 1 to 4, **characterized in that** it comprises:
(a) a biallelic homozygous mutation in the coding region of all following genes:
- gene comprising, or consisting of, nucleotide sequence SEQ ID NO: 1, wherein the mutation in the nucleotide sequence SEQ ID NO: 1 is 353delACAAC;
- gene comprising, or consisting of, nucleotide sequence SEQ ID NO: 2, wherein the mutation in the nucleotide sequence SEQ ID NO: 2 is 355delAA;
- gene comprising, or consisting of, nucleotide sequence SEQ ID NO: 4, wherein the mutation in the nucleotide sequence SEQ ID NO: 4 is 213delC;
- gene comprising, or consisting of, nucleotide sequence SEQ ID NO: 6, wherein the mutation in the nucleotide sequence SEQ ID NO: 6 is 204delCA;
- gene comprising, or consisting of, nucleotide sequence SEQ ID NO: 7, wherein the mutation in the nucleotide sequence SEQ ID NO: 7 is 213insA;
- gene comprising, or consisting of, nucleotide sequence SEQ ID NO: 8, wherein the mutation in the nucleotide sequence SEQ ID NO: 8 is 289insC;
- gene comprising, or consisting of, nucleotide sequence SEQ ID NO: 9, wherein the mutation in the nucleotide sequence SEQ ID NO: 9 is 490insT; and
- gene comprising, or consisting of, nucleotide sequence SEQ ID NO: 13, wherein the mutation in the nucleotide sequence SEQ ID NO: 13 is 277insA;
and
(b) a biallelic heterozygous mutation in the coding region of all following genes:
- gene comprising, or consisting of, nucleotide sequence SEQ ID NO: 12, wherein the mutations in the nucleotide sequence SEQ ID NO: 12 are 274insA and 272delG; and
- gene comprising, or consisting of, nucleotide sequence SEQ ID NO: 14, wherein the mutations in the nucleotide sequence SEQ ID NO: 14 are 13delGCA, 598delC and 758insA;
and
(c) a monoallelic heterozygous mutation in the coding region of the following genes:
- gene comprising, or consisting of, nucleotide sequence SEQ ID NO: 3, wherein the mutation in the nucleotide sequence SEQ ID NO: 3 is 142insA and 225deICCATGCAAAGGCTCAA;
- gene comprising, or consisting of, nucleotide sequence SEQ ID NO: 4, wherein the mutation in the nucleotide sequence SEQ ID NO: 4 is 425insA;
- gene comprising, or consisting of, nucleotide sequence SEQ ID NO: 5, wherein the mutation in the nucleotide sequence SEQ ID NO: 5 is 225delC;
- gene comprising, or consisting of, nucleotide sequence SEQ ID NO: 10, wherein the mutation in the nucleotide sequence SEQ ID NO: 10 is 217insA; and
- gene comprising, or consisting of, nucleotide sequence SEQ ID NO: 15, wherein the mutation in the nucleotide sequence SEQ ID NO: 15 is 752insA.

6. The plant or part thereof, reproductive or propagating plant material or a plant cell, according to any one of the claims 1 to 4, **characterized in that** it comprises:
(a) a biallelic homozygous mutation in the coding region of the following genes:
- gene comprising, or consisting of, nucleotide sequence SEQ ID NO: 1, wherein the mutation in the nucleotide sequence SEQ ID NO: 1 is 353delACAAC;
- gene comprising, or consisting of, nucleotide sequence SEQ ID NO: 2, wherein the mutation in the nucleotide sequence SEQ ID NO: 2 is 355delAA;
- gene comprising, or consisting of, nucleotide sequence SEQ ID NO: 3, wherein the mutation in the nucleotide sequence SEQ ID NO: 3 is 142insA;
- gene comprising, or consisting of, nucleotide sequence SEQ ID NO: 4, wherein the mutation in the nucleotide sequence SEQ ID NO: 4 is 213delC;
- gene comprising, or consisting of, nucleotide sequence SEQ ID NO: 5, wherein the mutation in the nucleotide sequence SEQ ID NO: 5 is 225insA;
- gene comprising, or consisting of, nucleotide sequence SEQ ID NO: 6, wherein the mutation in the nucleotide sequence SEQ ID NO: 6 is 204delCA;
- gene comprising, or consisting of, nucleotide sequence SEQ ID NO: 7, wherein the mutation in the nucleotide sequence SEQ ID NO: 7 is 213insA;
- gene comprising, or consisting of, nucleotide sequence SEQ ID NO: 8, wherein the mutation in the nucleotide sequence SEQ ID NO: 8 is 289insC;
- gene comprising, or consisting of, nucleotide sequence SEQ ID NO: 9, wherein the mutation in the nucleotide sequence SEQ ID NO: 9 is 490insT;
- gene comprising, or consisting of, nucleotide sequence SEQ ID NO: 10, wherein the mutation in the nucleotide sequence SEQ ID NO: 10 is 217insA;
- gene comprising, or consisting of, nucleotide sequence SEQ ID NO: 11, wherein the mutation in the nucleotide sequence SEQ ID NO: 11 is 217delA;
- gene comprising, or consisting of, nucleotide sequence SEQ ID NO: 12, wherein the mutation in the nucleotide sequence SEQ ID NO: 12 is 135delTA and 274insA;
- gene comprising, or consisting of, nucleotide sequence SEQ ID NO: 14, wherein the mutation in the nucleotide sequence SEQ ID NO: 14 is 758insA;
- gene comprising, or consisting of, nucleotide sequence SEQ ID NO: 15, wherein the mutation in the nucleotide sequence SEQ ID NO: 15 is 693delG;
- gene comprising, or consisting of, nucleotide sequence SEQ ID NO: 18, wherein the mutation in the nucleotide sequence SEQ ID NO: 18 is 254deITCCATTGG; and
- gene comprising, or consisting of, nucleotide sequence SEQ ID NO: 19, wherein the mutation in the nucleotide sequence SEQ ID NO: 19 is 258insT;
and
(b) a biallelic heterozygous mutation in the coding region of all following genes
- gene comprising, or consisting of, nucleotide sequence SEQ ID NO: 13, wherein the mutations in the nucleotide sequences SEQ ID NO: 13 are either 123insT and 277insA in one allele and 277insG in the other allele or 123insT and 277insG in one allele and 277insA in the other allele;
and
- gene comprising, or consisting of, nucleotide sequence SEQ ID NO: 15, wherein the mutations in the nucleotide sequence SEQ ID NO: 15 are 749deITC and 750insA;
and
(c) a monoallelic heterozygous mutation in the coding region of the gene:
- gene comprising, or consisting of, nucleotide sequence SEQ ID NO: 4, wherein the mutation in the nucleotide sequence SEQ ID NO: 4 is 425insT.

7. A method for producing a plant or part thereof, reproductive or propagating plant material or a plant cell, according to any one of the claims 1 to 6, comprising subjecting the plant belonging to *Nicotiana tabacum* or part thereof, the reproductive or propagating plant material, or the plant cell, to directed mutagenesis introducing:
(a) at least one monoallelic heterozygous mutation, biallelic heterozygous mutation or biallelic homozygous mutation in the coding region of the following genes:
- gene comprising, or consisting of, nucleotide sequence SEQ ID NO: 1,
- gene comprising, or consisting of, nucleotide sequence SEQ ID NO: 2,
- gene comprising, or consisting of, nucleotide sequence SEQ ID NO: 4,
- gene comprising, or consisting of, nucleotide sequence SEQ ID NO: 6,
- gene comprising, or consisting of, nucleotide sequence SEQ ID NO: 7,
- gene comprising, or consisting of, nucleotide sequence SEQ ID NO: 8, and
- gene comprising, or consisting of, nucleotide sequence SEQ ID NO: 9,
- gene comprising, or consisting of, nucleotide sequence SEQ ID NO: 12, and
- gene comprising, or consisting of, nucleotide sequence SEQ ID NO: 13,
and
(b) at least one monoallelic heterozygous mutation, biallelic heterozygous mutation or biallelic homozygous mutation in the coding region of at least one of the genes selected from the list consisting of:
- gene comprising, or consisting of, nucleotide sequence SEQ ID NO: 3,
- gene comprising, or consisting of, nucleotide sequence SEQ ID NO: 5,
- gene comprising, or consisting of, nucleotide sequence SEQ ID NO: 10,
- gene comprising, or consisting of, nucleotide sequence SEQ ID NO: 11,
- gene comprising, or consisting of, nucleotide sequence SEQ ID NO: 14,
- gene comprising, or consisting of, nucleotide sequence SEQ ID NO: 15,
- gene comprising, or consisting of, nucleotide sequence SEQ ID NO: 16, and
- gene comprising, or consisting of, nucleotide sequence SEQ ID NO: 17, and any combination thereof,
wherein the plant or part thereof, reproductive or propagating plant material, or a plant cell, is not obtained by means of an essentially biological process.

8. The method according to claim 7 wherein the directed mutagenesis is achieved by homologous recombination-dependent gene targeting, directed transposon insertion, or genome editing techniques, preferably wherein the genome editing technique is CRISPR/Cas technique.

9. The method according to claim 8 further comprising Agrobacterium-mediated genetic transformation, preferably in combination with CRISPR/Cas technique.

10. The method according to claim 8 or 9, wherein the CRISPR/Cas technique is carried out by using the vector comprising, or consisting of, the nucleotide sequence SEQ ID NO: 92, SEQ ID NO: 93, SEQ ID NO: 94 or SEQ ID NO: 95.

11. Use of the plant or part thereof, the reproductive or propagating plant material, or the plant cell, according to any one of claims 1 to 6 for producing recombinant products, preferably the recombinant product is a recombinant protein, a recombinant nucleic acid, or a recombinant metabolite.

12. A method for producing recombinant products comprising:
- transforming the plant or part thereof, the reproductive or propagating plant material, or the plant cell, according to any one of claims 1 to 6 with a strain of *Agrobacterium* comprising a vector, wherein said vector comprising or encoding at least one recombinant product.

13. The method according to claim 12, wherein the expression cassette comprises a promoter, a terminator region and/or an infective viral clone, preferably the promoter is the cauliflower mosaic virus 358 (35SCaMV) promoter.

14. The method according to claim 12, wherein the vector is a geminivirus vector, preferably wherein the virus is the Bean Yellow Dwarf Virus (BeYDV).
